# EUROPEAN PATENT APPLICATION

(11) **EP 3 838 345 A1**
(43) Date of publication of application: **23.06.2021**
(21) Application number: 19218576.7
(22) Date of filing: 20.12.2019
(51) Int. Cl.: A61P 25/16, A61K 38/12

(54) **MACROCYCLIC PEPTIDES**

(71) Applicant: Herantis Pharma Oyj, 02600 Espoo (FI)
(72) Inventor: HUTTUNEN, Henri, 00670 Helsinki (FI); BHATTACHARJEE, Arnab, 02760 Espoo (FI); KULESSKAYA, Natalia, 00790 Helsinki (FI); JOHNSSON, Richard, 24541 Staffanstorp (SE); VOLKOVA, Maria, 22274 Lund (SE)
(74) Representative: Boco IP Oy Ab

(57) **Abstract**

The present disclosure relates to the field of unconventional neurotrophic factors and to the field of treating degenerative, chronic or progressive diseases and disorders, and monogenic hereditary diseases having ER stress as a pathogenic compound. More particularly the disclosure relates to modified peptides, particularly macrocyclic peptides. The disclosure also relates to pharmaceutical compositions comprising said peptides. Further, the disclosure also relates to said peptides, and pharmaceutical compositions for use as a medicament and in the treatment of degenerative, chronic or progressive diseases and disorders, and monogenic hereditary diseases having ER stress as a pathogenic compound as well as to methods for treating said diseases and disorders.

SEQUENCE LISTING

<110> Herantis Pharma Oyj

<120> Macrocyclic peptides

<130> P4701EP00

<160> 70

<170> BiSSAP 1.3.6

<210> 1
<211> 27
<212> PRT
<213> Artificial Sequence

<220>
<223> disulphide bridge

<220>
<221> DISULFID
<222> join(22..25)

<400> 1
Val Asp Leu Arg Lys Met Arg Val Ala Glu Leu Lys Gln Ile Leu His
1               5                   10                  15
Ser Trp Gly Glu Glu Cys Arg Ala Cys Ala Glu
            20                  25

<210> 2
<211> 27
<212> PRT
<213> Artificial Sequence

<220>
<223> head-to-tail cyclic, disulphide bridge

<220>
<221> DISULFID
<222> join(22..25)

<400> 2
Val Asp Leu Arg Lys Met Arg Val Ala Glu Leu Lys Gln Ile Leu His
1               5                   10                  15
Ser Trp Gly Glu Glu Cys Arg Ala Cys Ala Glu
            20                  25

<210> 3
<211> 27
<212> PRT
<213> Artificial Sequence

<220>
<223> disulphide bridge

<220>
<221> DISULFID
<222> join(22..25)

<400> 3
Val Asp Leu Lys Lys Leu Arg Val Lys Glu Leu Lys Lys Ile Leu Asp
1               5                   10                  15
Asp Trp Gly Glu Thr Cys Lys Gly Cys Ala Glu
            20                  25

<210> 4
<211> 27
<212> PRT
<213> Artificial Sequence

<220>
<223> head-to-tail cyclic,disulphide bridge

<220>
<221> DISULFID
<222> join(22..25)

<400> 4
Val Asp Leu Lys Lys Leu Arg Val Lys Glu Leu Lys Lys Ile Leu Asp
1               5                   10                  15
Asp Trp Gly Glu Thr Cys Lys Gly Cys Ala Glu
            20                  25

<210> 5
<211> 23
<212> PRT
<213> Artificial Sequence

<220>
<223> disulphide bridge

<220>
<221> DISULFID
<222> join(17..20)

<400> 5
Met Arg Val Ala Glu Leu Lys Gln Ile Leu His Ser Trp Gly Glu Glu
1               5                   10                  15
Cys Arg Ala Cys Ala Glu Lys
            20

<210> 6
<211> 23
<212> PRT
<213> Artificial Sequence

<220>
<223> head-to-tail cyclic, disulphide bridge

<220>
<221> DISULFID
<222> join(17..20)

<400> 6
Met Arg Val Ala Glu Leu Lys Gln Ile Leu His Ser Trp Gly Glu Glu
1               5                   10                  15
Cys Arg Ala Cys Ala Glu Lys
            20

<210> 7
<211> 23
<212> PRT
<213> Artificial Sequence

<220>
<223> disulphide bridge, head-to-tail

<220>
<221> DISULFID
<222> join(17..20)

<400> 7
Leu Arg Val Lys Glu Leu Lys Lys Ile Leu Asp Asp Trp Gly Glu Thr
1               5                   10                  15
Cys Lys Gly Cys Ala Glu Lys
            20

<210> 8
<211> 23
<212> PRT
<213> Artificial Sequence

<220>
<223> head-to-tail cyclic, disulphide bridge

<220>
<221> DISULFID
<222> join(17..20)

<400> 8
Leu Arg Val Lys Glu Leu Lys Lys Ile Leu Asp Asp Trp Gly Glu Thr
1               5                   10                  15
Cys Lys Gly Cys Ala Glu Lys
            20

<210> 9
<211> 12
<212> PRT
<213> Artificial Sequence

<220>
<223> disulphide bridge

<220>
<221> DISULFID
<222> join(5..8)

<400> 9
Trp Gly Glu Glu Cys Arg Ala Cys Ala Glu Lys Thr
1               5                   10

<210> 10
<211> 12
<212> PRT
<213> Artificial Sequence

<220>
<223> head-to-tail cyclic, disulphide bridge

<220>
<221> DISULFID
<222> join(5..8)

<400> 10
Trp Gly Glu Glu Cys Arg Ala Cys Ala Glu Lys Thr
1               5                   10

<210> 11
<211> 12
<212> PRT
<213> Artificial Sequence


<220>
<223> disulphide bridge

<220>
<221> DISULFID
<222> join(5..8)

<400> 11
Trp Gly Glu Thr Cys Lys Gly Cys Ala Glu Lys Ser
1               5                   10

<210> 12
<211> 12
<212> PRT
<213> Artificial Sequence


<220>
<223> head-to-tail cyclic, disulphide bridge

<220>
<221> DISULFID
<222> join(5..8)

<400> 12
Trp Gly Glu Thr Cys Lys Gly Cys Ala Glu Lys Ser
1               5                   10

<210> 13
<211> 11
<212> PRT
<213> Artificial Sequence


<220>
<223> disulphide bridge

<220>
<221> DISULFID
<222> join(4..7)

<400> 13
Gly Glu Glu Cys Arg Ala Cys Ala Glu Lys Thr
1               5                   10

<210> 14
<211> 12
<212> PRT

<213> Artificial Sequence

<220>
<223> disulphide bridge

<220>
<221> DISULFID
<222> join(4..8)

<400> 14
Gly Glu Glu Cys Arg Gly Ala Cys Ala Glu Lys Thr
1               5                   10

<210> 15
<211> 12
<212> PRT
<213> Artificial Sequence

<220>
<223> disulphide bridge

<220>
<221> DISULFID
<222> join(4..8)

<400> 15
Gly Glu Glu Cys Arg Ala Ala Cys Ala Glu Lys Thr
1               5                   10

<210> 16
<211> 12
<212> PRT
<213> Artificial Sequence

<220>
<223> disulphide bridge

<220>
<221> DISULFID
<222> join(4..8)

<400> 16
Gly Glu Glu Cys Arg Ser Ala Cys Ala Glu Lys Thr
1               5                   10

<210> 17
<211> 11
<212> PRT
<213> Artificial Sequence

<220>
<223> disulphide bridge

<220>
<221> DISULFID
<222> join(4..7)

<400> 17
Gly Glu Thr Cys Lys Gly Cys Ala Glu Lys Ser

1                    5                        10

<210> 18
<211> 12
<212> PRT
<213> Artificial Sequence

<220>
<223> disulphide bridge

<220>
<221> DISULFID
<222> join(4..8)

<400> 18
Gly Glu Thr Cys Lys Gly Gly Cys Ala Glu Lys Ser
1                    5                        10

<210> 19
<211> 12
<212> PRT
<213> Artificial Sequence

<220>
<223> disulphide bridge

<220>
<221> DISULFID
<222> join(4..8)

<400> 19
Gly Glu Thr Cys Lys Ala Gly Cys Ala Glu Lys Ser
1                    5                        10

<210> 20
<211> 12
<212> PRT
<213> Artificial Sequence

<220>
<223> disulphide bridge

<220>
<221> DISULFID
<222> join(4..8)

<400> 20
Gly Glu Thr Cys Lys Ser Gly Cys Ala Glu Lys Ser
1                    5                        10

<210> 21
<211> 13
<212> PRT
<213> Artificial Sequence

<220>
<223> disulphide bridge

<220>

<221> DISULFID
<222> join(5..9)

<400> 21
Trp Gly Glu Glu Cys Arg Gly Ala Cys Ala Glu Lys Thr
1               5                   10

<210> 22
<211> 13
<212> PRT
<213> Artificial Sequence

<220>
<223> head-to-tail cyclic, disulphide bridge

<220>
<221> DISULFID
<222> join(5..9)

<400> 22
Trp Gly Glu Glu Cys Arg Gly Ala Cys Ala Glu Lys Thr
1               5                   10

<210> 23
<211> 13
<212> PRT
<213> Artificial Sequence

<220>
<223> disulphide bridge

<220>
<221> DISULFID
<222> join(5..9)

<400> 23
Trp Gly Glu Thr Cys Lys Gly Gly Cys Ala Glu Lys Ser
1               5                   10

<210> 24
<211> 13
<212> PRT
<213> Artificial Sequence

<220>
<223> head-to-tail cyclic, disulphide bridge

<220>
<221> DISULFID
<222> join(5..9)

<400> 24
Trp Gly Glu Thr Cys Lys Gly Gly Cys Ala Glu Lys Ser
1               5                   10

<210> 25
<211> 5
<212> PRT
<213> Artificial Sequence

<220>
<223> peptide

<220>
<221> VARIANT
<222> 2
<223> X1 = absent, R, K, I, G, A or S

<220>
<221> VARIANT
<222> 3
<223> X2 = G, A, R, K, I or S

<220>
<221> VARIANT
<222> 4
<223> X3 = A, G or S

<400> 25
Cys Xaa Xaa Xaa Cys
1               5

<210> 26
<211> 9
<212> PRT
<213> Artificial Sequence


<220>
<223> peptide

<220>
<221> VARIANT
<222> 2
<223> X4 = E, T, V, D, M or G

<220>
<221> VARIANT
<222> 4
<223> X1 = R, K, I, G, A or S

<220>
<221> VARIANT
<222> 5
<223> X2 = absent, G, A, R, K, I or S

<220>
<221> VARIANT
<222> 6
<223> X3 = A, G or S

<400> 26
Glu Xaa Cys Xaa Xaa Xaa Cys Ala Glu
1               5

<210> 27
<211> 16
<212> PRT
<213> Artificial Sequence


<220>

<223> peptide

<220>
<221> VARIANT
<222> 1
<223> X5 = absent, H, D, Q, R, Y, N or S

<220>
<221> VARIANT
<222> 2
<223> X6 = absent, S, D, G, N or R

<220>
<221> VARIANT
<222> 3
<223> X7 = absent or W

<220>
<221> VARIANT
<222> 4
<223> X8 = absent or G

<220>
<221> VARIANT
<222> 6
<223> X4 = E, T, V, D, M or G

<220>
<221> VARIANT
<222> 8
<223> X1 = R, K, I, G, A or S

<220>
<221> VARIANT
<222> 9
<223> X2 = absent, G, A, R, K, I or S

<220>
<221> VARIANT
<222> 10
<223> X3 = A, G or S

<220>
<221> VARIANT
<222> 14
<223> X9 = absent or K

<220>
<221> VARIANT
<222> 15
<223> X10 = absent, T, S, A, I or N

<220>
<221> VARIANT
<222> 16
<223> X11 = absent, D or E

<400> 27
Xaa Xaa Xaa Xaa Glu Xaa Cys Xaa Xaa Xaa Cys Ala Glu Xaa Xaa Xaa
1               5                   10                  15

<210> 28
<211> 36
<212> PRT
<213> Artificial Sequence

<220>
<223> peptide

<220>
<221> VARIANT
<222> 1
<223> X12 = absent, L, I or V

<220>
<221> VARIANT
<222> 2
<223> X13 = absent or D

<220>
<221> VARIANT
<222> 3
<223> X14 = absent, L or W

<220>
<221> VARIANT
<222> 4
<223> X15 = absent, A, S, T, E or N

<220>
<221> VARIANT
<222> 5
<223> X16 = absent, S or T

<220>
<221> VARIANT
<222> 6
<223> X17 = absent, V or D

<220>
<221> VARIANT
<222> 7
<223> X18 = absent, D or A

<220>
<221> VARIANT
<222> 8
<223> X19 = absent or L

<220>
<221> VARIANT
<222> 9
<223> X20 = absent, R, K, S or W

<220>
<221> VARIANT
<222> 10
<223> X21 = absent or K

<220>
<221> VARIANT
<222> 11
<223> X22 = absent, M, L, I or V

<220>
<221> VARIANT
<222> 12
<223> X23 = absent or R

<220>
<221> VARIANT
<222> 14
<223> X24 = A, K, T, L or V

<220>
<221> VARIANT
<222> 18
<223> X25 = Q, K or R

<220>
<221> VARIANT
<222> 19
<223> X26 = I or V

<220>
<221> VARIANT
<222> 21
<223> X5 = absent, H, D, Q, R, Y, N or S

<220>
<221> VARIANT
<222> 22
<223> X6 = absent, S, D, G, N or R

<220>
<221> VARIANT
<222> 23
<223> X7 = absent or W

<220>
<221> VARIANT
<222> 24
<223> X8 = absent or G

<220>
<221> VARIANT
<222> 26
<223> X4 = E, T, V, D, M or G

<220>
<221> VARIANT
<222> 28
<223> X1 = R, K, I, G, A or S

<220>
<221> VARIANT
<222> 29
<223> X2 = absent, G, A, R, K, I or S

<220>
<221> VARIANT
<222> 30
<223> X3 = A, G or S

<220>
<221> VARIANT

<222> 34
<223> X9 = absent or K

<220>
<221> VARIANT
<222> 35
<223> X10 = absent, T, S, A, I or N

<220>
<221> VARIANT
<222> 36
<223> X11 = absent, D or E

<400> 28
Xaa Xaa Xaa Xaa Xaa Xaa Xaa Xaa Xaa Xaa Xaa Xaa Val Xaa Glu Leu
1               5                   10                  15
Lys Xaa Xaa Leu Xaa Xaa Xaa Xaa Glu Xaa Cys Xaa Xaa Xaa Cys Ala
            20                  25                  30
Glu Xaa Xaa Xaa
        35

<210> 29
<211> 37
<212> PRT
<213> Homo sapiens

<220>
<223> native human CDNF peptide

<400> 29
Arg Val Ala Glu Leu Lys Gln Ile Leu His Ser Trp Gly Glu Glu Cys
1               5                   10                  15
Arg Ala Cys Ala Glu Lys Thr Asp Tyr Val Asn Leu Ile Gln Glu Leu
            20                  25                  30
Ala Pro Lys Tyr Ala
        35

<210> 30
<211> 37
<212> PRT
<213> Homo sapiens

<220>
<223> native human MANF peptide

<400> 30
Arg Val Lys Glu Leu Lys Lys Ile Leu Asp Asp Trp Gly Glu Thr Cys
1               5                   10                  15
Lys Gly Cys Ala Glu Lys Ser Asp Tyr Ile Arg Lys Ile Asn Glu Leu
            20                  25                  30
Met Pro Lys Tyr Ala
        35

<210> 31
<211> 32
<212> PRT
<213> Artificial Sequence

<220>
<223> peptide

<220>
<221> VARIANT
<222> 1
<223> X16 = absent, S or T

<220>
<221> VARIANT
<222> 2
<223> X17 = absent, V or D

<220>
<221> VARIANT
<222> 3
<223> X18 = absent, D or A

<220>
<221> VARIANT
<222> 4
<223> X19 = absent or L

<220>
<221> VARIANT
<222> 5
<223> X20 = absent, R, K, S or W

<220>
<221> VARIANT
<222> 6
<223> X21 = absent or K

<220>
<221> VARIANT
<222> 7
<223> X22 = absent, M, L, I or V

<220>
<221> VARIANT
<222> 8
<223> X23 = absent or R

<220>
<221> VARIANT
<222> 10
<223> X24 = A, K, T, L or V

<220>
<221> VARIANT
<222> 14
<223> X25 = Q, K or R

<220>
<221> VARIANT
<222> 15
<223> X26 = I or V

<220>
<221> VARIANT
<222> 17
<223> X5 = absent, H, D, Q, R, Y, N or S

<220>
<221> VARIANT

<222> 18
<223> X6 = absent, S, D, G, N or R

<220>
<221> VARIANT
<222> 19
<223> X7 = absent or W

<220>
<221> VARIANT
<222> 20
<223> X8 = absent or G

<220>
<221> VARIANT
<222> 22
<223> X4 = E, T, V, D, M or G

<220>
<221> VARIANT
<222> 24
<223> X1 = R, K, I, G, A or S

<220>
<221> VARIANT
<222> 25
<223> X2 = absent, G, A, R, K, I or S

<220>
<221> VARIANT
<222> 26
<223> X3 = A, G or S

<220>
<221> VARIANT
<222> 30
<223> X9 = absent or K

<220>
<221> VARIANT
<222> 31
<223> X10 = absent, T, S, A, I or N

<220>
<221> VARIANT
<222> 32
<223> X11 = absent, D or E

<400> 31
Xaa Xaa Xaa Xaa Xaa Xaa Xaa Xaa Val Xaa Glu Leu Lys Xaa Xaa Leu
1               5                   10                  15
Xaa Xaa Xaa Xaa Glu Xaa Cys Xaa Xaa Xaa Cys Ala Glu Xaa Xaa Xaa
            20                  25                  30

<210> 32
<211> 32
<212> PRT
<213> Artificial Sequence

<220>

<223> peptide

<220>
<221> VARIANT
<222> 1
<223> X15 = absent, A, S, T, E or N

<220>
<221> VARIANT
<222> 2
<223> X16 = absent, S or T

<220>
<221> VARIANT
<222> 3
<223> X17 = absent, V or D

<220>
<221> VARIANT
<222> 4
<223> X18 = absent, D or A

<220>
<221> VARIANT
<222> 5
<223> X19 = absent or L

<220>
<221> VARIANT
<222> 6
<223> X20 = absent, R, K, S or W

<220>
<221> VARIANT
<222> 7
<223> X21 = absent or K

<220>
<221> VARIANT
<222> 8
<223> X22 = absent, M, L, I or V

<220>
<221> VARIANT
<222> 9
<223> X23 = absent or R

<220>
<221> VARIANT
<222> 9
<223> X23 = absent or R

<220>
<221> VARIANT
<222> 11
<223> X24 = A, K, T, L or V

<220>
<221> VARIANT
<222> 15
<223> X25 = Q, K or R

<220>

<221> VARIANT
<222> 16
<223> X26 = I or V

<220>
<221> VARIANT
<222> 18
<223> X5 = absent, H, D, Q, R, Y, N or S

<220>
<221> VARIANT
<222> 19
<223> X6 = absent, S, D, G, N or R

<220>
<221> VARIANT
<222> 20
<223> X7 = absent or W

<220>
<221> VARIANT
<222> 21
<223> X8 = absent or G

<220>
<221> VARIANT
<222> 23
<223> X4 = E, T, V, D, M or G

<220>
<221> VARIANT
<222> 25
<223> X1 = R, K, I, G, A or S

<220>
<221> VARIANT
<222> 26
<223> X2 = absent, G, A, R, K, I or S

<220>
<221> VARIANT
<222> 27
<223> X3 = A, G or S

<220>
<221> VARIANT
<222> 31
<223> X9 = absent or K

<400> 32
Xaa Xaa Xaa Xaa Xaa Xaa Xaa Xaa Xaa Val Xaa Glu Leu Lys Xaa Xaa
1               5                   10                  15
Leu Xaa Xaa Xaa Xaa Glu Xaa Cys Xaa Xaa Xaa Cys Ala Glu Xaa Xaa
            20                  25                  30

<210> 33
<211> 32
<212> PRT
<213> Artificial Sequence

<220>
<223> peptide

<220>
<221> VARIANT
<222> 1
<223> X14 = absent, L or W

<220>
<221> VARIANT
<222> 2
<223> X15 = absent, A, S, T, E or N

<220>
<221> VARIANT
<222> 3
<223> X16 = absent, S or T

<220>
<221> VARIANT
<222> 4
<223> X17 = absent, V or D

<220>
<221> VARIANT
<222> 5
<223> X18 = absent, D or A

<220>
<221> VARIANT
<222> 6
<223> X19 = absent or L

<220>
<221> VARIANT
<222> 7
<223> X20 = absent, R, K, S or W

<220>
<221> VARIANT
<222> 8
<223> X21 = absent or K

<220>
<221> VARIANT
<222> 9
<223> X22 = absent, M, L, I or V

<220>
<221> VARIANT
<222> 10
<223> X23 = absent or R

<220>
<221> VARIANT
<222> 12
<223> X24 = A, K, T, L or V

<220>
<221> VARIANT
<222> 16
<223> X25 = Q, K or R

<220>
<221> VARIANT
<222> 17
<223> X26 = I or V

<220>
<221> VARIANT
<222> 19
<223> X5 = absent, H, D, Q, R, Y, N or S

<220>
<221> VARIANT
<222> 20
<223> X6 = absent, S, D, G, N or R

<220>
<221> VARIANT
<222> 21
<223> X7 = absent or W

<220>
<221> VARIANT
<222> 22
<223> X8 = absent or G

<220>
<221> VARIANT
<222> 24
<223> X4 = E, T, V, D, M or G

<220>
<221> VARIANT
<222> 26
<223> X1 = R, K, I, G, A or S

<220>
<221> VARIANT
<222> 27
<223> X2 = absent, G, A, R, K, I or S

<220>
<221> VARIANT
<222> 28
<223> X3 = A, G or S

<220>
<221> VARIANT
<222> 32
<223> X9 = absent or K

<400> 33
Xaa Xaa Xaa Xaa Xaa Xaa Xaa Xaa Xaa Xaa Val Xaa Glu Leu Lys Xaa
1               5                   10                  15
Xaa Leu Xaa Xaa Xaa Xaa Glu Xaa Cys Xaa Xaa Xaa Cys Ala Glu Xaa
            20                  25                  30

<210> 34
<211> 32
<212> PRT
<213> Artificial Sequence

<220>
<223> peptide

<220>
<221> VARIANT
<222> 1
<223> X13 = absent or D

<220>
<221> VARIANT
<222> 2
<223> X14 = absent, L or W

<220>
<221> VARIANT
<222> 3
<223> X15 = absent, A, S, T, E or N

<220>
<221> VARIANT
<222> 4
<223> X16 = absent, S or T

<220>
<221> VARIANT
<222> 5
<223> X17 = absent, V or D

<220>
<221> VARIANT
<222> 6
<223> X18 = absent, D or A

<220>
<221> VARIANT
<222> 7
<223> X19 = absent or L

<220>
<221> VARIANT
<222> 8
<223> X20 = absent, R, K, S or W

<220>
<221> VARIANT
<222> 9
<223> X21 = absent or K

<220>
<221> VARIANT
<222> 10
<223> X22 = absent, M, L, I or V

<220>
<221> VARIANT
<222> 11
<223> X23 = absent or R

<220>
<221> VARIANT
<222> 13
<223> X24 = A, K, T, L or V

<220>
<221> VARIANT
<222> 17
<223> X25 = Q, K or R

<220>
<221> VARIANT
<222> 18
<223> X26 = I or V

<220>
<221> VARIANT
<222> 20
<223> X5 = absent, H, D, Q, R, Y, N or S

<220>
<221> VARIANT
<222> 21
<223> X6 = absent, S, D, G, N or R

<220>
<221> VARIANT
<222> 22
<223> X7 = absent or W

<220>
<221> VARIANT
<222> 23
<223> X8 = absent or G

<220>
<221> VARIANT
<222> 25
<223> X4 = E, T, V, D, M or G

<220>
<221> VARIANT
<222> 27
<223> X1 = R, K, I, G, A or S

<220>
<221> VARIANT
<222> 28
<223> X2 = absent, G, A, R, K, I or S

<220>
<221> VARIANT
<222> 29
<223> X3 = A, G or S

<400> 34
Xaa Xaa Xaa Xaa Xaa Xaa Xaa Xaa Xaa Xaa Xaa Val Xaa Glu Leu Lys
1               5                   10                  15
Xaa Xaa Leu Xaa Xaa Xaa Xaa Glu Xaa Cys Xaa Xaa Xaa Cys Ala Glu
            20                  25                  30


<210> 35
<211> 4
<212> PRT
<213> Artificial Sequence

<220>
<223> peptide

<400> 35
Lys Asp Glu Leu
1

<210> 36
<211> 4
<212> PRT
<213> Artificial Sequence

<220>
<223> peptide

<400> 36
Cys Arg Ala Cys
1

<210> 37
<211> 4
<212> PRT
<213> Artificial Sequence

<220>
<223> peptide

<400> 37
Cys Lys Gly Cys
1

<210> 38
<211> 4
<212> PRT
<213> Artificial Sequence

<220>
<223> peptide

<220>
<221> VARIANT
<222> 2
<223> X1 = K or R

<220>
<221> VARIANT
<222> 3
<223> X2 = G or A

<400> 38
Cys Xaa Xaa Cys
1

<210> 39
<211> 8
<212> PRT
<213> Artificial Sequence

<220>
<223> peptide

<400> 39
Glu Thr Cys Lys Gly Cys Ala Glu
1               5

<210> 40
<211> 6
<212> PRT
<213> Artificial Sequence


<220>
<223> peptide

<400> 40
Thr Cys Lys Gly Cys Ala
1               5

<210> 41
<211> 187
<212> PRT
<213> Homo sapiens


<220>
<223> NCBI Reference Sequence: NP_001025125.2

<400> 41
Met Trp Cys Ala Ser Pro Val Ala Val Val Ala Phe Cys Ala Gly Leu
1               5                   10                  15
Leu Val Ser His Pro Val Leu Thr Gln Gly Gln Glu Ala Gly Gly Arg
            20                  25                  30
Pro Gly Ala Asp Cys Glu Val Cys Lys Glu Phe Leu Asn Arg Phe Tyr
        35                  40                  45
Lys Ser Leu Ile Asp Arg Gly Val Asn Phe Ser Leu Asp Thr Ile Glu
    50                  55                  60
Lys Glu Leu Ile Ser Phe Cys Leu Asp Thr Lys Gly Lys Glu Asn Arg
65                  70                  75                  80
Leu Cys Tyr Tyr Leu Gly Ala Thr Lys Asp Ala Ala Thr Lys Ile Leu
                85                  90                  95
Ser Glu Val Thr Arg Pro Met Ser Val His Met Pro Ala Met Lys Ile
            100                 105                 110
Cys Glu Lys Leu Lys Lys Leu Asp Ser Gln Ile Cys Glu Leu Lys Tyr
        115                 120                 125
Glu Lys Thr Leu Asp Leu Ala Ser Val Asp Leu Arg Lys Met Arg Val
    130                 135                 140
Ala Glu Leu Lys Gln Ile Leu His Ser Trp Gly Glu Glu Cys Arg Ala
145                 150                 155                 160
Cys Ala Glu Lys Thr Asp Tyr Val Asn Leu Ile Gln Glu Leu Ala Pro
                165                 170                 175
Lys Tyr Ala Ala Thr His Pro Lys Thr Glu Leu
            180                 185

<210> 42
<211> 182
<212> PRT
<213> Homo sapiens


<220>
<223> NCBI Reference Sequence: NP_006001.5

<400> 42
Met Arg Arg Met Trp Ala Thr Gln Gly Leu Ala Val Ala Leu Ala Leu
1               5                   10                  15
Ser Val Leu Pro Gly Ser Arg Ala Leu Arg Pro Gly Asp Cys Glu Val
            20                  25                  30
Cys Ile Ser Tyr Leu Gly Arg Phe Tyr Gln Asp Leu Lys Asp Arg Asp
        35                  40                  45
Val Thr Phe Ser Pro Ala Thr Ile Glu Asn Glu Leu Ile Lys Phe Cys
    50                  55                  60
Arg Glu Ala Arg Gly Lys Glu Asn Arg Leu Cys Tyr Tyr Ile Gly Ala
65                  70                  75                  80
Thr Asp Asp Ala Ala Thr Lys Ile Ile Asn Glu Val Ser Lys Pro Leu
                85                  90                  95
Ala His His Ile Pro Val Glu Lys Ile Cys Glu Lys Leu Lys Lys Lys
            100                 105                 110
Asp Ser Gln Ile Cys Glu Leu Lys Tyr Asp Lys Gln Ile Asp Leu Ser
        115                 120                 125
Thr Val Asp Leu Lys Lys Leu Arg Val Lys Glu Leu Lys Lys Ile Leu
    130                 135                 140
Asp Asp Trp Gly Glu Thr Cys Lys Gly Cys Ala Glu Lys Ser Asp Tyr
145                 150                 155                 160
Ile Arg Lys Ile Asn Glu Leu Met Pro Lys Tyr Ala Pro Lys Ala Ala
                165                 170                 175
Ser Ala Arg Thr Asp Leu
            180

<210> 43
<211> 49
<212> PRT
<213> Homo sapiens


<400> 43
Thr Leu Asp Leu Ala Ser Val Asp Leu Arg Lys Met Arg Val Ala Glu
1               5                   10                  15
Leu Lys Gln Ile Leu His Ser Trp Gly Glu Glu Cys Arg Ala Cys Ala
            20                  25                  30
Glu Lys Thr Asp Tyr Val Asn Leu Ile Gln Glu Leu Ala Pro Lys Tyr
        35                  40                  45
Ala


<210> 44
<211> 37
<212> PRT
<213> Homo sapiens


<400> 44
Arg Val Ala Glu Leu Lys Gln Ile Leu His Ser Trp Gly Glu Glu Cys
1               5                   10                  15
Arg Ala Cys Ala Glu Lys Thr Asp Tyr Val Asn Leu Ile Gln Glu Leu
            20                  25                  30
Ala Pro Lys Tyr Ala
        35

<210> 45
<211> 35
<212> PRT
<213> Homo sapiens

<400> 45
Thr Leu Asp Leu Ala Ser Val Asp Leu Arg Lys Met Arg Val Ala Glu
1               5                   10                  15
Leu Lys Gln Ile Leu His Ser Trp Gly Glu Glu Cys Arg Ala Cys Ala
            20                  25                  30
Glu Lys Thr
        35

<210> 46
<211> 32
<212> PRT
<213> Homo sapiens

<400> 46
Leu Ala Ser Val Asp Leu Arg Lys Met Arg Val Ala Glu Leu Lys Gln
1               5                   10                  15
Ile Leu His Ser Trp Gly Glu Glu Cys Arg Ala Cys Ala Glu Lys Thr
            20                  25                  30

<210> 47
<211> 49
<212> PRT
<213> Homo sapiens

<400> 47
Gln Ile Asp Leu Ser Thr Val Asp Leu Lys Lys Leu Arg Val Lys Glu
1               5                   10                  15
Leu Lys Lys Ile Leu Asp Asp Trp Gly Glu Thr Cys Lys Gly Cys Ala
            20                  25                  30
Glu Lys Ser Asp Tyr Ile Arg Lys Ile Asn Glu Leu Met Pro Lys Tyr
        35                  40                  45
Ala

<210> 48
<211> 37
<212> PRT
<213> Homo sapiens

<400> 48
Arg Val Lys Glu Leu Lys Lys Ile Leu Asp Asp Trp Gly Glu Thr Cys
1               5                   10                  15
Lys Gly Cys Ala Glu Lys Ser Asp Tyr Ile Arg Lys Ile Asn Glu Leu
            20                  25                  30
Met Pro Lys Tyr Ala
        35

<210> 49
<211> 35
<212> PRT
<213> Homo sapiens

<400> 49
Gln Ile Asp Leu Ser Thr Val Asp Leu Lys Lys Leu Arg Val Lys Glu
1               5                   10                  15
Leu Lys Lys Ile Leu Asp Asp Trp Gly Glu Thr Cys Lys Gly Cys Ala
            20                  25                  30

Glu Lys Ser
        35

<210> 50
<211> 32
<212> PRT
<213> Homo sapiens


<400> 50
Leu Ser Thr Val Asp Leu Lys Lys Leu Arg Val Lys Glu Leu Lys Lys
1               5                   10                  15
Ile Leu Asp Asp Trp Gly Glu Thr Cys Lys Gly Cys Ala Glu Lys Ser
            20                  25                  30


<210> 51
<211> 61
<212> PRT
<213> Homo sapiens


<220>
<223> Human CDNF (NP_001025125.2)

<400> 51
Lys Tyr Glu Lys Thr Leu Asp Leu Ala Ser Val Asp Leu Arg Lys Met
1               5                   10                  15
Arg Val Ala Glu Leu Lys Gln Ile Leu His Ser Trp Gly Glu Glu Cys
            20                  25                  30
Arg Ala Cys Ala Glu Lys Thr Asp Tyr Val Asn Leu Ile Gln Glu Leu
        35                  40                  45
Ala Pro Lys Tyr Ala Ala Thr His Pro Lys Thr Glu Leu
    50                  55                  60

<210> 52
<211> 61
<212> PRT
<213> Equus


<220>
<223> Horse CDNF (XP_001498617.2)

<400> 52
Lys Tyr Glu Lys Lys Leu Asp Leu Ala Ser Val Asp Leu Leu Lys Met
1               5                   10                  15
Arg Val Ala Glu Leu Lys Gln Ile Leu Asn Ser Trp Gly Glu Glu Cys
            20                  25                  30
Arg Ala Cys Ala Glu Lys Ser Asp Tyr Val Asn Leu Ile Lys Glu Leu
        35                  40                  45
Ala Pro Lys Tyr Ala Ala Met His Pro Lys Thr Glu Leu
    50                  55                  60

<210> 53
<211> 61
<212> PRT
<213> Bos


<220>
<223> Bison CDNF (XP_010858254.1)

<400> 53
Lys Tyr Glu Lys Lys Leu Asp Leu Ala Ser Val Asp Leu Ser Lys Met
1               5                   10                  15
Arg Val Ala Glu Leu Lys Gln Ile Leu His Gly Trp Gly Glu Glu Cys
            20                  25                  30
Arg Ala Cys Ala Glu Lys Thr Asp Tyr Val Asn Leu Ile Lys Glu Leu
        35                  40                  45
Ala Pro Lys Tyr Ala Ala Thr His Pro Gln Thr Glu Leu
    50                  55                  60

<210> 54
<211> 61
<212> PRT
<213> Sus

<220>
<223> Pig CDNF (XP_003130787.1)

<400> 54
Lys Tyr Glu Lys Lys Leu Asp Leu Ala Ser Val Asp Leu Ser Lys Met
1               5                   10                  15
Arg Val Ala Glu Leu Lys Gln Ile Leu Tyr Ser Trp Gly Glu Glu Cys
            20                  25                  30
Arg Ala Cys Ala Glu Lys Thr Asp Tyr Val Asn Leu Ile Lys Glu Leu
        35                  40                  45
Ala Pro Lys Tyr Thr Glu Thr Pro Pro Gln Thr Glu Leu
    50                  55                  60

<210> 55
<211> 61
<212> PRT
<213> Canis

<220>
<223> Dog CDNF (XP_848954.2)

<400> 55
Lys Tyr Glu Lys Lys Leu Asp Leu Ala Ser Val Asp Leu Ser Lys Met
1               5                   10                  15
Arg Val Ala Glu Leu Lys Gln Ile Leu His Ser Trp Gly Glu Glu Cys
            20                  25                  30
Ile Ala Cys Ala Glu Lys Thr Asp Tyr Val Asn Leu Ile Thr Glu Leu
        35                  40                  45
Ala Pro Lys Tyr Ala Ala Ala His Pro Lys Thr Glu Leu
    50                  55                  60

<210> 56
<211> 61
<212> PRT
<213> Musculus

<220>
<223> Mouse CDNF (NP_808315.1)

<400> 56
Lys Tyr Gly Lys Lys Leu Asp Leu Ala Ser Val Asp Leu Trp Lys Met
1               5                   10                  15
Arg Val Ala Glu Leu Lys Gln Ile Leu Gln Arg Trp Gly Glu Glu Cys
            20                  25                  30
Arg Ala Cys Ala Glu Lys Ser Asp Tyr Val Asn Leu Ile Arg Glu Leu

        35                      40                      45
Ala Pro Lys Tyr Val Glu Ile Tyr Pro Gln Thr Glu Leu
    50                      55                      60

<210> 57
<211> 61
<212> PRT
<213> Cricetus

<220>
<223> Hamster CDNF (XP_027261009.1)

<400> 57
Asn Tyr Glu Lys Lys Leu Asp Leu Ala Ser Val Asp Leu Trp Lys Met
1               5                   10                  15
Arg Asp Ala Glu Leu Lys Gln Ile Leu His Ser Trp Gly Glu Glu Cys
            20                  25                  30
Arg Ala Cys Ala Glu Lys Asn Asp Tyr Val Asn Leu Ile Lys Glu Leu
        35                      40                      45
Ala Pro Lys Tyr Val Glu Ile His Pro Gln Ile Glu Leu
    50                      55                      60

<210> 58
<211> 61
<212> PRT
<213> Alligator

<220>
<223> Alligator CDNF (XP_019343086.1)

<400> 58
Lys Tyr Glu Arg Lys Leu Asp Leu Thr Ser Val Asp Leu Ser Lys Met
1               5                   10                  15
Arg Val Ala Glu Leu Arg Lys Ile Leu Asp Ser Trp Gly Glu Val Cys
            20                  25                  30
Lys Ala Cys Ile Glu Lys Thr Glu Phe Val Asn Leu Ile Lys Glu Leu
        35                      40                      45
Ala Pro Lys Tyr Ala Pro Pro Asn Ser Arg Ala Asp Leu
    50                      55                      60

<210> 59
<211> 61
<212> PRT
<213> Delphinus

<220>
<223> Dolphin CDNF (XP_026977721.1)

<400> 59
Lys Tyr Glu Lys Lys Leu Asp Leu Ala Ser Val Asp Leu Ser Lys Met
1               5                   10                  15
Arg Val Ala Glu Leu Lys Gln Ile Leu Tyr Ser Trp Gly Glu Glu Cys
            20                  25                  30
Arg Ala Cys Val Glu Lys Thr Asp Tyr Val Asn Leu Ile Lys Glu Leu
        35                      40                      45
Ala Pro Lys Tyr Thr Ala Thr Tyr Pro Lys Thr Glu Leu
    50                      55                      60

<210> 60
<211> 65

<212> PRT
<213> Danio rerio

<220>
<223> Zebrafish CDNF (NP_001116753.1)

<400> 60
Arg Tyr Glu Arg Leu Val Leu Asp Trp Ser Thr Asp Ala Leu Ser Lys
1               5                   10                  15
Met Arg Ala Leu Glu Leu Lys Arg Val Leu Ala Ser Trp Gly Glu Glu
            20                  25                  30
Cys Arg Ala Cys Leu Glu Lys Ser Glu Phe Ile Ala Leu Ile Gln Glu
        35                  40                  45
Val Ala Pro Lys His Ser Ala Ser Glu His Arg Ala His Thr Glu Glu
    50                  55                  60
Phe
65

<210> 61
<211> 63
<212> PRT
<213> Homo sapiens

<220>
<223> Human MANF (NP_006001.5)

<400> 61
Lys Tyr Asp Lys Gln Ile Asp Leu Ser Thr Val Asp Leu Lys Lys Leu
1               5                   10                  15
Arg Val Lys Glu Leu Lys Lys Ile Leu Asp Asp Trp Gly Glu Thr Cys
            20                  25                  30
Lys Gly Cys Ala Glu Lys Ser Asp Tyr Ile Arg Lys Ile Asn Glu Leu
        35                  40                  45
Met Pro Lys Tyr Ala Pro Lys Ala Ala Ser Ala Arg Thr Asp Leu
    50                  55                  60

<210> 62
<211> 63
<212> PRT
<213> Equus

<220>
<223> Horse MANF (NP_001184244.1)

<400> 62
Lys Tyr Asp Lys Gln Ile Asp Leu Ser Thr Val Asp Leu Lys Lys Leu
1               5                   10                  15
Arg Val Lys Glu Leu Lys Lys Ile Leu Asp Asp Trp Gly Glu Thr Cys
            20                  25                  30
Lys Gly Cys Ala Glu Lys Ser Asp Tyr Ile Arg Lys Ile Asn Glu Leu
        35                  40                  45
Met Pro Lys Tyr Ala Pro Lys Ala Ala Ser Ser Arg Thr Asp Leu
    50                  55                  60

<210> 63
<211> 63
<212> PRT
<213> Bison

<220>
<223> Bison MANF (XP_010850093.1)

<400> 63
Lys Tyr Asp Lys Gln Ile Asp Leu Ser Thr Val Asp Leu Lys Lys Leu
1               5                   10                  15
Arg Val Lys Glu Leu Lys Lys Ile Leu Asp Asp Trp Gly Glu Thr Cys
            20                  25                  30
Lys Gly Cys Ala Glu Lys Ser Asp Tyr Ile Arg Lys Ile Asn Glu Leu
        35                  40                  45
Met Pro Lys Tyr Ala Pro Lys Ala Ala Ser Ser Arg Thr Asp Leu
    50                  55                  60

<210> 64
<211> 63
<212> PRT
<213> Sus

<220>
<223> Pig MANF (NP_001231584.1)

<400> 64
Lys Tyr Asp Lys Gln Ile Asp Leu Ser Thr Val Asp Leu Lys Lys Leu
1               5                   10                  15
Arg Val Lys Glu Leu Lys Lys Ile Leu Asp Asp Trp Gly Glu Thr Cys
            20                  25                  30
Lys Gly Cys Ala Glu Lys Ser Asp Tyr Ile Arg Lys Ile Asn Glu Leu
        35                  40                  45
Met Pro Lys Tyr Ala Pro Lys Ala Ala Ser Ser Arg Thr Asp Leu
    50                  55                  60

<210> 65
<211> 63
<212> PRT
<213> Canis

<220>
<223> Dog MANF (XP_003639808.2)

<400> 65
Lys Tyr Asp Lys Gln Ile Asp Leu Arg Thr Val Asp Leu Lys Lys Leu
1               5                   10                  15
Arg Val Arg Glu Leu Lys Lys Ile Leu Asp Asp Trp Gly Glu Thr Cys
            20                  25                  30
Lys Gly Cys Ala Glu Lys Ser Asp Tyr Ile Arg Lys Ile Asn Glu Leu
        35                  40                  45
Met Pro Lys Tyr Ala Pro Lys Ala Ala Ser Ser Arg Thr Asp Leu
    50                  55                  60

<210> 66
<211> 63
<212> PRT
<213> Musculus

<220>
<223> Mouse MANF (NP_083379.2)

<400> 66
Lys Tyr Asp Lys Gln Ile Asp Leu Ser Thr Val Asp Leu Lys Lys Leu
1               5                   10                  15

Arg Val Lys Glu Leu Lys Lys Ile Leu Asp Asp Trp Gly Glu Met Cys
            20                  25                  30
Lys Gly Cys Ala Glu Lys Ser Asp Tyr Ile Arg Lys Ile Asn Glu Leu
        35                  40                  45
Met Pro Lys Tyr Ala Pro Lys Ala Ala Ser Ala Arg Thr Asp Leu
    50                  55                  60

<210> 67
<211> 63
<212> PRT
<213> Cricetus

<220>
<223> Hamster MANF (RLQ67668)

<400> 67
Lys Tyr Asp Lys Gln Ile Asp Leu Ser Thr Val Asp Leu Lys Lys Leu
1               5                   10                  15
Arg Val Lys Glu Leu Lys Lys Ile Leu Asp Asp Trp Gly Glu Met Cys
            20                  25                  30
Lys Gly Cys Ala Glu Lys Ser Asp Tyr Ile Arg Lys Ile Asn Glu Leu
        35                  40                  45
Met Pro Lys Tyr Ala Pro Lys Ala Ala Ser Ala Arg Thr Asp Leu
    50                  55                  60

<210> 68
<211> 63
<212> PRT
<213> Alligator

<220>
<223> Alligator MANF (XP_014455597.1)

<400> 68
Lys Tyr Asp Lys Gln Ile Asp Leu Ser Thr Val Asp Leu Lys Lys Leu
1               5                   10                  15
Arg Val Lys Glu Leu Lys Lys Ile Leu Asp Asp Trp Gly Glu Thr Cys
            20                  25                  30
Lys Gly Cys Ala Glu Lys Ser Asp Tyr Ile Arg Lys Ile Asn Glu Leu
        35                  40                  45
Met Pro Lys Tyr Ala Pro Lys Ala Ala Ser Ser Arg Thr Asp Leu
    50                  55                  60

<210> 69
<211> 63
<212> PRT
<213> Delphinus

<220>
<223> Dolphin MANF (XP_026976745.1)

<400> 69
Lys Tyr Asp Lys Gln Ile Asp Leu Ser Thr Val Asp Leu Lys Lys Leu
1               5                   10                  15
Arg Val Lys Glu Leu Lys Lys Ile Leu Asp Asp Trp Gly Glu Thr Cys
            20                  25                  30
Lys Gly Cys Ala Glu Lys Ser Asp Tyr Ile Arg Lys Ile Asn Glu Leu
        35                  40                  45
Met Pro Lys Tyr Ala Pro Lys Ala Ala Ser Ser Arg Thr Asp Leu
    50                  55                  60

<210> 70
<211> 63
<212> PRT
<213> Danio rerio

<220>
<223> Zebrafish MANF (NP_001070097.1)

<400> 70
Lys Tyr Asp Lys Gln Val Asp Leu Ser Ser Val Asp Leu Lys Lys Leu
1               5                   10                  15
Lys Val Lys Asp Leu Lys Lys Ile Leu Glu Glu Trp Gly Glu Ser Cys
            20                  25                  30
Lys Gly Cys Val Glu Lys Ser Asp Phe Ile Arg Lys Ile Asn Glu Leu
        35                  40                  45
Met Pro Lys Tyr Ala Pro Ser Ala Ala Lys Ala Arg Thr Asp Leu
    50                  55                  60

## Description

### FIELD OF THE DISCLOSURE

The present disclosure relates to the field of unconventional neurotrophic factors and endoplasmic reticulum (ER) located proteins and to the field of treating degenerative, chronic or progressive diseases and disorders, and monogenic hereditary diseases having ER stress as a pathogenic compound. More particularly the disclosure relates to macrocyclic peptides. The disclosure also relates to pharmaceutical compositions comprising said peptides. Further, the disclosure also relates to said peptides and pharmaceutical compositions for use as a medicament and in the treatment of degenerative, chronic or progressive diseases and disorders, and monogenic hereditary diseases having ER stress as a pathogenic compound as well as to methods for treating said diseases and disorders.

### BACKGROUND OF THE DISCLOSURE

Neurotrophic factors (NTF) are a subgroup of growth factors that promote survival and differentiation of neurons and have neuroprotective and neurorestorative properties (Hefti, 1994). NTFs are small proteins that support the growth, survival and differentiation of developing and mature neurons, and protect them from injury and toxins. Cerebral dopamine neurotrophic (CDNF), together with its closest relative mesencephalic astrocyte-derived neurotrophic factor (MANF), form a novel family of unconventional NTF that are both structurally and mechanistically distinct from other growth factors (Lindholm and Saarma, 2010; Huttunen and Saarma, 2019). CDNF and MANF are small monomeric proteins with a molecular weight of approximately 18 kDa, mature proteins 161 and 158 amino acids, respectively, that are expressed in the central nervous system but also in non-neuronal tissues. CDNF and MANF are localized mainly to the lumen of endoplasmic reticulum (ER). They contain an N-terminal signal peptide that directs them to the ER. Both CDNF and MANF also contain a C-terminal KDEL (SEQ ID NO: 35)-like ER-retention signal that is typically absent in growth factors destined for secretion. They interact with ER proteins such as BiP/GRP78, modulate unfolded protein response (UPR) signaling and protect from ER stress-induced cell death. Both CDNF and MANF accumulate in the ER lumen in healthy cells and disruption of the C-terminal ER-retention signal results in their secretion. Detectable levels of CDNF and MANF are found in normal human serum, and MANF also in cerebrospinal fluid (CSF). Based on these characteristics, CDNF and MANF are considered to be general stress-protective proteins rather than highly specific neurotrophic factors (Huttunen and Saarma, 2019). MANF has also been described as a cardiomyokine (Glembotski, 2011).

CDNF and MANF are currently the most efficient proteins for the treatment of degenerating dopamine neurons in the rat 6-OHDA model of Parkinson's disease (Lindholm and Saarma, 2010). Both factors potently prevent the 6-OHDA-induced loss of dopamine neurons of the Parkinson's disease-like motor symptoms when applied before the toxin (Lindholm et al., 2007; Voutilainen et al., 2009). More importantly, post-lesion administration of either factor efficiently restored the normal motor behavior and dopaminergic innervations of the striatum when applied at the stage when the 6-OHDA-induced symptoms of the Parkinson's disease are already far-reaching (Lindholm et al., 2007; Voutilainen et al., 2011). CDNF protects and repairs dopamine neurons also in mouse MPTP model of Parkinson's disease (Airavaara et al., 2012), and in a severe 6-OHDA model it is more efficient than glial cell line-derived neurotrophic factor (GDNF) (Airavaara et al., 2012; Voutilainen et al 2011). The mechanisms behind the neuronal protection for these factors are not fully clear but it has been suggested they activate pathways, which aim at alleviating oxidative- and ER stress and depressing apoptotic cell death. Many pathophysiological conditions including diabetes and neurodegenerative diseases such as Parkinson's disease, Alzheimer's disease (AD) and amyotrophic lateral sclerosis (ALS) are associated with ER stress. Accordingly, the effect of CDNF and MANF has been shown in various central nervous system diseases (WO2009133247; WO2007068803; and Airavaara et al, 2009). Non-cell autonomous mechanisms, including modulation of responses of immune and glial cells, have been shown to contribute to the cell-protective effects of CDNF and MANF (Sousa-Victor et al, 2018). Specifically, CDNF and MANF have been shown to suppress neuroinflammation, which is involved in the pathophysiology of most if not all CNS diseases and injuries (Nadella et al, 2014; Zhao et al, 2013).

CDNF and MANF share ca. 60% amino acid sequence homology, but they have highly similar three-dimensional structures. Both CDNF and MANF are composed of two independently folded domains connected by a flexible loop region (Lindholm and Saarma, 2010). The secondary structure is predominantly a α-helical, with five α-helices in the N-terminal domain, and three α-helices in the C-terminal domain. Three disulphide bridges stabilize the N-terminal domain while the C-terminal CRAC (SEQ ID NO: 36) sequence, CKGC (SEQ ID NO: 37) in MANF, forms an internal disulphide bridge. This CXXC (SEQ ID NO: 38) disulphide bridge is found in both in CDNF and MANF and plays a central role in the cytoprotective activity of these proteins.

CDNF is expressed in the brain but also in a number of other tissues, including e.g. skeletal muscle, liver, heart, lung, pancreas, testis, salivary gland and enteric nervous system (Lindholm et al, 2007). MANF is also expressed in the brain but also in peripheral tissues such as the pancreas and heart.

Natural peptides such as those disclosed in publications WO 2013/3034805 and WO 2018/202957 can rarely be used as pharmaceutical products.

Document WO 2013/3034805 A1 discloses MANF and CDNF fragments with the length of 4 - 40 amino acids comprising the sequence CKGC (SEQ ID NO: 37) or CRAC (SEQ ID NO: 36). Document WO 2018/202957 A1 discloses CDNF fragments which have the length of at least 50 amino acids.

Hellmann et al., 2011, disclose an active C-terminal fragment construct of MANF comprising residues 96-158. Fletcher and Hughes 2006 disclose a CRAC (SEQ ID NO: 36)-containing brain-drived neurotrophic factor (BDNF)-derived peptide with engineered cysteines for loop generation. Therefore, there remains a need in the art for therapeutics with improved metabolic stability and distribution properties.

### BRIEF DESCRIPTION OF THE INVENTION

An aim of the present disclosure is to provide novel modified macrocyclic peptides. Another aim of the present disclosure is to provide uses of said novel peptides.

The present disclosure provides tools with these aforementioned properties by utilizing, especially macrocyclic peptides in a novel and inventive way.

The present inventors found that linear native CDNF and MANF peptides are poor drug molecules due to their quick metabolism and poor distribution when administered to humans or animals, particularly with parenteral administration. Therefore, natural unmodified peptides such as those disclosed in the prior art can rarely be used as pharmaceutical products. The present inventors developed novel stabilized peptides derived from CDNF and MANF that recapitulate the cell-protective effects of CDNF and MANF but are well-suited for non-invasive peripheral administration. The present inventors found that macrocyclization of CDNF and MANF peptides significantly improves their metabolic stability and distribution properties without loss of their cell-protective activity, as shown by the data of the present disclosure. Also, the present modified peptides are shorter than those disclosed in the prior art.

The biological activity of CDNF/MANF is localized to the C-terminal domain of the protein. The present disclosure describes 8-32 amino acid peptides derived from the C-terminal domain of CDNF and MANF, specifically in a macrocyclic form. Short linear octapeptides around the CXXC (SEQ ID NO: 38) motif showed cell-protective activity comparable to full-length protein and ability to penetrate cell membranes and experimental blood-brain barrier in *in vitro* models as disclosed herein.

The present inventors show for the first time ever that short macrocyclic peptides, such as head-to-tail cyclized CDNF/MANF macrocyclic peptides having a CXXC (SEQ ID NO: 38) motif, or a specific type of CXXXC (SEQ ID NO: 25) motif, have significantly improved pharmaceutical properties, e.g. metabolic stability, blood-brain barrier (BBB) penetration and *in vivo* pharmacokinetics. These short and semi-short macrocyclic peptides may be used for developing medicaments for degenerative, chronic and/or progressive diseases and disorders or monogenic hereditary diseases having ER stress as a pathogenic component.

The present disclosure provides a macrocyclic peptide with a length of 8 - 32 amino acids or a pharmaceutically acceptable salt thereof, comprising an amino acid sequence of C-X₁-X₂-X₃-C (SEQ ID NO: 25),
wherein
X₁ is selected from the group consisting of R, K, I, G, A and S;
X₂ is absent or selected from the group consisting of G, A, R, K, I and S; and
X₃ is selected from the group consisting of A, G and S.

In some embodiments, the macrocyclic peptide is a peptide wherein the N-terminal of the peptide is linked to the C-terminus of the peptide (i.e., "head-to-tail linkage"). In some instances, the peptide is a pseudopeptide. In some instances, the peptide has at least one (e.g., 1, 2, 3, 4, 5, 6, or 7) of the following properties: (i) the peptide can dose-dependently protect TH-positive neurons from MPP⁺ toxicity; (ii) the peptide reduces the number of a-synuclein inclusions in TH-positive neurons; (iii) the peptide has improved stability in plasma compared to its linear counterpart; (iv) the peptide has improved stability in hepatocytes compared to its linear counterpart; or (v) the peptide has improved ability to pass through the blood brain barrier compared to its linear counterpart.

The present disclosure further provides said macrocyclic peptide for use as a medicament.

The present disclosure further provides said macrocyclic peptide for use in the treatment of a degenerative, chronic and/or progressive diseases and disorders such as a neurodegenerative disease or disorder, or monogenic hereditary diseases having ER stress as a pathogenic component.

The present disclosure further provides a pharmaceutical composition comprising said macrocyclic peptide and at least one of the following: a pharmaceutically acceptable carrier, a pharmaceutically acceptable excipient, a preservative, a stabilizer and/or a diluent.

The present disclosure further provides the pharmaceutical composition for use as a medicament.

The present disclosure further provides a pharmaceutical composition for use in the treatment of a degenerative, chronic and/or progressive diseases and disorders such as a neurodegenerative disease or disorder, or monogenic hereditary diseases having ER stress as a pathogenic component.
The present disclosure further provides a method for treating a degenerative, chronic, or progressive disease or disorder, such as a neurodegenerative disease or disorder, or monogenic hereditary diseases having ER stress as a pathogenic component in a subject in need thereof, the method comprising administering to the subject a pharmaceutical composition comprising the aforementioned macrocyclic peptide(s).

In the following the invention will be described in more detail by means of preferred embodiments.

### BRIEF DESCRIPTION OF THE FIGURES

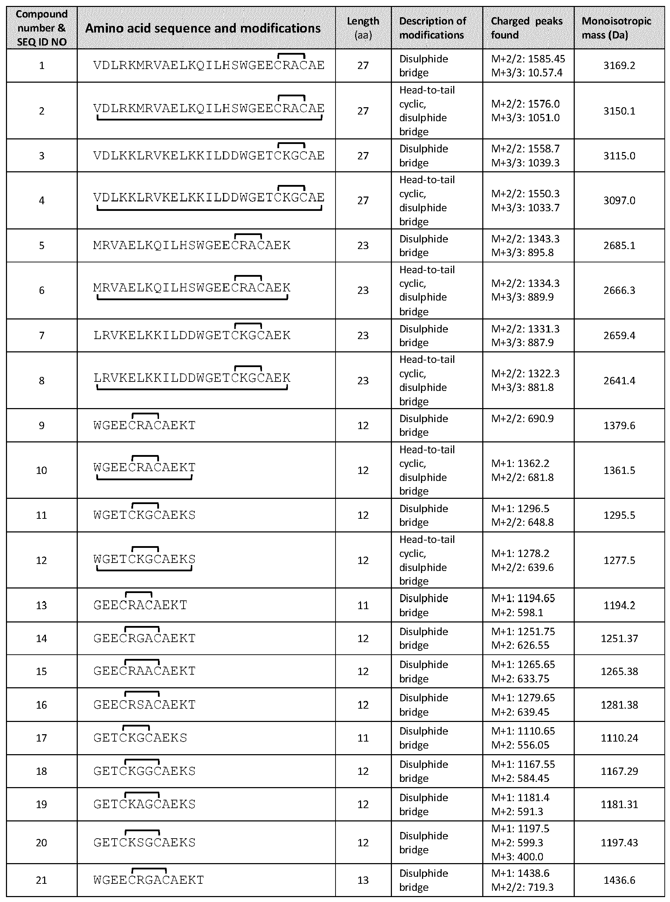

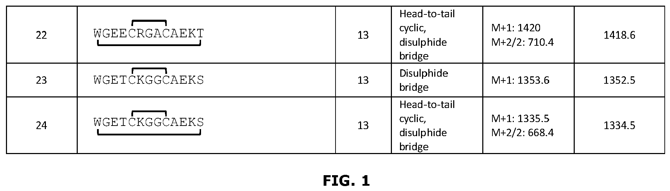

FIG. 1 shows a list of compounds studied. Compound number, SEQ ID NO, amino acid sequence indicating the cyclization scheme and the Cys-Cys sulphide bond, length of the sequence, description of modifications, and monoisotropic mass (Da) are presented. Column 5 shows the detailed charged mass peaks seen in the MS spectra and column 6 presents the monoisotropic mass of the compounds. The difference of -18 Da (mass of a water molecule) in the monoisotropic mass between the linear and cyclic form of the same peptides confirms the cyclization reaction being done successfully.

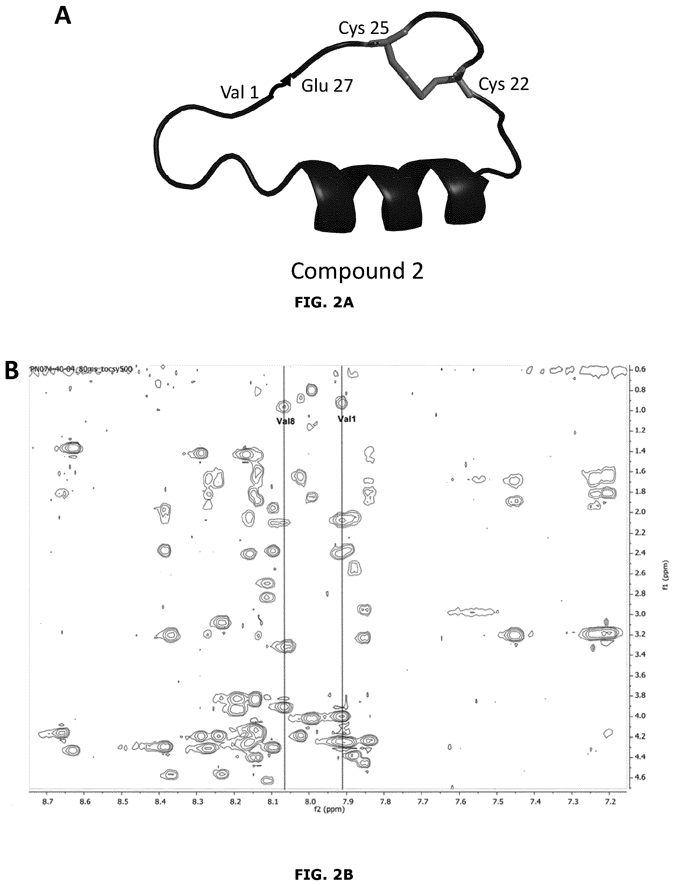

**FIGs. 2A-2P** shows biophysical characterization of the macrocyclic compounds 2 (SEQ ID NO: 2), compound 4 (SEQ ID NO: 4), compound 6 (SEQ ID NO: 6), compound 8 (SEQ ID NO: 8), compound 10 (SEQ ID NO: 10), compound 12 (SEQ ID NO: 12), compound 22 (SEQ ID NO: 22) and compound 24 (SEQ ID NO: 24).
**FIG. 2A** presents the compound 2 (SEQ ID NO: 2), showing the ring closing zone by an arrow between the corresponding amino-terminal (head; Val1) and carboxyl-terminal (tail; Glu27) residues. The disulphide bridge-forming cysteines are shown in gray with stick representation and labelled accordingly.
**FIG. 2B** shows a contour plot of the number of observed amide protons in the 2D ¹H NMR TOCSY amide fingerprint for compound 2 (SEQ ID NO: 2). The horizontal axis to be the direct dimension (F2, presented in ppm) and the vertical axis to be the indirect dimension (F1, presented in ppm). The number of observed amide protons in the TOCSY data is consistent with a 27 amino acid long cyclic peptide i.e. the Val1 amide proton is observed, in consistency with the anticipated amidation of the N-terminal, as opposed to its linear analogue where the N-terminal amide proton is not observed. The Val1 and Val8 protons are assigned and indicated by the straight lines corresponding to H^{N} 7.9 ppm and 8.05, respectively.

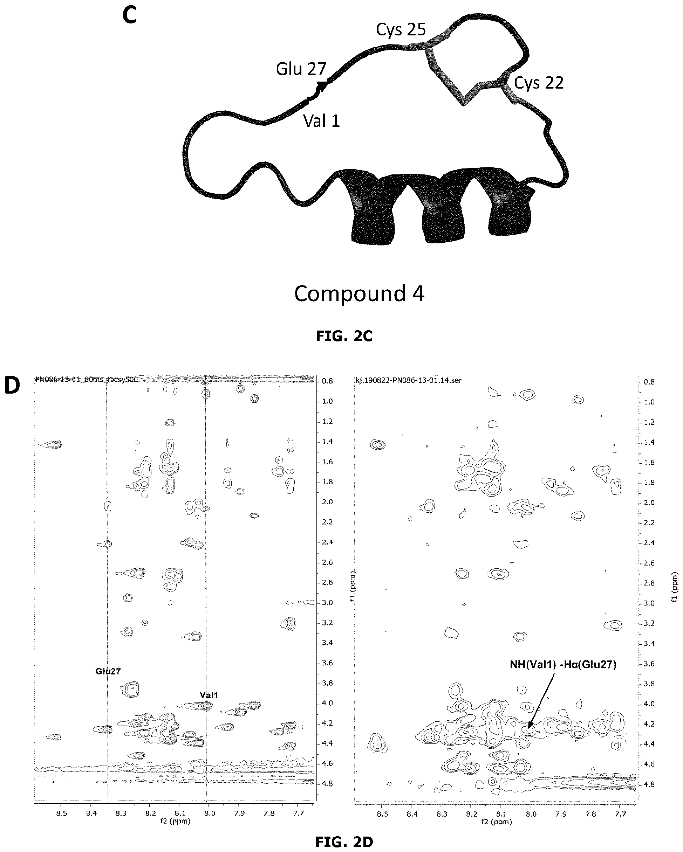

**FIG. 2C** presents compound 4 (SEQ ID NO: 4), showing the ring closing zone by an arrow between the corresponding amino-terminal (Val1) and carboxyl-terminal (Glu27) residues. The disulphide bridge-forming cysteines are shown in gray with stick representation and labelled accordingly.
**FIG. 2D** shows a contour plot of the number of observed amide protons in the 2D ¹H NMR TOCSY amide fingerprint (left panel) for compound 4 (SEQ ID NO: 4). The horizontal axis to be the direct dimension (F2, presented in ppm) and the vertical axis to be the indirect dimension (F1, presented in ppm). The number of observed amide protons in the TOCSY data is consistent with a 27 amino acid long cyclic peptide i.e. the Val1 amide proton is observed, in consistency with the anticipated amidation of the N-terminal, as opposed to its linear analogue where the N-terminal amide proton is not observed. ¹H chemical shifts for 1st (Val1) and last (Glu27) residues are assigned and indicated by straight lines corresponding to H^{N} 8.0 ppm and 8.35, respectively.
   For both these residues, a strong ROE between the amide proton and the alpha proton of the preceding amino acid residue, HN(i)-Hα(i_-1), are observed as shown in the expansion of the 2D ¹H ROESY fingerprint region (right panel). Establishment of the Val1:HN-Glu27:Hα correlation at 8.02/4.25 ppm strongly suggest that the peptide is in its cyclized form and the protons are separated by less than 5 Å.

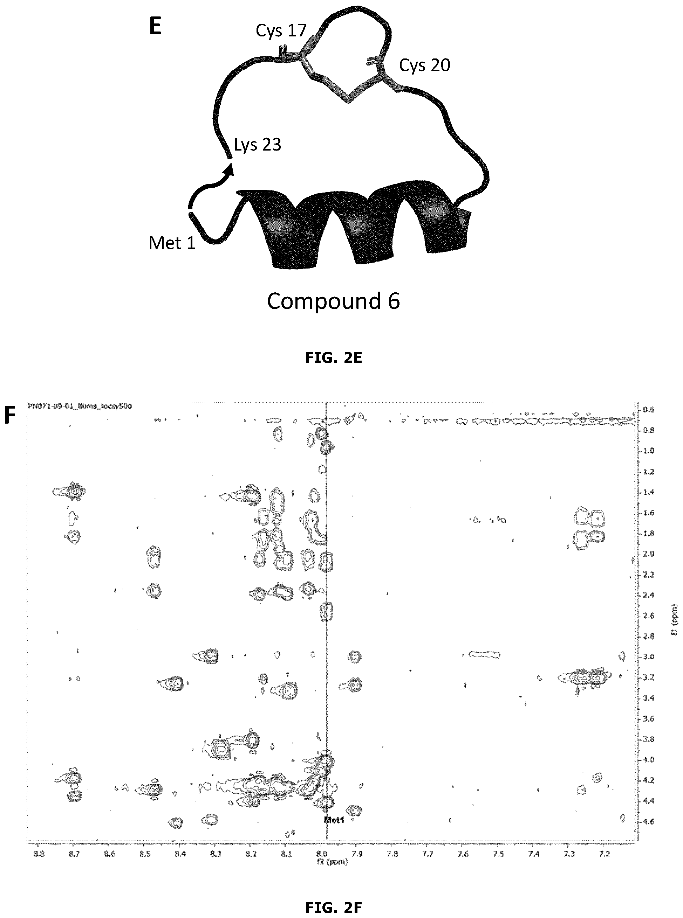

**FIG. 2E** presents compound 6 (SEQ ID NO: 6), showing the ring closing zone by an arrow between the corresponding amino-terminal (Met1) and carboxyl-terminal (Lys23) residues. The disulphide bridge-forming cysteines are shown in gray with stick representation and labelled accordingly.
**FIG. 2F** shows a contour plot of the number of observed amide protons in the 2D ¹H NMR TOCSY amide fingerprint (left panel) for compound 6 (SEQ ID NO: 6). The horizontal axis to be the direct dimension (F2, presented in ppm) and the vertical axis to be the indirect dimension (F1, presented in ppm). The number of observed amide protons in the TOCSY data is consistent with a 23 amino acid long cyclic peptide i.e. the Met1 amide proton is observed, in consistency with the anticipated amidation of the N-terminal, as opposed to its linear analogue where the N-terminal amide proton is not observed. The Met1 amide proton are assigned and indicated by the straight line corresponding to H^{N} 7.98 ppm.

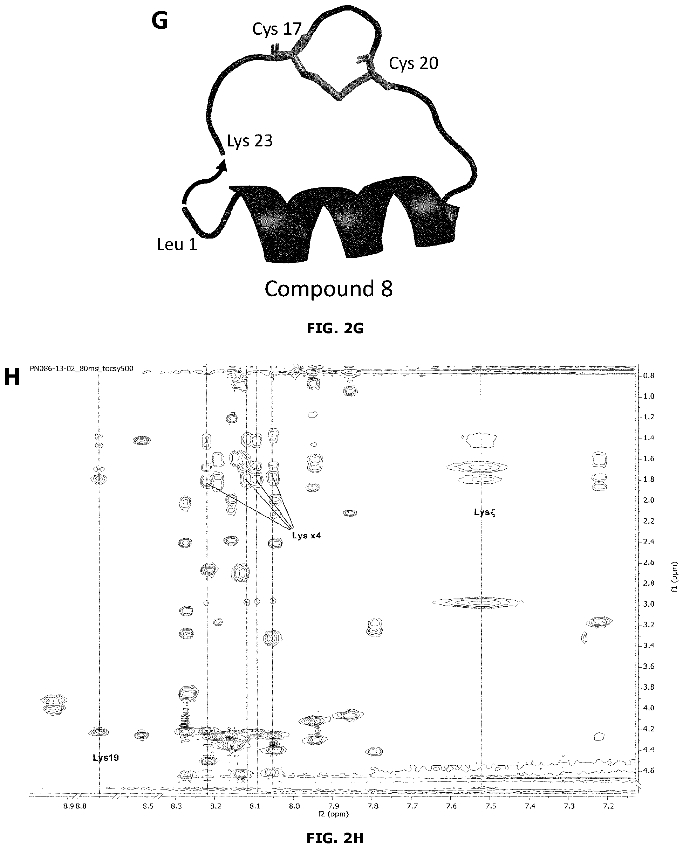

**FIG. 2G** presents compound 8 (SEQ ID NO: 8), showing the ring closing zone by an arrow between the corresponding amino-terminal (Leu1) and carboxyl-terminal (Lys23) residues. The disulphide bridge-forming cysteines are shown in gray with stick representation and labelled accordingly.
**FIG. 2H** shows a contour plot of the number of observed amide protons in the 2D ¹H NMR TOCSY amide fingerprint (left panel) for compound 8 (SEQ ID NO: 8). The horizontal axis to be the direct dimension (F2, presented in ppm) and the vertical axis to be the indirect dimension (F1, presented in ppm). The number of observed amide protons in the TOCSY data is consistent with a 23 amino acid long cyclic peptide i.e. five lysine amide protons are observed, in consistency with the anticipated amidation of the N-terminal, as opposed to its linear analogue where the N-terminal Lys1 amide proton is not observed. ¹H chemical shifts for all of the Lys amide proton are assigned and indicated by the straight line corresponding to H^{N} 7.51, 8.05, 8.09, 8.11 and 8.21 ppm, respectively.

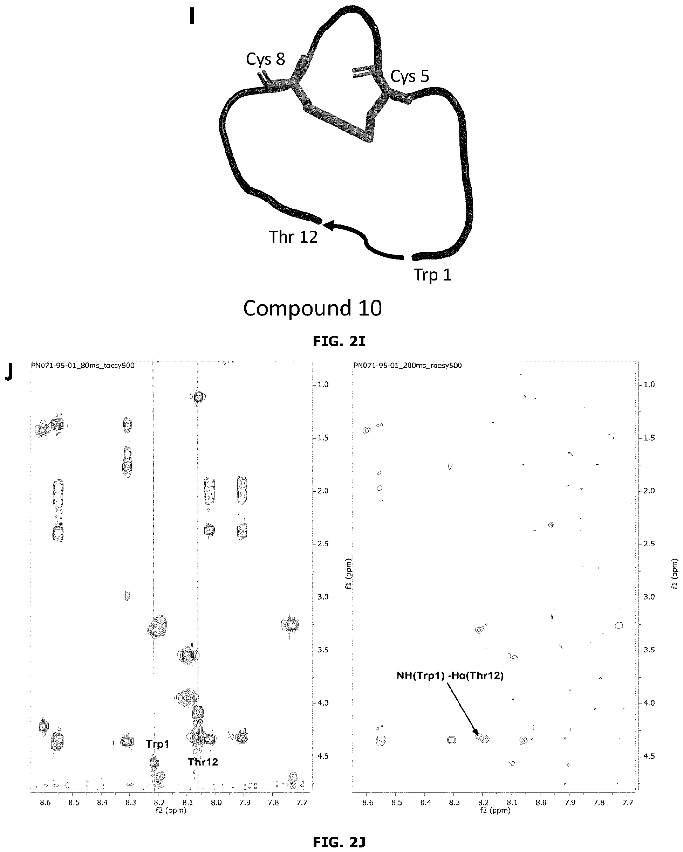

**FIG. 2I** presents compound 10 (SEQ ID NO: 10), showing the ring closing zone by an arrow between the corresponding amino-terminal (Trp1) and carboxyl-terminal (Thr12) residues. The disulphide bridge-forming cysteines are shown in gray with stick representation and labelled accordingly.
**FIG. 2J** shows a contour plot of the number of observed amide protons in the 2D ¹H NMR TOCSY amide fingerprint (left panel) for compound 10 (SEQ ID NO: 10). The horizontal axis to be the direct dimension (F2, presented in ppm) and the vertical axis to be the indirect dimension (F1, presented in ppm). The number of observed amide protons in the TOCSY data is consistent with a 12 amino acid long cyclic peptide i.e. the Val1 amide proton is observed, in consistency with the anticipated amidation of the N-terminal, as opposed to its linear analogue where the N-terminal amide proton is not observed. ¹H chemical shifts for 1st (Trp1) and last (Thr12) residues are assigned and indicated by straight lines corresponding to H^{N} 8.21 ppm and 8.06, respectively. For both these residues, a strong ROE between the amide proton and the alpha proton of the preceding amino acid residue, HN(i)-Hα(i_-1), are observed as shown in the expansion of the 2D ¹H ROESY fingerprint region (right panel). Establishment of the Val1:HN-Glu27:Hα correlation at 8.21/4.30 ppm strongly suggest that the peptide is in its cyclized form and the protons are separated by less than 5 Å.

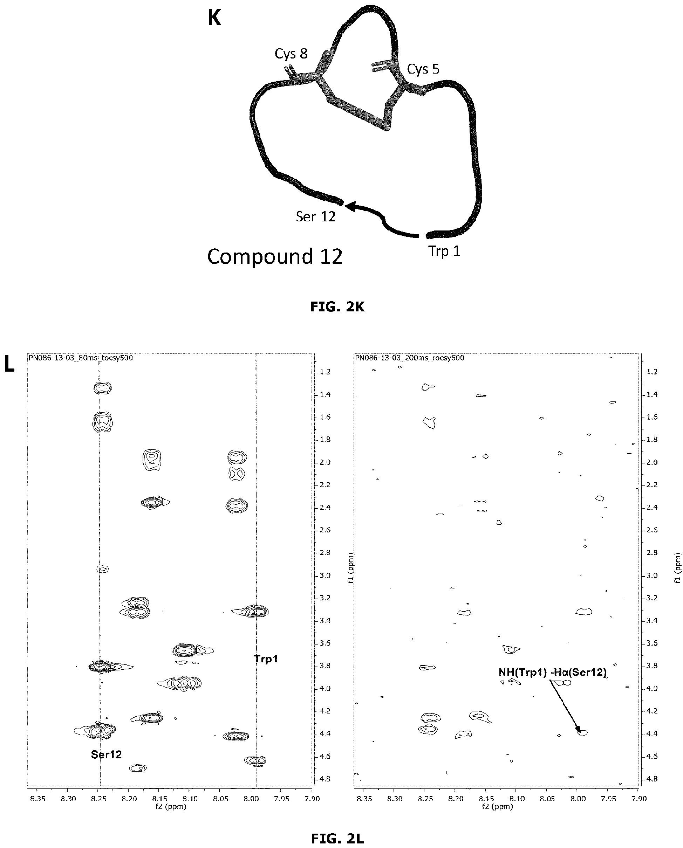

**FIG. 2K** presents compound 12 (SEQ ID NO: 12), showing the ring closing zone by an arrow between the corresponding amino-terminal (Trp1) and carboxyl-terminal (Ser12) residues. The disulphide bridge-forming cysteines are shown in gray with stick representation and labelled accordingly.
**FIG. 2L** shows a contour plot of the number of observed amide protons in the 2D ¹H NMR TOCSY amide fingerprint (left panel) for compound 12 (SEQ ID NO: 12). The horizontal axis to be the direct dimension (F2, presented in ppm) and the vertical axis to be the indirect dimension (F1, presented in ppm). The number of observed amide protons in the TOCSY data is consistent with a 12 amino acid long cyclic peptide i.e. the Trp1 amide proton is observed, in consistency with the anticipated amidation of the N-terminal, as opposed to its linear analogue where the N-terminal amide proton is not observed. ¹H chemical shifts for 1st (Trp1) and last (Ser12) residues are assigned and indicated by straight lines corresponding to H^{N} 7.98 ppm and 8.244, respectively.
   For both these residues, a strong ROE between the amide proton and the alpha proton of the preceding amino acid residue, HN(i)-Hα(i_-1), are observed as shown in the expansion of the 2D ¹H ROESY fingerprint region (right panel). Establishment of the Val1 :HN-Glu27:Hα correlation at 7.99/4.38 ppm strongly suggest that the peptide is in its cyclized form and the protons are separated by less than 5 Å.

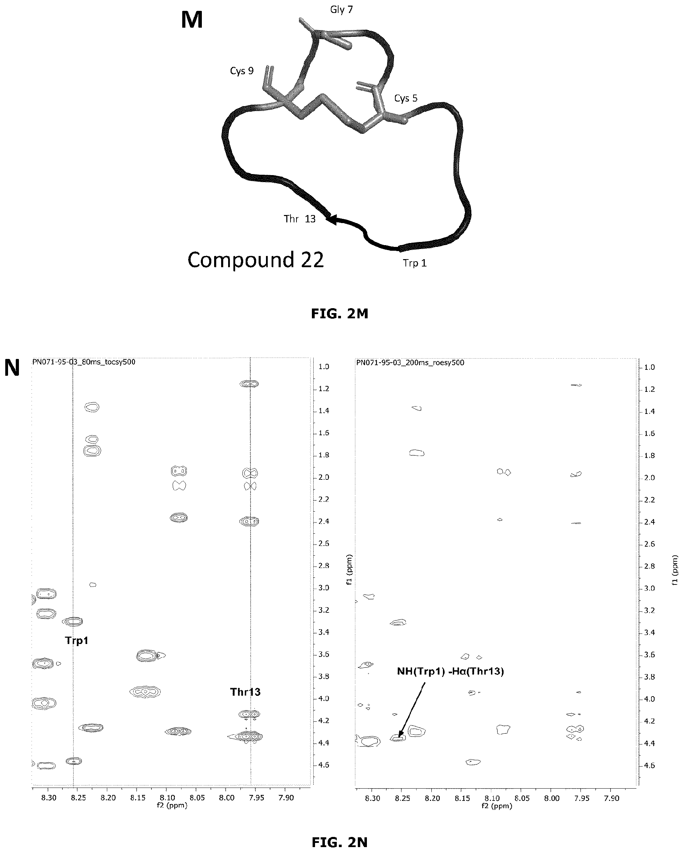

**FIG. 2M** presents compound 22 (SEQ ID NO: 22), showing the ring closing zone by an arrow between the corresponding amino-terminal (Trp1) and carboxyl-terminal (Thr13) residues. The disulphide bridge-forming cysteines are shown in gray with stick representation and labelled accordingly. The inserted glycine residue between the Cys residues of the CXXC motif in compound 22 (SEQ ID NO: 22) are also shown in gray stick representation.
**FIG. 2N** shows a contour plot of the number of observed amide protons in the 2D ¹H NMR TOCSY amide fingerprint (left panel) for compound 22 (SEQ ID NO: 22). The horizontal axis to be the direct dimension (F2, presented in ppm) and the vertical axis to be the indirect dimension (F1, presented in ppm). The number of observed amide protons in the TOCSY data is consistent with a 13 amino acid long cyclic peptide i.e. the Trp1 amide proton is observed, in consistency with the anticipated amidation of the N-terminal, as opposed to its linear analogue where the N-terminal amide proton is not observed. ¹H chemical shifts for 1st (Trp1) and last (Thr13) residues are assigned and indicated by straight lines corresponding to H^{N} 7.96 ppm and 8.26, respectively. For both these residues, a strong ROE between the amide proton and the alpha proton of the preceding amino acid residue, HN(i)-Hα(i_-1), are observed as shown in the expansion of the 2D ¹H ROESY fingerprint region (right panel). Establishment of the Val1 :HN-Glu27:Hα correlation at 8.26/4.35 ppm strongly suggest that the peptide is in its cyclized form and the protons are separated by less than 5 Å.

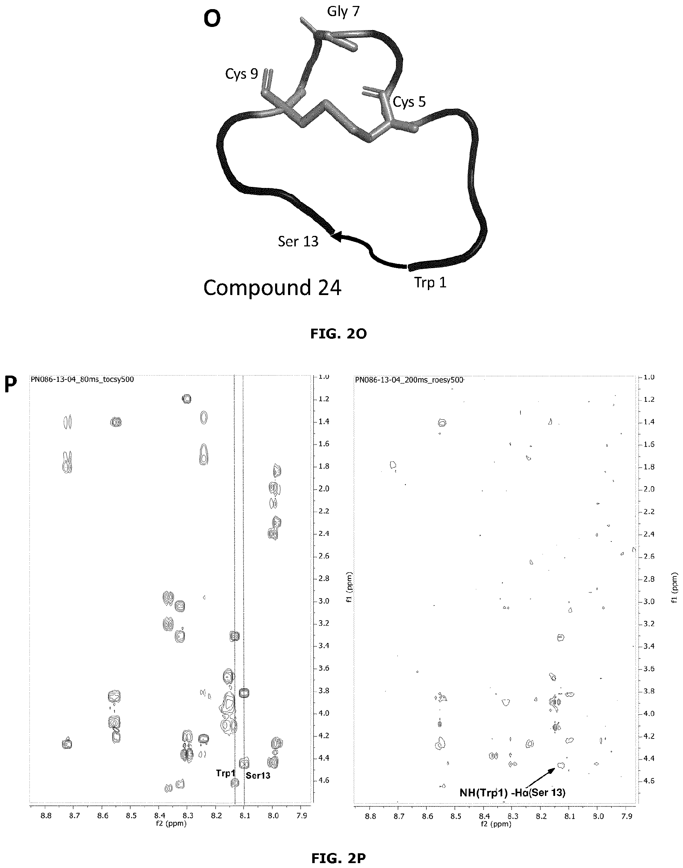

**FIG. 2O** presents compound 24 (SEQ ID NO: 24), showing the ring closing zone by an arrow between the corresponding amino-terminal (Trp1) and carboxyl-terminal (Ser13) residues. The disulphide bridge-forming cysteines are shown in gray with stick representation and labelled accordingly. The inserted glycine residue in compound 24 (SEQ ID NO: 24) is also shown in gray stick representation.
**FIG. 2P** shows a contour plot of the number of observed amide protons in the 2D ¹H NMR TOCSY amide fingerprint (left panel) for compound 24 (SEQ ID NO: 24). The horizontal axis to be the direct dimension (F2, presented in ppm) and the vertical axis to be the indirect dimension (F1, presented in ppm). The number of observed amide protons in the TOCSY data is consistent with a 13 amino acid long cyclic peptide i.e. the Trp1 amide proton is observed, in consistency with the anticipated amidation of the N-terminal, as opposed to its linear analogue where the N-terminal amide proton is not observed. ¹H chemical shifts for 1st (Trp1) and last (Ser13) residues are assigned and indicated by straight lines corresponding to H^{N} 8.13 ppm and 8.1, respectively. For both these residues, a strong ROE between the amide proton and the alpha proton of the preceding amino acid residue, HN(i)-Hα(i_-1), are observed as shown in the expansion of the 2D ¹H ROESY fingerprint region (right panel). Establishment of the Val1 :HN-Glu27:Hα correlation at 8.13/4.45 ppm strongly suggest that the peptide is in its cyclized form and the protons are separated by less than 5Å.

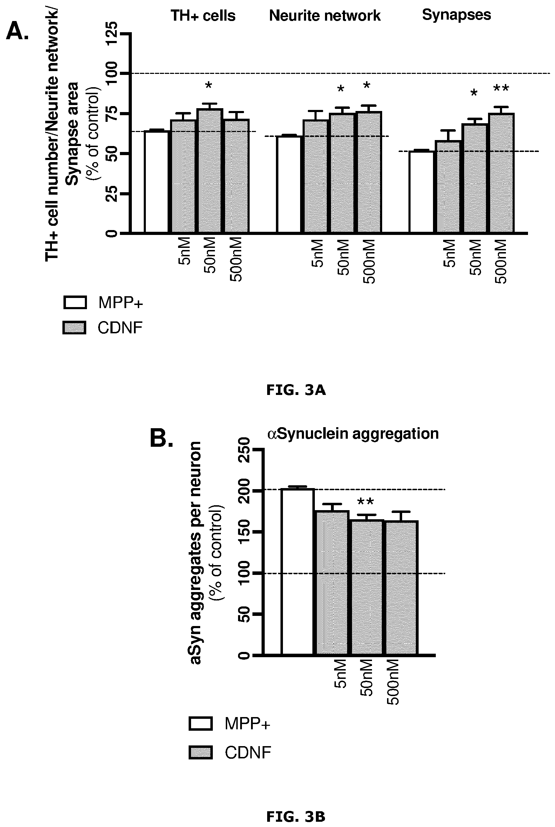

**FIGs. 3A-3N** show the neuroprotective effects of rhCDNF and linear and macrocyclic compounds (compounds 1 - 12 having SEQ ID NOs: 1 - 12, respectively) on dopaminergic TH (tyrosine hydroxylase)-positive neurons injured with MPP+ (1-methyl-4-phenylpyridinium), and the effect on alpha-synuclein aggregation in TH-positive neurons. Data are expressed as a percentage of control non-injured condition as mean +/- SEM (n=4-6; MPP+ negative control n=122-127 collected across the multiple studies). *p<0.05, **p<0.01, ***p<0.001, ****p<0.0001 Brown-Forsythe and Welch ANOVA test with post-hoc Dunnett pairwise comparison vs MPP+ negative control. # p<0.05, ## p<0.01, ### p<0.001, #### p<0.0001 Brown-Forsythe and Welch ANOVA test with post-hoc Dunnett pairwise comparison between linear and corresponding macrocyclic compound in the same concentration.
**FIG. 3A** shows the number of TH neurons, total neurite network of TH neurons, and number of synapses on TH neurites in a primary culture of mesencephalic cells after MPP+ injury in the presence of increasing concentrations of rhCDNF. Upper dashed line represents a control level of parameters (100%) obtained from non-injured cells; lower dashed line represents a negative control level of parameters obtained from cells injured with MPP+ without additional treatment with compounds.
**FIG. 3B** shows alpha-synuclein aggregation in TH neurons of a primary culture of mesencephalic cells after MPP+ injury in the presence of increasing concentrations of rhCDNF. Upper dashed line represents a negative control level of alpha-synuclein aggregation in cells injured with MPP+ without additional treatment with test compounds; lower dashed line represents a negative control level of parameters (100%) obtained from non-injured cells.
**FIG. 3C** shows the number of TH neurons, total neurite network of TH neurons, and the number of synapses on TH neurites in a primary culture of mesencephalic cells after MPP+ injury in the presence of compound 1 (SEQ ID NO: 1) and compound 2 (SEQ ID NO: 2).
**FIG. 3D** shows alpha-synuclein aggregation in TH neurons of a primary culture of mesencephalic cells after MPP+ injury in the presence of compound 1 (SEQ ID NO: 1) and compound 2 (SEQ ID NO :2).
**FIG. 3E** shows the number of TH neurons, total neurite network of TH neurons, and the number of synapses on TH neurites in a primary culture of mesencephalic cells after MPP+ injury in the presence of compound 3 (SEQ ID NO: 3) and compound 4 (SEQ ID NO: 4).
**FIG. 3F** shows alpha-synuclein aggregation in TH neurons of a primary culture of mesencephalic cells after MPP+ injury in the presence of compound 3 (SEQ ID NO: 3) and compound 4 (SEQ ID NO: 4).
**FIG. 3G** shows the number of TH neurons, total neurite network of TH neurons, and the number of synapses on TH neurites in a primary culture of mesencephalic cells after MPP+ injury in the presence of compound 5 (SEQ ID NO: 5) and compound 6 (SEQ ID NO: 6).
**FIG. 3H** shows alpha-synuclein aggregation in TH neurons of a primary culture of mesencephalic cells after MPP+ injury in the presence of compound 5 (SEQ ID NO: 5) and compound 6 (SEQ ID NO: 6).
**FIG. 3I** shows the number of TH neurons, total neurite network of TH neurons, and the number of synapses on TH neurites in a primary culture of mesencephalic cells after MPP+ injury in the presence of compound 7 (SEQ ID NO: 7) and compound 8 (SEQ ID NO: 8).
**FIG. 3J** shows alpha-synuclein aggregation in TH neurons of a primary culture of mesencephalic cells after MPP+ injury in the presence of compound 7 (SEQ ID NO: 7) and compound 8 (SEQ ID NO: 8).
**FIG. 3K** shows the number of TH neurons, total neurite network of TH neurons, and the number of synapses on TH neurites in a primary culture of mesencephalic cells after MPP+ injury in the presence of compound 9 (SEQ ID NO: 9) and compound 10 (SEQ ID NO: 10).
**FIG. 3L** shows alpha-synuclein aggregation in TH neurons of a primary culture of mesencephalic cells after MPP+ injury in the presence of compound 9 (SEQ ID NO: 9) and compound 10 (SEQ ID NO: 10).
**FIG. 3M** shows the number of TH neurons, total neurite network of TH neurons, and the number of synapses on TH neurites in a primary culture of mesencephalic cells after MPP+ injury in the presence of compound 11 (SEQ ID NO: 11) and compound 12 (SEQ ID NO: 12).
**FIG. 3N** shows alpha-synuclein aggregation in TH neurons of a primary culture of mesencephalic cells after MPP+ injury in the presence of compound 11 (SEQ ID NO: 11) and compound 12 (SEQ ID NO: 12).

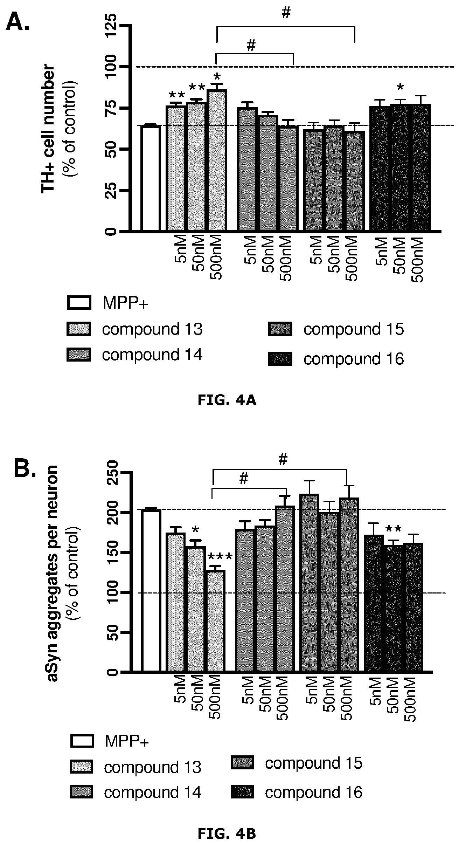

**FIGs. 4A-4H** show neuroprotective effect of linear and macrocyclic compounds (compounds 13 - 24 having SEQ ID NOs: 13 - 24, respectively) on dopaminergic TH-positive neurons injured with MPP+, and the effect on alpha-synuclein aggregation in TH-positive neurons. Data are expressed as a percentage of control non-injured condition as mean +/- SEM (n=4-6; MPP+ negative control n=122-127 collected across the multiple studies). *p<0.05, **p<0.01, ***p<0.001, ****p<0.0001 Brown-Forsythe and Welch ANOVA test with post-hoc Dunnett pairwise comparison vs MPP+ negative control. # p<0.05, ## p<0.01, ### p<0.001, #### p<0.0001 Brown-Forsythe and Welch ANOVA test with post-hoc Dunnett pairwise comparison between linear and corresponding macrocyclic compound in the same concentration.
**FIG. 4A** shows the number of TH neurons in a primary culture of mesencephalic cells after MPP+ injury in the presence of compound 13 (SEQ ID NO: 13), compound 14 (SEQ ID NO: 14), compound 15 (SEQ ID NO: 15) and compound 16 (SEQ ID NO: 16). Upper dashed line represents a control level of parameters (100%) obtained from non-injured cells; lower dashed line represents a negative control level of parameters obtained from cell injured with MPP+ without additional treatment with compounds.
**FIG. 4B** shows alpha-synuclein aggregation in TH neurons of a primary culture of mesencephalic cells after MPP+ injury in the presence of compound 13 (SEQ ID NO: 13), compound 14 (SEQ ID NO: 14), compound 15 (SEQ ID NO: 15) and compound 16 (SEQ ID NO: 16). Upper dashed line represents a negative control level of alpha-synuclein aggregations in cells injured with MPP+ without additional treatment with test compounds; lower dashed line represents a negative control level of parameters (100%) obtained from non-injured cells.

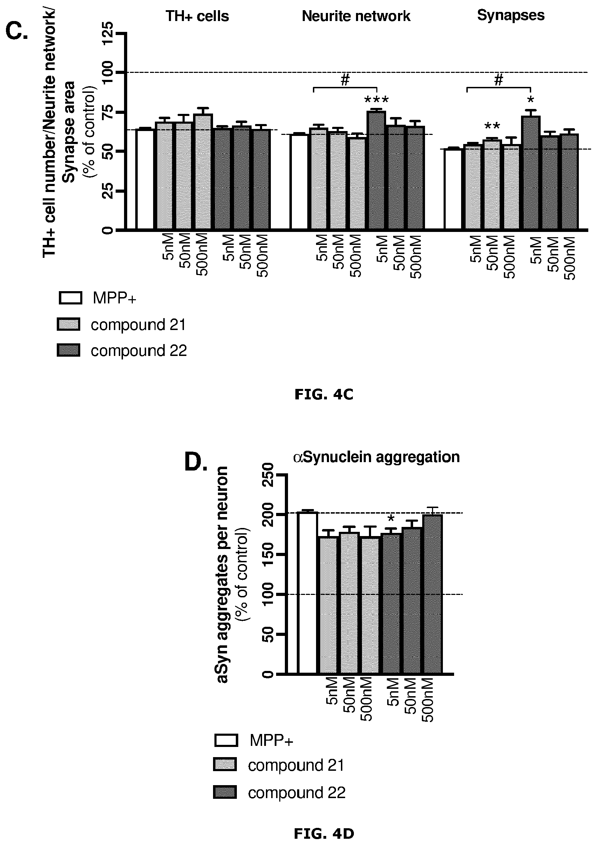

**FIG. 4C** shows the number of TH neurons, total neurite network of TH neurons, and the number of synapses on TH neurites in a primary culture of mesencephalic cells after MPP+ injury in the presence of compound 21 (SEQ ID NO: 21) and compound 22 (SEQ ID NO: 22).
**FIG. 4D** shows alpha-synuclein aggregation in TH neurons of a primary culture of mesencephalic cells after MPP+ injury in the presence of compound 21 (SEQ ID NO: 21) and compound 22 (SEQ ID NO: 22).

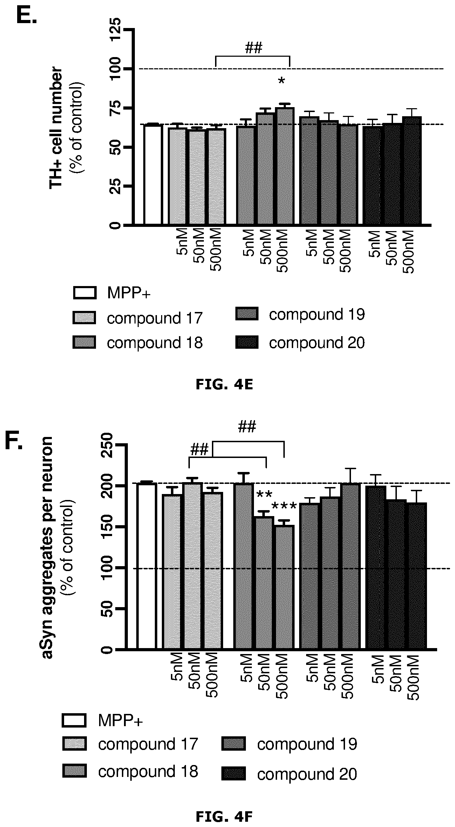

**FIG. 4E** shows the number of TH neurons in a primary culture of mesencephalic cells after MPP+ injury in the presence of compound 17 (SEQ ID NO: 17), compound 18 (SEQ ID NO: 18), compound 19 (SEQ ID NO: 19) and compound 20 (SEQ ID NO: 20).
**FIG. 4F** shows alpha-synuclein aggregation in TH neurons of a primary culture of mesencephalic cells after MPP+ injury in the presence of compound 17 (SEQ ID NO: 17), compound 18 (SEQ ID NO: 18), compound 19 (SEQ ID NO: 19) and compound 20 (SEQ ID NO: 20).

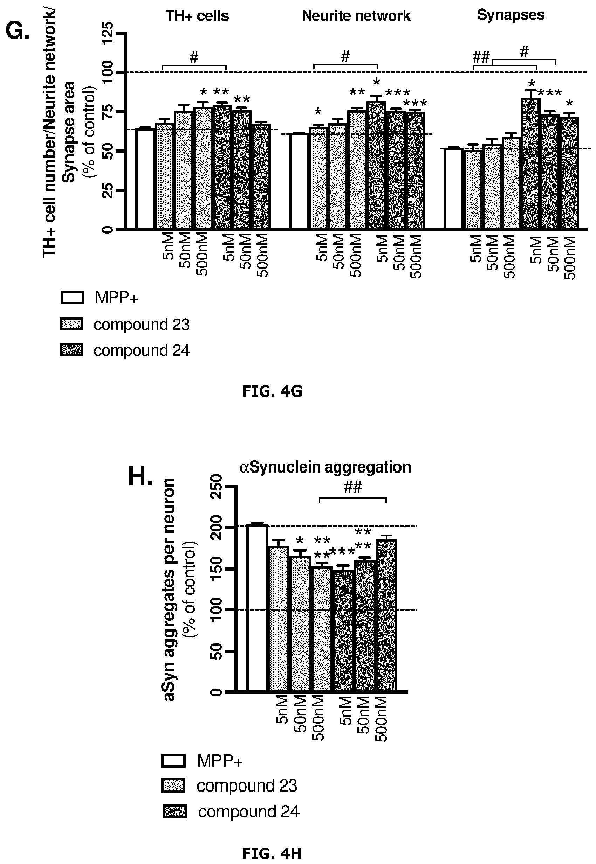

**FIG. 4G** shows the number of TH neurons, total neurite network of TH neurons, and the number of synapses on TH neurites in a primary culture of mesencephalic cells after MPP+ injury in the presence of compound 23 (SEQ ID NO: 23) and compound 24 (SEQ ID NO: 24).
**FIG. 4H** shows alpha-synuclein aggregation in TH neurons of a primary culture of mesencephalic cells after MPP+ injury in the presence of compound 23 (SEQ ID NO: 23) and compound 24 (SEQ ID NO: 24).

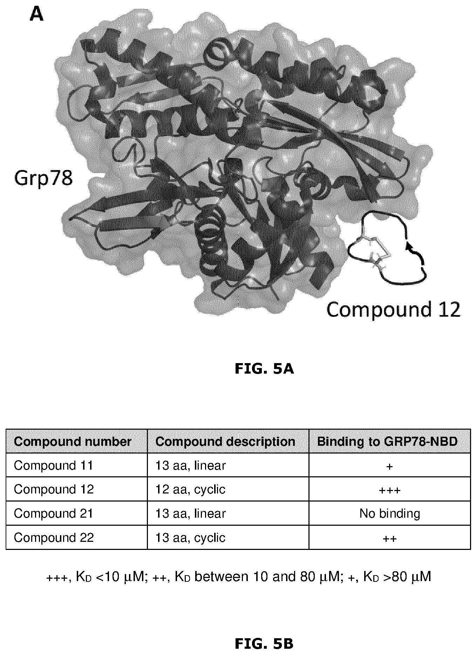

**FIG. 5A** shows the computational molecular model of the nucleotide-binding domain of GRP78 in complex with Compound 12. GRP78 is shown in a translucent surface model along with its cartoon trace. The Cys-Cys bond in compound 12 (SEQ ID NO: 12) is shown in sticks.
**FIG. 5B** shows the microscale thermophoresis-based binding affinities of a representative set of peptides, compound 11 (SEQ ID NO: 11), compound 12 (SEQ ID NO: 12), compound 21 (SEQ ID NO: 21) and compound 22 (SEQ ID NO:22) to GRP78 in a tabulated manner. Binding affinity is indicated as follows: +++, K_{D} <10 µM; ++, K_{D} 10-80 µM; +, K_{D} >80 µM, and no binding.

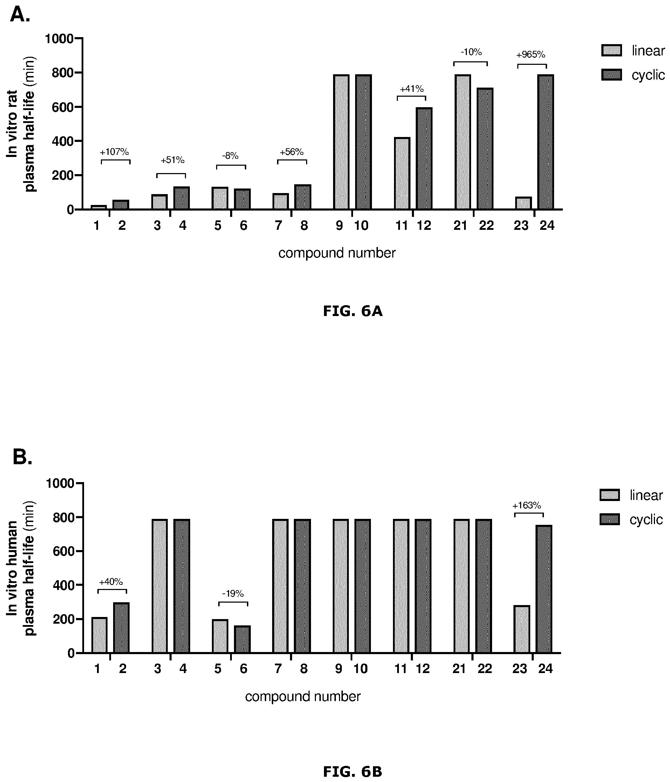

**FIGs. 6A-6B** show *in vitro* metabolic stability of linear and macrocyclic compounds (compounds 1 -12 and compounds 21-24 having SEQ ID NOs: 1 - 12, and 21-24, respectively) in rat and human plasma.
**FIG. 6A** shows the calculated half-life based on compound disappearance in rat plasma.
**FIG. 6B** shows the calculated half-life based on compound disappearance in human plasma. Arks and numbers above the columns reflect the change of plasma half-life of macrocyclic compounds as a percentage of corresponding linear compounds. The maximum calculated half-life at 789 min reflects experiment cut-off time limitation.

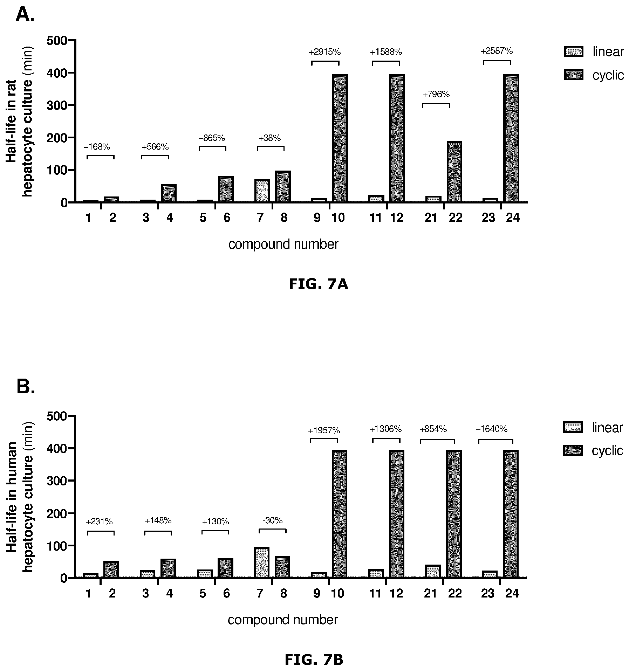

**FIGs. 7A-7B** show *in vitro* metabolic stability of linear and macrocyclic compounds (compounds 1 -12 and compounds 21-24 having SEQ ID NOs: 1 - 12 and 21-24, respectively) in rat and human hepatocytes.
**FIG. 7A** shows the calculated half-life based on compound disappearance in rat liver hepatocytes.
**FIG. 7B** shows the calculated half-life based on compound disappearance in human liver hepatocytes. Arks and numbers above the columns reflect the change of half-life of macrocyclic compounds in hepatocytes as a percentage of corresponding linear compounds. The maximum calculated half-life at 395 min reflects experiment cut-off time limitation.

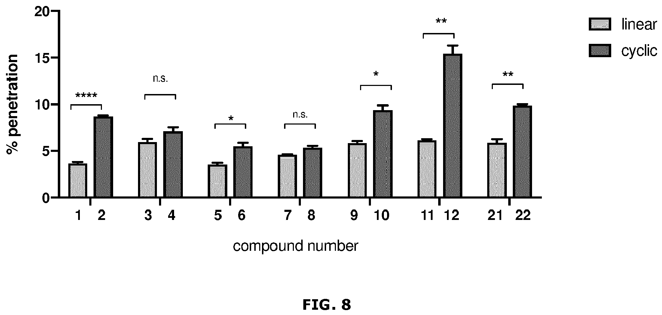

**FIG. 8** shows penetration of linear and macrocyclic compounds (compounds 1 - 12 and compounds 21-22 having SEQ ID NOs: 1 - 12 and 21-22, respectively) through a 3D *in vitro* model of blood brain barrier. The amount of compound crossed the artificial blood brain barrier expressed in percentage of compound original applied concentration. Data are presented as mean +/- SEM (n=3-4) *p<0.05, **p<0.01, ***p<0.001, ****p<0.0001, n.s. not significant, Brown-Forsythe and Welch ANOVA test with post-hoc Dunnett pairwise comparison between linear and corresponding macrocyclic compound.

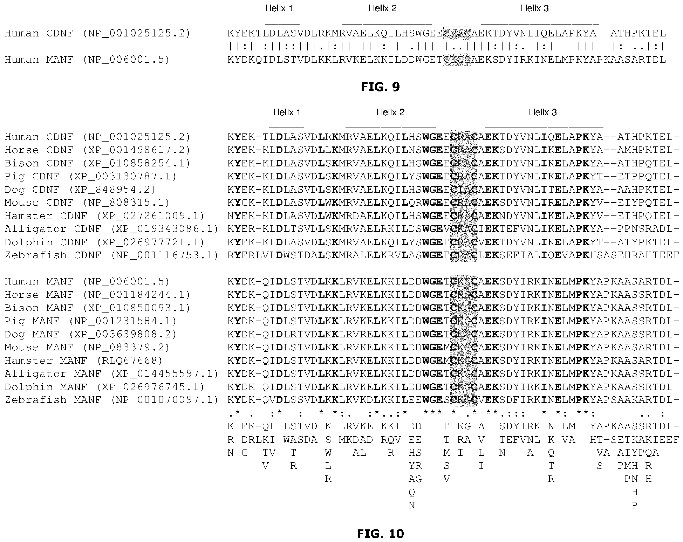

**FIG. 9** shows pairwise alignment of human CDNF and MANF C-terminal domains (61-63 aa). The alignment was performed using the following Genbank-retrieved sequences: for human CDNF accession # NP_001025125.2, and for human MANF accession # NP_006001.5. The CXXC motif is indicated gray background and the position of the three α-helices are shown.
**FIG. 10** shows ClustalW multiple sequence alignment of the C-terminal domains of CDNF and MANF (61-63 aa) from 10 different species (SEQ ID NO:s 51-70 respectively). The Genbank accession numbers are shown in the sequence alignment. The CXXC motif is indicated gray background and the position of the three α-helices are shown. Those residues conserved between these representative sequences (in both CDNF and MANF) are shown in bold. Below the sequence alignment, natural variants found in the representative 10 species per each position are shown. The presented list of sequences and species can be used to identify conserved and variable positions and shows that only limited variation is possible for most non-essential amino acid residues.

### SEQUENCE LISTING

**SEQ ID NO: 1** VDLRKMRVAELKQILHSWGEECRACAE
**SEQ ID NO: 2** VDLRKMRVAELKQILHSWGEECRACAE, head-to-tail cyclic
**SEQ ID NO: 3** VDLKKLRVKELKKILDDWGETCKGCAE
**SEQ ID NO: 4** VDLKKLRVKELKKILDDWGETCKGCAE, head-to-tail cyclic
**SEQ ID NO: 5** MRVAELKQILHSWGEECRACAEK
**SEQ ID NO: 6** MRVAELKQILHSWGEECRACAEK, head-to-tail cyclic
**SEQ ID NO: 7** LRVKELKKILDDWGETCKGCAEK
**SEQ ID NO: 8** LRVKELKKILDDWGETCKGCAEK, head-to-tail cyclic
**SEQ ID NO: 9** WGEECRACAEKT
**SEQ ID NO: 10** WGEECRACAEKT, head-to-tail cyclic
**SEQ ID NO: 11** WGETCKGCAEKS
**SEQ ID NO: 12** WGETCKGCAEKS, head-to-tail cyclic
**SEQ ID NO: 13** GEECRACAEKT
**SEQ ID NO: 14** GEECRGACAEKT
**SEQ ID NO: 15** GEECRAACAEKT
**SEQ ID NO: 16** GEECRSACAEKT
**SEQ ID NO: 17** GETCKGCAEKS
**SEQ ID NO: 18** GETCKGGCAEKS
**SEQ ID NO: 19** GETCKAGCAEKS
**SEQ ID NO: 20** GETCKSGCAEKS
**SEQ ID NO: 21** WGEECRGACAEKT
**SEQ ID NO: 22** WGEECRGACAEKT, head-to-tail cyclic
**SEQ ID NO: 23** WGETCKGGCAEKS
**SEQ ID NO: 24** WGETCKGGCAEKS, head-to-tail cyclic
**SEQ ID NO: 25** C-X₁-X₂-X₃-C
**SEQ ID NO: 26** E-X₄-C-X₁-X₂-X₃-C-A-E
**SEQ ID NO: 27** X₅-X₆-X₇-X₈-E-X₄-C-X₁-X₂-X₃-C-A-E-X₉-X₁₀-X₁₁ 
SEQ ID NO: 28 X12-X13-X14-X15-X16-X17-X18-X19-X20-X21-X22-X23-V-X24-E-L-K-X25-X26-L-X5 X6-X7-X8-E-
X4-C-X1-X2-X3-C-A-E-X9-X10-X11
**SEQ ID NO: 29** RVAELKQILHSWGEECRACAEKTDYVNLIQELAPKYA, native human CDNF peptide
**SEQ ID NO: 30** RVKELKKILDDWGETCKGCAEKSDYIRKINELMPKYA, native human MANF peptide 
SEQ ID NO: 31 X16-X17-X18-X19-X20-X21-X22-X23-V-X24-E-L-K-X25-X26-L-X5-X6-X7-X8-E-X4-C-X1-X2-X3-
C-A-E-X9-X10-X11
 
SEQ ID NO: 32 X15-X16-X17-X18-X19-X20-X21-X22-X23-V-X24-E-L-K-X25-X26-L-X5-X6-X7-X8-E-X4-C-X1-
X2-X3-C-A-E-X9-X10
 
SEQ ID NO: 33 X14-X15-X16-X17-X18-X19-X20-X21-X22-X23-V-X24-E-L-K-X25-X26-L-X5-X6-X7-X8-E-X4-C-
X1-X2-X3-C-A-E-X9
 
SEQ ID NO: 34 X13-X14-X15-X16-X17-X18-X19-X20-X21-X22-X23-V-X24-E-L-K-X25-X26-L-X5-X6-X7-X8-E-X4-
C-X1-X2-X3-C-A-E
**SEQ ID NO: 35** KDEL
**SEQ ID NO: 36** CRAC
**SEQ ID NO: 37** CKGC
**SEQ ID NO: 38** CXXC
**SEQ ID NO: 39** ETCKGCAE
**SEQ ID NO: 40** TCKGCA 
SEQ ID NO: 41 MWCASPVAVV AFCAGLLVSH PVLTQGQEAG GRPGADCEVC KEFLNRFYKS
LIDRGVNFSL DTIEKELISF CLDTKGKENR LCYYLGATKD AATKILSEVT RPMSVHMPAM
KICEKLKKLD SQICELKYEK TLDLASVDLR KMRVAELKQI LHSWGEECRA CAEKTDYVNL
IQELAPKYAA THPKTEL, full length CDNF (NCBI Reference Sequence: NP_001025125.2)
 
SEQ ID NO: 42 MRRMWATQGL AVALALSVLP GSRALRPGDC EVCISYLGRF YQDLKDRDVT
FSPATIENEL IKFCREARGK ENRLCYYIGA TDDAATKIIN EVSKPLAHHI PVEKICEKLK
KKDSQICELK YDKQIDLSTV DLKKLRVKEL KKILDDWGET CKGCAEKSDY IRKINELMPK
YAPKAASART DL, full length MANF (NCBI Reference Sequence: NP_006001.5)
**SEQ ID NO: 43** TLDLASVDLRKMRVAELKQILHSWGEECRACAEKTDYVNLIQELAPKYA (49 aa CDNF)
**SEQ ID NO: 44** RVAELKQILHSWGEECRACAEKTDYVNLIQELAPKYA (37 aa CDNF)
**SEQ ID NO: 45** TLDLASVDLRKMRVAELKQILHSWGEECRACAEKT (35 aa CDNF)
**SEQ ID NO: 46** LASVDLRKMRVAELKQILHSWGEECRACAEKT (32 CDNF)
**SEQ ID NO: 47** QIDLSTVDLKKLRVKELKKILDDWGETCKGCAEKSDYIRKINELMPKYA (49 aa MANF)
**SEQ ID NO: 48** RVKELKKILDDWGETCKGCAEKSDYIRKINELMPKYA (37 aa MANF)
**SEQ ID NO: 49** QIDLSTVDLKKLRVKELKKILDDWGETCKGCAEKS (35 aa MANF)
**SEQ ID NO: 50** LSTVDLKKLRVKELKKILDDWGETCKGCAEKS (32 aa MANF)

SEQ ID NO: 51 KYEKTLDLASVDLRKMRVAELKQILHSWGEECRACAEKTDYVNLIQELAPKYA
ATHPKTEL, Human CDNF (NP_001025125.2)

SEQ ID NO: 52 KYEK-KLDLASVDLLKMRVAELKQILNSWGEECRACAEKSDYVNLIKELAPKYA
AMHPKTEL, Horse CDNF (XP_001498617.2)

SEQ ID NO: 53 KYEKKLDLASVDLSKMRVAELKQILHGWGEECRACAEKTDYVNLIKELAPKYA
ATHPQTEL, Bison CDNF (XP_010858254.1)

SEQ ID NO: 54 KYEK-KLDLASVDLSKMRVAELQILYSWGEECRACAEKTDYVNLIKELAPKYTE
TPPQTEL, Pig CDNF (XP_003130787.1)

SEQ ID NO: 55 KYEK-KLDLASVDLSKMRVAELKQILHSWGEECIACAEKTDYVNLITELAPKYAA
AHPKTEL, Dog CDNF (XP_848954.2)

SEQ ID NO: 56 KYGK-KLDLASVDLWKMRVAELKQILQRWGEECRACAEKSDYVNLIRELAPKY
VEIYPQTEL, Mouse CDNF (NP_808315.1)

SEQ ID NO: 57 NYEK-KLDLASVDLWKMRDAELKQILHSWGEECRACAEKNDYVNLIKELAPKY
VEIHPQIEL, Hamster CDNF (XP_027261009.1)

SEQ ID NO: 58 KYER-KLDLTSVDLSKMRVAELRKILDSWGEVCKACIEKTEFVNLIKELAPKYA
PPNSRADL, Alligator CDNF (XP_019343086.1)

SEQ ID NO: 59 KYEK-KLDLASVDLSKMRVAELKQILYSWGEECRACVEKTDYVNLIKELAPKYT
ATYPKTEL, Dolphin CDNF (XP_026977721.1)

SEQ ID NO: 60 RYERLVLDWSTDALSKMRALELKRVLASWGEECRACLEKSEFIALIQEVAPKH
SASEHRAHTEEF, Zebrafish CDNF (NP_001116753.1)

SEQ ID NO: 61 KYDK-QIDLSTVDLKKLRVKELKKILDDWGETCKGCAEKSDYIRKINELMPKYAP
KAASARTDL, Human MANF (NP_006001.5)

SEQ ID NO: 62 KYDK-QIDLSTVDLKKLRVKELKKILDDWGETCKGCAEKSDYIRKINELMPKYAP
KAASSRTDL, Horse MANF (NP_001184244.1)

SEQ ID NO: 63 KYDK-QIDLSTVDLKKLRVKELKKILDDWGETCKGCAEKSDYIRKINELMPKYAP
KAASSRTDL, Bison MANF (XP_010850093.1)

SEQ ID NO: 64 KYDK-QIDLSTVDLKKLRVKELKKILDDWGETCKGCAEKSDYIRKINELMPKYAP
KAASSRTDL, Pig MANF (NP_001231584.1)

SEQ ID NO: 65 KYDK-QIDLRTVDLKKLRVRELKKILDDWGETCKGCAEKSDYIRKINELMPKYAP
KAASSRTDL, Dog MANF (XP_003639808.2)

SEQ ID NO: 66 KYDK-QIDLSTVDLKKLRVKELKKILDDWGEMCKGCAEKSDYIRKINELMPKYAP
KAASARTDL, Mouse MANF (NP_083379.2)

SEQ ID NO: 67 KYDK-QIDLSTVDLKKLRVKELKKILDDWGEMCKGCAEKSDYIRKINELMPKYAP
KAASARTDL, Hamster MANF (RLQ67668)

SEQ ID NO: 68 KYDK-QIDLSTVDLKKLRVKELKKILDDWGETCKGCAEKSDYIRKINELMPKYAP
KAASSRTDL, Alligator MANF (XP_014455597.1)

SEQ ID NO: 69 KYDK-QIDLSTVDLKKLRVKELKKILDDWGETCKGCAEKSDYIRKINELMPKYAP
KAASSRTDL, Dolphin MANF (XP_026976745.1)

SEQ ID NO: 70 KYDK-QVDLSSVDLKKLKVKDLKKILEEWGESCKGCVEKSDFIRKINELMPKYA
PSAAKARTDL, Zebrafish MANF (NP_001070097.1)

### DETAILED DESCRIPTION OF THE INVENTION

The term "modified peptide" refers to a peptide or polypeptide, which has been modified. Peptide modification or synthesis options include e.g. macrocyclic peptides, peptidomimetics, N-terminal modifications, C-terminal modifications, isotope labeled peptides, biotinylated and tagged peptides, fluorescent dye labeled peptides, peptide dimers, post-translational modifications, internally quenched/FRET peptides, linker/spacer/PEGylations, peptide pooling, protein conjugation, immunogenic peptides, and incorporation of non-naturally encoded amino acids. A "non-naturally encoded amino acid" refers to an amino acid that is not one of the 20 common amino acids or pyrrolysine or selenocysteine. Other terms that may be used synonymously with the term "non-naturally encoded amino acid" are "non-natural amino acid," "unnatural amino acid," "non-naturally-occurring amino acid," and variously hyphenated and non-hyphenated versions thereof. The term "non-naturally encoded amino acid" also includes, but is not limited to, [amino acids that occur by modification (e.g. post-translational modifications) of a naturally encoded amino acid (including but not limited to, the 20 common amino acids or pyrrolysine and selenocysteine) but are not themselves naturally incorporated into a growing polypeptide chain by the translation complex. Examples of such non-naturally-occurring amino acids include, but are not limited to, N-acetylglucosaminyl-L-serine, N-acetylglucosaminyl-L-threonine, and O-phosphotyrosine]

The term "macrocyclic peptide" refers to a polypeptide chain having a cyclic ring structure. As used herein, if the two cysteine residues in the CX₁X₂X₃C motif are present in disulphide bridge form, the macrocyclic peptide comprises a ring structure in addition to the ring structure formed by the disulphide bridge. In certain embodiments, the macrocyclic peptide comprises a ring structure formed by more than five amino acid residues. The ring structure may involve linkages including N-terminus - to - C-terminus (head-to-tail), head-to-side-chain, side-chain-to-tail, and side-chain-to-side-chain linkages. In one embodiment, the present application provides peptides with a head-to-tail linkage. The linkages may be formed by linking one amino acid of the peptide to another amino acid of the peptide (e.g. end-to-end, side-chain-to-end). with an amide bond or other chemically stable bonds such as lactone, ether, thioether, disulfide etc. In one embodiment bi and monocyclic peptides maybe cyclized through a disulfide bond between two cysteines. Options for cyclization include e.g.: Cys-Cys (up to 4 disulfide bonds in one peptide, site-specific or thermodynamic cyclization), cyclized peptides via amide (head-to-tail or side-chain-to-side-chain), thioether (Cys-bromoacetate).

The term "pseudopeptide" refers to an amide of an amino acid that does not occur in natural peptides or proteins, especially one introduced into a polypeptide chain. Pseudopeptides or amino bond surrogates are among a variety of terms that can be used to describe backbone-modified peptides. These synthetic analogs of peptides have a variety of potential uses, but most of the expanded interest in these areas focuses on their potential for developing metabolically stabilized and perhaps orally active peptide hormone analogs or enzyme inhibitors with enhanced biological potency. The term specifically includes peptide back-bone modifications (i.e., amide bond mimetics) known to those skilled in the art. Such modifications include modifications of the amide nitrogen, the α-carbon, amide carbonyl, complete replacement of the amide bond, extensions, deletions or backbone crosslinks. Several peptide backbone modifications are known, including ψ[OPH23] ψ[OPH2NH] ψ[CSNH2], ψ[NHCO], ψ[COCH2], and ψ[(E) or (Z) CH=CH]. In the nomenclature used above, ψ indicates the absence of an amide bond. The structure that replaces the amide group is specified within the brackets.

As used herein, when two entities are "conjugated" to one another they are linked by a direct or indirect covalent or non-covalent interaction. In certain aspects, the association is covalent. In other aspects, the association is non-covalent. Non-covalent interactions include hydrogen bonding, van der Waals interactions, hydrophobic interactions, magnetic interactions, electrostatic interactions, etc. An indirect covalent interaction is when two entities are covalently connected, optionally through a linker group. "Conjugation" means herein that a peptide is conjugated or coupled to a detectable chemical or biochemical moiety, or PEG or other moieties that are used to prolong plasma half-life. In some instances, one or more peptides disclosed herein can be conjugated, for example, to a carrier protein. Such conjugated compositions can be monovalent or multivalent. For example, conjugated compositions can include one peptide disclosed herein conjugated to a carrier protein. Alternatively, conjugated compositions can include two or more peptides disclosed herein conjugated to a carrier.

The "blood-brain barrier" or "BBB" is a highly selective semipermeable membrane barrier that separates the circulating blood from the brain and extracellular fluid in the central nervous system. The BBB is formed is formed by endothelial cells of the capillary wall, astrocyte end-feet ensheathing the capillary, and pericytes embedded in the capillary basement membrane. The system allows the passage of some molecules by passive diffusion, as well as the selective transport of molecules such as glucose, water and amino acids that are crucial to neural function. Large molecules such as proteins typically cannot pass through the BBB. However, some peptides can cross the BBB through various mechanisms, and also some proteins, that contain specific recognition motifs for transporter proteins residing at the surface of brain vascular endothelial cells, can get transported across the BBB.

As used herein, "pharmaceutically acceptable carrier" may include one or more solvents, buffers, solutions, dispersion media, coatings, antibacterial and antifungal agents, isotonic and absorption delaying agents and the like acceptable for use in formulating pharmaceuticals, such as pharmaceuticals suitable for administration to humans. The use of such media and agents for pharmaceutically active substances is well known in the art. Supplementary active ingredients also can be incorporated into the compositions.

CDNF and MANF share ca. 60% amino acid sequence homology (Figs 9 and 10), but they have highly similar three-dimensional structures. Both CDNF and MANF are composed of two independently folded domains connected by a flexible loop region. The secondary structure is predominantly α-helical, with five α-helices in the N-terminal domain, and three α-helices in the C-terminal domain. Three disulphide bridges stabilize the N-terminal domain while the C-terminal CRAC (SEQ ID NO: 36) sequence (CKGC) (SEQ ID NO: 37) in MANF) forms an internal disulphide bridge. This CXXC (SEQ ID NO: 38) disulfide bridge is found both in CDNF and MANF. The CXXC (SEQ ID NO: 38) motif is beneficial for the neuroprotective activity of MANF and CDNF. However, the data presented here show that the CXXC (SEQ ID NO: 38) motif can accommodate some modifications, such as addition of a small amino acid (e.g. glycine and serine), i.e. specific types of CXXXC motifs can also be used. In some aspects, CDNF has a sequence derived from NP_001025125.2 (SEQ ID NO: 41). In some aspects, MANF has a sequence derived from NP_006001.5 (SEQ ID NO: 42).

In addition to naturally occurring allelic variants derived from MANF and CDNF peptides, changes can be introduced by mutation into MANF/CDNF sequences that incur alterations in the amino acid sequences of the encoded MANF/CDNF peptide. Nucleotide substitutions leading to amino acid substitutions at "non-essential" amino acid residues can be made in the sequence of a MANF/CDNF peptide. MANF/CDNF peptides or functional fragments thereof comprising one or more "non-essential" substitutions can be seen as equivalents to wild-type MANF/CDNF peptides disclosed herein.

Each amino acid can be a natural or non-natural amino acid. The term "non-natural amino acid" refers to an organic compound that is a congener of a natural amino acid in that it has a structure similar to a natural amino acid so that it mimics the structure and reactivity of a natural amino acid. The non-natural amino acid can be a modified amino acid, and/or amino acid analog, that is not one of the 20 common naturally occurring amino acids or the rare natural amino acids selenocysteine or pyrolysine.

Examples of suitable amino acids include, but are not limited to, alanine, alloisoleucine, arginine, asparagine, aspartic acid, cysteine, cyclohexylalanine, 2,3-diaminopropionic acid, 4-fluorophenylalanine, glutamine, glutamic acid, glycine, histidine, homoproline, isoleucine, leucine, lysine, methionine, naphthylalanine, norleucine, phenylalanine, phenylglycine, pipecolic acid, proline, pyroglutamic acid, sarcosine, serine, selenocysteine, threonine, tryptophan, tyrosine, valine, a derivative, or combinations thereof.

The term "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

A "pharmaceutically acceptable salt" is intended to mean a salt of a free acid or base of a compound represented herein that is non-toxic, biologically tolerable, or otherwise biologically suitable for administration to the subject. See, generally, S.M. Berge, et al., "Pharmaceutical Salts," J. Pharm. Sci., 1977, 66, 1-19. Preferred pharmaceutically acceptable salts are those that are pharmacologically effective and suitable for contact with the tissues of subjects without undue toxicity, irritation, or allergic response. A compound described herein may possess a sufficiently acidic group, a sufficiently basic group, both types of functional groups, or more than one of each type, and accordingly react with a number of inorganic or organic bases, and inorganic and organic acids, to form a pharmaceutically acceptable salt.

For a compound described herein that contains a basic group, such as an amine, a pharmaceutically acceptable salt may be prepared by any suitable method available in the art, e.g., treatment of the free base with an inorganic acid, such as hydrochloric acid, hydrobromic acid, sulfuric acid, sulfamic acid, nitric acid, boric acid, phosphoric acid, and the like, or with an organic acid, such as acetic acid, phenylacetic acid, propionic acid, stearic acid, lactic acid, ascorbic acid, maleic acid, hydroxymaleic acid, isethionic acid, succinic acid, valeric acid, fumaric acid, malonic acid, pyruvic acid, oxalic acid, glycolic acid, salicylic acid, oleic acid, palmitic acid, lauric acid, a pyranosidyl acid, such as glucuronic acid or galacturonic acid, an alpha-hydroxy acid, such as mandelic acid, citric acid, or tartaric acid, an amino acid, such as aspartic acid or glutamic acid, an aromatic acid, such as benzoic acid, 2-acetoxybenzoic acid, naphthoic acid, or cinnamic acid, a sulfonic acid, such as laurylsulfonic acid, p-toluenesulfonic acid, methanesulfonic acid, or ethanesulfonic acid, or any compatible mixture of acids such as those given as examples herein, and any other acid and mixture thereof that are regarded as equivalents or acceptable substitutes in light of the ordinary level of skill in this technology.

For a compound described herein that contains an acidic group, such as a carboxylic acid group, base addition salts can be prepared by any suitable method available in the art, e.g., treatment of such compound with a sufficient amount of the desired the desired base, either neat or in a suitable inert solvent. Examples of pharmaceutically acceptable base addition salts include, but are not limited to, lithium, sodium, potassium, calcium, ammonium, zinc, or magnesium salt, or other metal salts; organic amino salts, such as, alkyl, dialkyl, trialkyl, or tetra-alkyl ammonium salts.

Other examples of pharmaceutically acceptable salts include, but are not limited to, camsylate, sulfates, pyrosulfates, bisulfates, sulfites, bisulfites, phosphates, monohydrogenphosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, methylsulfonates, propylsulfonates, besylates, xylenesulfonates, naphthalene-1-sulfonates, naphthalene-2-sulfonates, phenylacetates, phenylpropionates, phenylbutyrates, citrates, lactates, γ-hydroxybutyrates, glycolates, tartrates, and mandelates. Lists of other suitable pharmaceutically acceptable salts are found in Remington's Pharmaceutical Sciences, 17th Edition, Mack Publishing Company, Easton, Pa., 1985.

The neutral forms of the compounds are preferably regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present application.

In the embodiments of the disclosure, the length of the macrocyclic peptide or fragment is in the range of 8 - 32 amino acids wherein the macrocyclic peptide or fragment thereof comprises CX₁X₂X₃C (SEQ ID NO: 25) as described herein. In certain embodiments, the preferred peptides or fragments can consist of 8, 9, 10, 11, 12, 13, 14, 15, 16, 17, 18, 19, 20, 21, 22, 23, 24, 25, 26, 27, 28, 29, 30, 31, or 32 amino acids. In certain embodiments, the length of the peptide or fragment is in the range of 8-31, 8-29, 8-27, 8-25, 8-23, 8-21, 8-19, 8-17, 8-15, 11-27, 11-25, 11-23, 11-21, 11-19, 11-17, 11-15, 13-25, 13-23, 13-21, 13-19, 13-17, 13-15, 23-27, 24-27, or 25-27 amino acids. In certain embodiments, the length of the peptide or fragment is in the range of 12 - 27, 13 - 27, 12 - 23, 13 - 23 or 23 - 27 amino acids. The peptides or fragment thereof may comprise any of the naturally occurring amino acids such as alanine [Ala (A)], arginine [Arg (R)], asparagine [Asn (N)], aspartic acid [Asp (D)], cysteine [Cys (C)], glutamine [Gln (Q)], glutamic acid [Glu (E))], glycine [Gly (G)], histidine [His (H)], isoleucine [Ile (I)], leucine [Leu (L)], lysine (Lys (K)], methionine [Met (M)], phenylalanine Phe (F)], proline [Pro (P)], serine [Ser (S)], threonine [Thr (T)], tryptophan [Trp (W)], tyrosine [Tyr (Y)], and valine [Val (V)] as well as non-natural or modified amino acids.

Cyclotides are small disulfide rich peptides isolated from plants. Cyclotides typically contain 28-37 amino acids, they have head-to-tail cyclized peptide backbones and interlocking arrangement of three disulfide bonds. Although the family of plant cyclotides may contain macrocyclic peptides with potential CXXC and CXXXC motifs, they are not known to have similar cytoprotective properties in mammalian cells as CDNF and MANF do, i.e. protection from ER stress induced apoptosis.

Preferably, the peptides disclosed in the present disclosure are not from thioredoxin and/or protein disulphide isomerase families of proteins.

The present disclosure provides a macrocyclic peptide having a length of 8 - 32 amino acids, or a pharmaceutically acceptable salt thereof comprising an amino acid sequence of C-X₁-X₂-X₃-C (SEQ ID NO: 25),
wherein
X₁ is selected from the group consisting of R, K, I, G, A and S;
X₂ is absent or selected from the group consisting of G, A, R, K, I and S; and
X₃ is selected from the group consisting of A, G and S.
In one embodiment, the macrocyclic peptide is one that has a linkage between the N-terminus and the C-terminus of the peptide.

In a preferred embodiment the macrocyclic peptide comprises an amino acid sequence of E-X4-C-X₁-X₂-X₃-C-A-E (SEQ ID NO: 26),
wherein
X₁ is selected from the group consisting of R, K, I, G, A and S;
X₂ is absent or selected from the group consisting of G, A, R, K, I, and S;
X₃ is selected from the group consisting of A, G and S; and
X₄ is selected from the group consisting of E, T, V, D, M and G.
In one embodiment, the macrocyclic peptide is one that has a linkage between the N-terminus and the C-terminus of the peptide.

Based on the natural variation in CDNF and MANF sequences in different species (human, horse, bison, pig, dog, mouse, hamster, alligator, dolphin and zebrafish CDNF and MANF are used as example sequences in Fig. 10), limited changes in the peptide sequence regarding X-groups compared to the human sequences can be accommodated without losing biological activity.

In another preferred embodiment the macrocyclic peptide comprises an amino sequence of X₅-X₆-X₇-X₈-E-X₄-C-X₁-X₂-X₃-C-A-E-X₉-X₁₀-X₁₁ (SEQ ID NO: 27),
wherein
X₁ is selected from the group consisting of R, K, I, G, A, and S;
X₂ is absent or selected from the group consisting of G, A, R, K, I and S;
X₃ is selected from the group consisting of A, G and S;
X₄ is selected from the group consisting of E, T, V, D, M and G;
X₅ is absent or selected from the group consisting of H, D, Q, R, Y, N and S;
X₆ is absent or selected from the group consisting of S, D, G, N and R;
X₇ is absent or W;
X₈ is absent or G;
X₉ is absent or K;
X₁₀ is absent or selected from the group consisting of T, S, A, I and N; and
X₁₁ is absent or selected from D and E.
In one embodiment, the macrocyclic peptide is one that has a linkage between the N-terminus and the C-terminus of the peptide.

In another preferred embodiment the macrocyclic peptide comprises an amino sequence, which is within an amino acid sequence of X₁₂-X₁₃-X₁₄-X₁₅-X₁₆-X₁₇-X₁₈-X₁₉-X₂₀-X₂₁-X₂₂-X₂₃-V-X₂₄-E-L-K-X₂₅-X₂₆-L-X₅-X₆-X₇-X₈-E-X₄-C-X₁-X₂-X₃-C-A-E-X₉-X₁₀-X₁₁ (SEQ ID NO: 28),
wherein
X₁ is selected from the group consisting of R, K, I, G, A and S;
X₂ is absent or selected from the group consisting of G, A, R, K, I and S;
X₃ is selected from the group consisting of A, G and S;
X₄ is selected from the group consisting of E, T, V, D, M and G;
X₅ is absent or selected from the group consisting of H, D, Q, R, Y, N and S;
X₆ is absent or selected from the group consisting of S, D, G, N and R;
X₇ is absent or W;
X₈ is absent or G;
X₉ is absent or K;
X₁₀ is absent or selected from the group consisting of T, S, A, I and N;
X₁₁ is absent or selected from D and E,
X₁₂ is absent or selected from the group consisting of L, I and V;
X₁₃ is absent or D;
X₁₄ is absent or selected from L and W;
X₁₅ is absent or selected from the group consisting of A, S, T, E and N;
X₁₆ is absent or selected from S and T;
X₁₇ is absent or selected from V and D;
X₁₈ is absent or selected from D and A;
X₁₉ is absent or L;
X₂₀ is absent or selected from the group consisting of R, K, S and W;
X₂₁ is absent or K;
X₂₂ is absent or selected from the group consisting of M, L, I and V;
X₂₃ is absent or R;
X₂₄ is selected from the group consisting of A, K, T, L and V;
X₂₅ is selected from the group consisting of Q, K and R; and
X₂₆ is selected from I and V.
In one embodiment, the macrocyclic peptide is one that has a linkage between the N-terminus and the C-terminus of the peptide.

In some embodiments, the macrocyclic peptide comprises an 8-27 amino acid long peptide within SEQ ID NO: 28, wherein the macrocyclic peptide includes the CX₁X₂X₃C (SEQ ID NO: 25) motif.

In another preferred embodiment the macrocyclic peptide comprises an amino sequence, which is within an amino acid sequence selected from the group consisting of: 
X16-X17-X18-X19-X20-X21-X22-X23-V-X24-E-L-K-X25-X26-L-X5-X6-X7-X8-E-X4-C-X1-X2-X3-C-A-E-X9-X10-
X11 (SEQ ID NO: 31),
 
X15-X16-X17-X18-X19-X20-X21-X22-X23-V-X24-E-L-K-X25-X26-L-X5-X6-X7-X8-E-X4-C-X1-X2-X3-C-A-E-X9-
X10 (SEQ ID NO: 32),
 
X14-X15-X16-X17-X18-X19-X20-X21-X22-X23-V-X24-E-L-K-X25-X26-L-X5-X6-X7-X8-E-X4-C-X1-X2-X3-C-A-E-
X9 (SEQ ID NO: 33), and
 
X13-X14-X15-X16-X17-X18-X19-X20-X21-X22-X23-V-X24-E-L-K-X25-X26-L-X5-X6-X7-X8-E-X4-C-X1-X2-X3-C-A-
E (SEQ ID NO: 34).
 In some embodiments, the macrocyclic peptide comprises an 8-27 amino acid long peptide within any one of SEQ ID NOs: 31-34, wherein the peptide includes the CX₁X₂X₃C (SEQ ID NO: 25) motif.

In certain instances, the macrocyclic peptide comprises an amino acid sequence which may be within an amino acid sequence SEQ ID NO: 43 or within an amino acid sequence SEQ ID NO: 47.

Preferably, the macrocyclic peptide comprises or consists of a sequence selected from the group consisting of: VDLRKMRVAELKQILHSWGEECRACAE (SEQ ID NO: 2), VDLKKLRVKELKKILDDWGETCKGCAE (SEQ ID NO: 4), MRVAELKQILHSWGEECRACAEK (SEQ ID NO: 6), LRVKELKKILDDWGETCKGCAEK (SEQ ID NO: 8), WGEECRACAEKT (SEQ ID NO: 10), WGETCKGCAEKS (SEQ ID NO: 12), WGEECRGACAEKT (SEQ ID NO: 22), and WGETCKGGCAEKS (SEQ ID NO: 24).

In one embodiment, the macrocyclic peptide is one that has a linkage between the N-terminus and the C-terminus of the peptide.

In certain instances, the macrocyclic peptide is 11-32 amino acids in length. In certain instances, the peptide is 12-32 amino acids in length. In certain instances, the peptide is 12-27 amino acids in length. In certain instances, the peptide is 8-27 amino acids in length. In certain instances, the peptide is 8-13 amino acids in length. In certain instances, the peptide is 8-12 amino acids in length.

In a preferred embodiment in the macrocyclic peptide cysteine (C) is in a reduced form or in disulphide bridged form.

In certain embodiments, the macrocyclic peptide described herein binds to GRP78.
In some aspects, the macrocyclic peptide described herein is 1.5-fold more stable than its linear counterpart.

In some aspects, the macrocyclic peptide described has a half-life that is 1.5-fold higher than its linear counterpart.

In some aspects, the macrocyclic peptide may comprise a linkage connecting the N-terminus to the C-terminus of the peptide.

In some aspects, the N-terminus of the peptide may be acetylated.

In some aspects, the C-terminus of the peptide is amidated.

In some aspects the N-terminus of the peptide may be acetylated and the C-terminus of the peptide may be amidated.

In another preferred embodiment the macrocyclic peptide is conjugated to a detectable moiety, chemical moiety, biochemical moiety, or polyethylene glycol (PEG).

The macrocyclic peptide may be conjugated to a detectable chemical or biochemical moiety such as a fluorophore (e.g. fluorescein or rhodamine). Radiolabeling of the peptide may be used, e.g. for use in SPECT or PET imaging. As used herein, a "detectable chemical or biochemical moiety" means a chemical tag that exhibits an amino acid sequence or a detectable chemical or biochemical moiety for the purpose of facilitating detection of the peptide; such as a detectable molecule selected from among: a visible, fluorescent, chemiluminescent, or other detectable chemical tag; an enzyme that is detectable in the presence of a substrate, e.g., an alkaline phosphatase with NBT plus BCIP or a peroxidase with a suitable substrate; a detectable protein, e.g., a green fluorescent protein. Preferably, the tag does not prevent or hinder the penetration of the fragment into a target cell or otherwise alter the biological activity of the compound.

N- and/or C-terminal modifications of the C-terminal CDNF fragments or C-terminal MANF fragments to further increase the stability and/or cell permeability of the peptides or fragments are also preferred. Acetylation - amidation of the termini of the CDNF fragment or MANF fragment (i.e. N-terminal acetylation and C-terminal amidation) is one of the options known in the art (see e.g. Marino et al. 2015, ACS Chem. Biol. 10: 1754-1764).
For side-chain-to-side-chain cyclized peptides acetylation - amidation of the termini of the peptide (i.e., N-terminal acetylation and C-terminal amidation) increases the stability and cell permeability of the peptides.

In some instances, the macrocyclic peptide has at least one (e.g., 1, 2, 3, 4, 5, 6, or 7) of the following properties: (i) the peptide can dose-dependently protect TH-positive neurons from MPP⁺ toxicity; (ii) the peptide reduces the number of a-synuclein inclusions in TH-positive neurons; (iii) the peptide has improved stability in plasma compared to its linear counterpart; (iv) the peptide has improved stability in hepatocytes compared to its linear counterpart; or (v) the peptide has improved ability to pass through the blood brain barrier compared to its linear counterpart.

An embodiment provides the macrocyclic peptide as described herein for use as a medicament.

Since CDNF/MANF peptides potently protected the dopamine neurons from death the prior art such as WO2009133247, and EP 1969003 shows that the peptides can be used in the treatment of central nervous system (CNS) diseases such as Alzheimer's disease, Parkinson's disease (PD), multiple system atrophy, amyotrophic lateral sclerosis (ALS), frontotemporal lobar degeneration, dementia with Lewy bodies, mild cognitive impairment, Huntington's disease (HD), traumatic brain injury, drug addiction and stroke.

CDNF and MANF modulate signaling of the unfolded protein response (UPR) pathway and protect cells from ER stress-related cell death. ER stress is known to play an important pathophysiological role in diverse chronic diseases, such as neurodegenerative and metabolic diseases and acute injuries (Wang and Kaufman, 2016). GRP78 (a.k.a. BiP and HSPA5) is a major ER lumenal chaperone and a master regulator of the UPR (Bertolotti et al, 2000; Wang and Kaufman, 2016). Dynamic association and dissociation of GRP78 with UPR receptors IRE1α, PERK and ATF6 is a key step regulating the signaling activity of the UPR receptors under ER stress. Interaction of MANF with GRP78 regulates its cellular activities (Yan et al, 2019).

Accordingly, the present disclosure is directed to a method for treatment of a degenerative, chronic, or progressive disease or disorder, such as a CNS disease or disorder, or a monogenic hereditary disease (having ER stress as a pathogenic component), wherein a pharmaceutically effective amount of the macrocyclic peptide with the length of 8 - 32 amino acids or a pharmaceutically acceptable salt thereof comprising the sequence C-X₁-X₂-X₃-C (SEQ ID NO: 25), E-X₄-C-X₁-X₂-X₃-C-A-E (SEQ ID NO: 26), X₅-X₆-X₇-X₈-E-X₄-C-X₁-X₂-X₃-C-A-E-X₉-X₁₀-X₁₁ (SEQ ID NO: 27) or X₁₂-X₁₃-X₁₄-X₁₅-X₁₆-X₁₇-X₁₈-X₁₉-X₂₀-X₂₁-X₂₂-X₂₃-V-X₂₄-E-L-K-X₂₅-X₂₆-L-X₅ X₆-X₇-X₈-E-X₄-C-X₁-X₂-X₃-C-A-E-X₉-X₁₀-X₁₁ (SEQ ID NO: 28), is administered to a patient.

Another embodiment provides the macrocyclic peptide for use in the treatment of a degenerative, chronic, or progressive disease or disorder, such as a neurodegenerative disease or disorder.
Said neurodegenerative disease or disorder is preferably a central nervous system disease selected from the group consisting of: Parkinson's disease, Alzheimer's disease, multiple system atrophy, amyotrophic lateral sclerosis, frontotemporal lobar degeneration, dementia with Lewy bodies, mild cognitive impairment, Huntington's disease, traumatic brain injury, traumatic spinal cord injury, progressive supranuclear palsy, Pick's disease, pure autonomic failure, corticobasal degeneration, chronic traumatic encephalopathy, spinocerebellar ataxia, bipolar disorder, and peripheral neuropathy.

Another embodiment provides the macrocyclic peptide for use in the treatment of a monogenic hereditary disease selected from the group consisting of: Wolcott-Rallison syndrome, Wolfram syndrome, Marinesco-Sjögren syndrome, Machado-Joseph disease, and degenerative retinal diseases such as retinitis pigmentosa, and inherited nephrotic syndromes such as primary nephrotic syndrome and autosomal dominant polycystic kidney disease. Said monogenic hereditary disease is a disease having ER stress as a pathogenic component.

An embodiment provides the macrocyclic peptide for use according to the present disclosure, wherein said peptide is administered by peripheral administration such as intravenous, intraarterial, subcutaneous, intranasal, intraocular, intratympanic, or topical administration, enteral, parenteral or topical routes including oral, rectal, sublingual or buccal administration, intraperitoneal, intramuscular, intraarticular, transdermal, intracochlear, topic ocular, or inhalational administration, or intracranial, intrathecal, epidural or intralesional administration.

### Pharmaceutical compositions

One or more of the macrocyclic peptides disclosed herein can be formulated for use as or in pharmaceutical compositions. Such compositions can be formulated or adapted for administration to a subject via any route, e.g., any route approved by the appropriate authorities.

An embodiment provides a pharmaceutical composition comprising the macrocyclic peptide as described herein and at least one of the following: a pharmaceutically acceptable carrier, a pharmaceutically acceptable excipient, preservative, stabilizer and/or diluent.

In one embodiment, the present disclosure is further directed to a pharmaceutical composition comprising the macrocyclic peptide with the length of 8 - 32 amino acids or a pharmaceutically acceptable salt thereof, comprising the sequence C-X₁-X₂-X₃-C (SEQ ID NO: 25), E-X₄-C-X₁-X₂-X₃-C-A-E (SEQ ID NO: 26), X₅-X₆-X₇-X₈-E-X₄-C-X₁-X₂-X₃-C-A-E-X₉-X₁₀-X₁₁ (SEQ ID NO: 27) or X₁₂-X₁₃-X₁₄-X₁₅-X₁₆-X₁₇-X₁₈-X₁₉-X₂₀-X₂₁-X₂₂-X₂₃-V-X₂₄-E-L-K-X₂₅-X₂₆-L-X₅-X₆-X₇-X₈-E-X₄-C-X₁-X₂-X₃-C-A-E-X₉-X₁₀-X₁₁ (SEQ ID NO: 28).

In some instances, pharmaceutical compositions can include an effective amount of one or more macrocyclic peptides. The terms "effective amount" and "effective to treat," as used herein, refer to an amount or a concentration of one or more compounds or a pharmaceutical composition described herein utilized for a period of time (including acute or chronic administration and periodic or continuous administration) that is effective within the context of its administration for causing an intended effect or physiological outcome.

In one embodiment of the present disclosure, the macrocyclic peptide can be incorporated into pharmaceutical compositions. Such compositions of the disclosure are prepared for storage by mixing the peptide having the desired degree of purity with optional physiologically acceptable carriers (such as nanocarriers), excipients, buffers or stabilizers (Remington's Pharmaceutical Sciences, 22nd edition, Allen, Loyd V., Jr, Ed., (2012)), in the form of lyophilized cake or aqueous solutions. Acceptable carriers, excipients, or stabilizers are non-toxic to recipients at the dosages and concentrations employed, and include buffers such as phosphate, citrate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine, or lysine; monosaccharides, disaccharides, and other carbohydrates including glucose, mannose, or dextrins; chelating agents such as EDTA; sugar alcohols such as mannitol or sorbitol; salt-forming counter-ions such as sodium; and/or non-ionic surfactants such as Tween, Pluronics, polyethylene glycol (PEG), or excipients that are used to enhance nose-to-brain delivery, such as chitosan, methylated pectin, alkylsaccharide-based mucosal absorption enhancers, and hydroxy fatty-acyl esters of PEG.

The actual dosage amount of the peptide (e.g., an effective amount) that is administered to a patient can be determined by physical and physiological factors such as body weight, severity of condition, the type of disease being treated, previous or concurrent therapeutic interventions, idiopathy of the patient and on the route of administration. The practitioner responsible for administration can determine the concentration of active ingredient(s) in a composition and appropriate dose(s) for the individual subject.

The peptides may also be entrapped in microcapsules prepared, for example, by coacervation techniques or by interfacial polymerization (for example, hydroxymethylcellulose or gelatin-microcapsules and poly-(methylmethacylate) microcapsules, respectively), in colloidal drug delivery systems (for example, liposomes, albumin microspheres, microemulsions, nano-particles, and nanocapsules), or in macroemulsions. Such techniques are disclosed in Remington's Pharmaceutical Sciences, 22nd edition, Allen, Loyd V., Jr, Ed., (2012).

In an embodiment, pharmaceutical compositions may comprise, for example, at least about 0.1% of an active compound. In other embodiments, an active compound may comprise between about 2% to about 75% of the weight of the unit, or between about 25% to about 60%, for example, and any range derivable therein.

In other non-limiting examples, a dose of a pharmaceutical composition or formulation can comprise from about 1 ng/kg/body weight of the macrocyclic peptide, about 5 ng/kg/body weight, about 10 ng/kg/body weight, about 50 ng/kg/body weight, about 100 ng/kg/body weight, about 200 ng/kg/body weight, about 350 ng/kg/body weight, about 500 ng/kg/body weight, 1 µg/kg/body weight, about 5 µg/kg/body weight, about 10 µg/kg/body weight, about 50 µg/kg/body weight, about 100 µg/kg/body weight, about 200 µg/kg/body weight, about 350 µg/kg/body weight, about 500 µg/kg/body weight, about 1 mg/kg/body weight, about 5 mg/kg/body weight, about 10 mg/kg/body weight, about 50 mg/kg/body weight, about 100 mg/kg/body weight, about 200 mg/kg/body weight, about 350 mg/kg/body weight, about 500 mg/kg/body weight, to about 1000 mg/kg/body weight of the peptide or more per administration, and any range derivable therein. In non-limiting examples of a derivable range from the numbers listed herein, a range of about 5 mg/kg/body weight to about 100 mg/kg/body weight, about 5 µg/kg/body weight to about 500 mg/kg/body weight of peptide, etc., can be administered, based on the numbers described above.

The methods herein contemplate administration of an effective amount of compound or compound composition to achieve the desired or stated effect. Typically, the pharmaceutical compositions of this disclosure will be administered from about 1 to about 6 times per day or, alternatively, as a continuous infusion. Such administration can be used as a chronic or acute therapy. The amount of active ingredient that may be combined with the carrier materials to produce a single dosage form will vary depending upon the host treated and the particular mode of administration. A typical preparation will contain from about 5% to about 95% active compound (w/w). Alternatively, such preparations contain from about 20% to about 80% active compound.

Dosing can be determined using various techniques. The selected dosage level can depend upon a variety of factors, including, e.g., the activity of the particular compound employed, the route of administration, the time of administration, the rate of excretion or metabolism of the particular compound being employed, the duration of the treatment, other drugs, compounds, and/or materials used in combination with the particular compound employed, the age, sex, weight, condition, general health, and/or prior medical history of the patient being treated, and like factors well known in the medical arts. The dosage values can also vary with the severity of the condition to be alleviated. For any particular subject, specific dosage regimens can be adjusted over time according to the individual need and the professional judgment of the person administering or supervising the administration of the compositions.

In some aspects, a suitable daily dose of a compound of the disclosure can be that amount of the compound which is the lowest dose effective to produce a therapeutic effect. Such an effective dose will generally depend upon the factors described above. The precise time of administration and amount of any particular compound that will yield the most effective treatment in a given patient will depend upon the activity, pharmacokinetics, and bioavailability of a particular compound, physiological condition of the patient (including age, sex, disease type and stage, general physical condition, responsiveness to a given dosage and type of medication), route of administration, and the like.

A physician or veterinarian can prescribe the effective amount of the pharmaceutical composition required. For example, the physician or veterinarian could start doses of the compounds of the disclosure employed in the pharmaceutical composition at levels lower than that required in order to achieve the desired therapeutic effect and gradually increase the dosage until the desired effect is achieved.

Pharmaceutical compositions described herein can be in unit dosage forms suitable for single administration of precise dosages. In unit dosage form, the formulation is divided into unit doses containing appropriate quantities of one or more compounds. The unit dosage can be in the form of a package containing discrete quantities of the formulation. Non-limiting examples are liquids in vials or ampoules. Aqueous suspension compositions can be packaged in single-dose non-reclosable containers. Multiple-dose reclosable containers can be used, for example, in combination with a preservative. Formulations for parenteral injection can be presented in unit dosage form, for example, in ampoules, or in multi dose containers with a preservative.

The term "pharmaceutically-acceptable carrier or adjuvant" refers to a carrier or adjuvant that may be administered to a patient, together with a compound of this invention, and which does not destroy the pharmacological activity thereof and is nontoxic when administered in doses sufficient to deliver a therapeutic amount of the compound.

Pharmaceutically-acceptable carriers, adjuvants and vehicles that may be used in the pharmaceutical compositions of the present disclosure include, but are not limited to, ion exchangers, alumina, aluminum stearate, lecithin, self-emulsifying drug delivery systems (SEDDS) such as d-.alpha.-tocopherol polyethyleneglycol 1000 succinate, surfactants used in pharmaceutical dosage forms such as Tweens or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The pharmaceutical compositions of this disclosure may contain any conventional non-toxic pharmaceutically-acceptable carriers, adjuvants, or vehicles. In some cases, the pH of the formulation may be adjusted with pharmaceutically-acceptable acids, bases, or buffers to enhance the stability of the formulated compound or its delivery form. The term parenteral as used herein includes parenteral, epidural, subcutaneous, intra-cutaneous, intra-venous, intra-muscular, intra-articular, intra-arterial, intra-synovial, intra-sternal, intra-thecal, intra-lesional and intra-cranial injection or infusion techniques.

An effective amount of a compound of the disclosure can be administered in either single or multiple doses by any of the accepted modes of administration. Regardless of the route of administration selected, the compounds of the present disclosure, and/or the pharmaceutical compositions of the present disclosure, are formulated into pharmaceutically-acceptable dosage forms. The compounds according to the disclosure can be formulated for administration in any convenient way for use in human or veterinary medicine, by analogy with other pharmaceuticals.

In one aspect, the disclosure provides pharmaceutical formulation comprising a therapeutically-effective amount of one or more of the compounds described above, formulated together with one or more pharmaceutically acceptable carriers (additives) and/or diluents. In one aspect, one or more of the compounds described herein are formulated for parenteral administration for parenteral administration, one or more compounds disclosed herein can be formulated as aqueous or non-aqueous solutions, dispersions, suspensions, or emulsions or sterile powders which can be reconstituted into sterile injectable solutions or dispersions just prior to use. Such formulations can comprise sugars, alcohols, antioxidants, buffers, bacteriostats, solutes which render the formulation isotonic with the blood of the intended recipient or suspending or thickening agents. These compositions can also contain adjuvants such as preservatives, wetting agents, emulsifying agents and dispersing agents. Prevention of the action of microorganisms upon the subject compounds can be ensured by the inclusion of various antibacterial and antifungal agents, for example, paraben, chlorobutanol, phenol sorbic acid, and the like. It can also be desirable to include isotonic agents, such as sugars, sodium chloride, and the like into the compositions. In addition, prolonged absorption of the injectable pharmaceutical form can be brought about by the inclusion of agents which delay absorption such as aluminum monostearate and gelatin. If desired, the formulation can be diluted prior to use with, e.g., an isotonic saline solution or a dextrose solution. In some examples, the compound is formulated as an aqueous solution and is administered intravenously.

Pharmaceutical compositions can be in the form of a solution or powder for injection. Such compositions may be formulated according to techniques known in the art using suitable dispersing or wetting agents (such as, for example, Tween 80) and suspending agents. The sterile injectable preparation may also be a sterile injectable solution or suspension in a non-toxic parenterally acceptable diluent or solvent, for example, as a solution in 1,3-butanediol. Among the acceptable vehicles and solvents that may be employed are mannitol, water, Ringer's solution, and isotonic sodium chloride solution. In addition, sterile, fixed oils are conventionally employed as a solvent or suspending medium. For this purpose, any bland fixed oil may be employed including synthetic mono-or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, or carboxymethyl cellulose or similar dispersing agents which are commonly used in the formulation of pharmaceutically-acceptable dosage forms such as emulsions and or suspensions. Other commonly used surfactants such as Tweens or Spans and/or other similar emulsifying agents or bioavailability enhancers which are commonly used in the manufacture of pharmaceutically-acceptable solid, liquid, or other dosage forms may also be used for the purposes of formulation.

Pharmaceutical compositions can be orally administered in any orally acceptable dosage form including, but not limited to, capsules, tablets, emulsions and aqueous suspensions, dispersions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions and/or emulsions are administered orally, the active ingredient may be suspended or dissolved in an oily phase is combined with emulsifying and/or suspending agents. If desired, certain sweetening and/or flavoring and/or coloring agents may be added.

The pharmaceutical compositions of the present disclosure may also be administered in the form of suppositories for rectal administration. These compositions can be prepared by mixing a compound of the present disclosure with a suitable non-irritating excipient that is solid at room temperature but liquid at the rectal temperature and therefore will melt in the rectum to release the active components. Such materials include, but are not limited to, cocoa butter, beeswax and polyethylene glycols.

Alternatively or in addition, pharmaceutical compositions can be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

In some instances, one or more peptides disclosed herein can be conjugated, for example, to a carrier protein. Such conjugated compositions can be monovalent or multivalent. For example, conjugated compositions can include one peptide disclosed herein conjugated to a carrier protein. Alternatively, conjugated compositions can include two or more peptides disclosed herein conjugated to a carrier.

Provided herein are methods of using a peptide described herein. For example, the methods provided herein can include administering a peptide as described herein to a patient. A patient can include both mammals and non-mammals.

A pharmaceutically acceptable carrier can be selected on the basis of the selected route of administration and standard pharmaceutical practice. For example, the compositions can be formulated into suitable pharmaceutical preparations such as solutions, suspensions, tablets, dispersible tablets, pills, capsules, powders, sustained release formulations or elixirs, for oral administration or in sterile solutions or suspensions for parenteral administration and intraperitoneal injection, as well as transdermal patch preparation, dry powder inhalers, and ointments (see, e.g., Ansel, Introduction to Pharmaceutical Dosage Forms, Fourth Edition 1985, 126). A peptide and/or an immunoglobulin may be formulated into dosage forms according to standard practices in the field of pharmaceutical preparations. See Alphonso Gennaro, ed., Remington's Pharmaceutical Sciences, 18th Edition (1990), Mack Publishing Co., Easton, Pa.

For parenteral administration, a pharmaceutical composition can include a suitable carrier or diluent such as water, an oil (particularly a vegetable oil), ethanol, saline solution, aqueous dextrose (glucose) and related sugar solutions, glycerol, or a glycol such as propylene glycol or polyethylene glycol. Solutions for parenteral administration preferably contain a water soluble salt of a peptide and/or an active agent. Stabilizing agents, antioxidant agents and preservatives may also be added. Suitable antioxidant agents include sulfite, ascorbic acid, citric acid and its salts, and sodium EDTA. Suitable preservatives include benzalkonium chloride, methyl- or propyl-paraben, and chlorbutanol. The composition for parenteral administration may take the form of an aqueous or non-aqueous solution, dispersion, suspension or emulsion.

For oral administration, a pharmaceutical composition can include one or more solid inactive ingredients for the preparation of tablets, capsules, pills, powders, granules or other suitable oral dosage forms. For example, a pharmaceutical composition can include at least one excipient such as fillers, binders, humectants, disintegrating agents, solution retarders, absorption accelerators, wetting agents absorbents or lubricating agents.

The disclosure also features a pharmaceutical composition that can further include a neural cell. The neural cell can be, for example, a neuron, a neural stem cell, or a neuronal precursor cell.

The present disclosure relates to the pharmaceutical composition comprising the macrocyclic peptide as described herein and at least one of the following: a pharmaceutically acceptable carrier, excipient, preservative, stabilizer and/or diluent for use as a medicament.

In a method of treatment, a pharmaceutically effective amount of the macrocyclic peptide as defined herein is administered to a patient. In other words, the macrocyclic peptide according to the present disclosure is for use in the treatment of a degenerative, chronic, or progressive disease or disorder, such as a CNS disease or disorder, or a monogenic hereditary disease (having ER stress as a pathogenic component).

The pharmaceutical composition is for use in the treatment of a degenerative, chronic, or progressive disease or disorder, such as a neurodegenerative disease or disorder.

Said neurodegenerative disease or disorder is a central nervous system disease selected from the group consisting of: Parkinson's disease, Alzheimer's disease, multiple system atrophy, amyotrophic lateral sclerosis, frontotemporal lobar degeneration, dementia with Lewy bodies, mild cognitive impairment, Huntington's disease, traumatic brain injury, traumatic spinal cord injury, progressive supranuclear palsy, Pick's disease, pure autonomic failure, corticobasal degeneration, chronic traumatic encephalopathy, spinocerebellar ataxia, bipolar disorder, and peripheral neuropathy.

The route of macrocyclic peptide administration is in accord with known methods as well as the general routes of injection or infusion by intravenous, intraarterial, subcutaneous, intranasal, intraocular, intratympanic, or topical administration, enteral, parenteral or topical routes including oral, rectal, sublingual or buccal administration, intracranial, intrathecal or epidural, intraperitoneal, intramuscular, intraarticular, transdermal, intracochlear, topic ocular, intralesional, or inhalational administration or sustained release systems as noted below.

Suitable examples of sustained-release preparations include semipermeable matrices of solid hydrophobic polymers containing the peptide, which matrices are in the form of shaped articles, e.g., films, or microcapsules. Examples of sustained-release matrices include polyesters, hydrogels as described by Langer et al., J. Biomed. Mater. Res., 15: 167-277 (1981) and Langer, Chem. Tech., 12:98-105 (1982) or polyvinylalcohol, polylactides (U.S. Pat. No. 3,773,919, EP 58,481), or non-degradable ethylene-vinyl acetate (Langer et al., supra).

The present disclosure is also directed to methods for treatment of a degenerative, chronic, or progressive disease or disorder, such as a CNS disease or disorder, a monogenic hereditary disease (having ER stress as a pathogenic component), wherein a pharmaceutically effective amount of the macrocyclic peptide as defined herein is administered to a patient. Preferably, said fragment is administered peripherally.

The present disclosure is also directed to a use of the macrocyclic peptide as defined herein for the manufacture of a medicament for the treatment of a degenerative, chronic, or progressive disease or disorder, such as a CNS disease or disorder, or a monogenic hereditary disease (having ER stress as a pathogenic component).

The present disclosure relates to a method for treating a degenerative, chronic, or progressive disease or disorder, such as a neurodegenerative disease or disorder in a subject in need thereof, the method comprising administering to the subject a pharmaceutical composition comprising a macrocyclic peptide as described herein.

In an embodiment, a method for treating a neurodegenerative disease or disorder such as a central nervous system disease selected from the group consisting of: Parkinson's disease, Alzheimer's disease, multiple system atrophy, amyotrophic lateral sclerosis, frontotemporal lobar degeneration, dementia with Lewy bodies, mild cognitive impairment, Huntington's disease, traumatic brain injury, traumatic spinal cord injury, progressive supranuclear palsy, Pick's disease, pure autonomic failure, corticobasal degeneration, chronic traumatic encephalopathy, spinocerebellar ataxia, bipolar disorder, and peripheral neuropathy, comprises administering to the subject a pharmaceutical composition comprising a macrocyclic peptide as described herein.

The present disclosure relates to a method for treating a monogenic hereditary disease selected from the group consisting of: Wolcott-Rallison syndrome, Wolfram syndrome, Marinesco-Sjögren syndrome, Machado-Joseph disease, and degenerative retinal diseases such as retinitis pigmentosa, and inherited nephrotic syndromes such as primary nephrotic syndrome and autosomal dominant polycystic kidney disease, the method comprising administering to the subject a pharmaceutical composition comprising a macrocyclic peptide as described herein. Said monogenic hereditary disease has ER stress as a pathogenic component.

The subject in need may be a human.

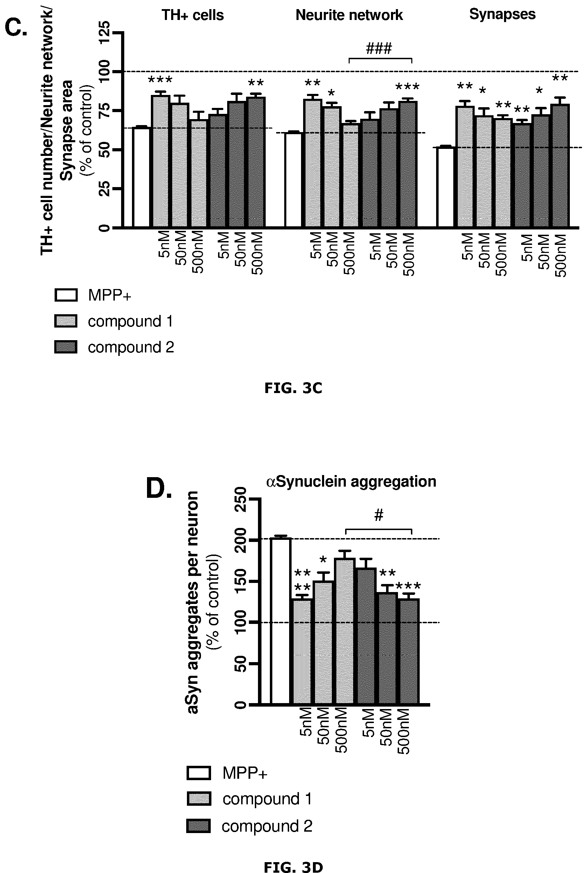

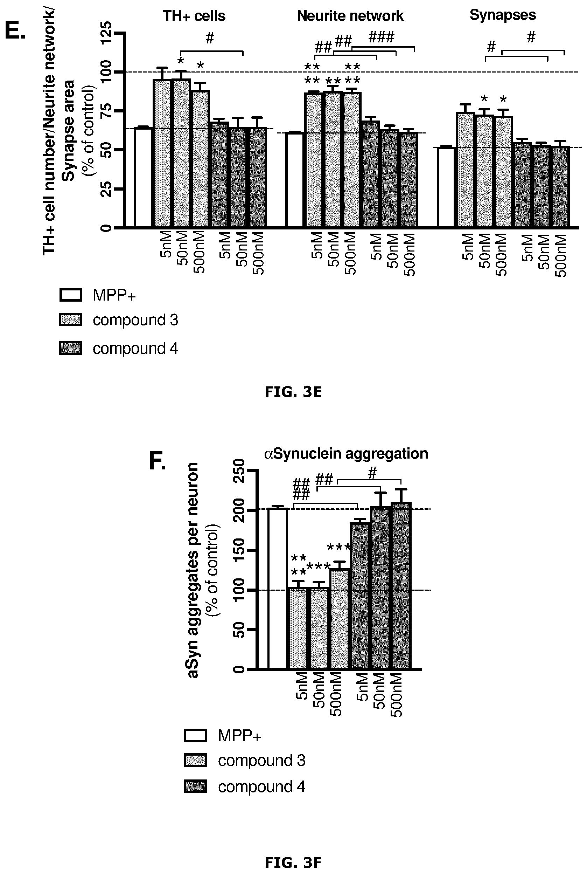

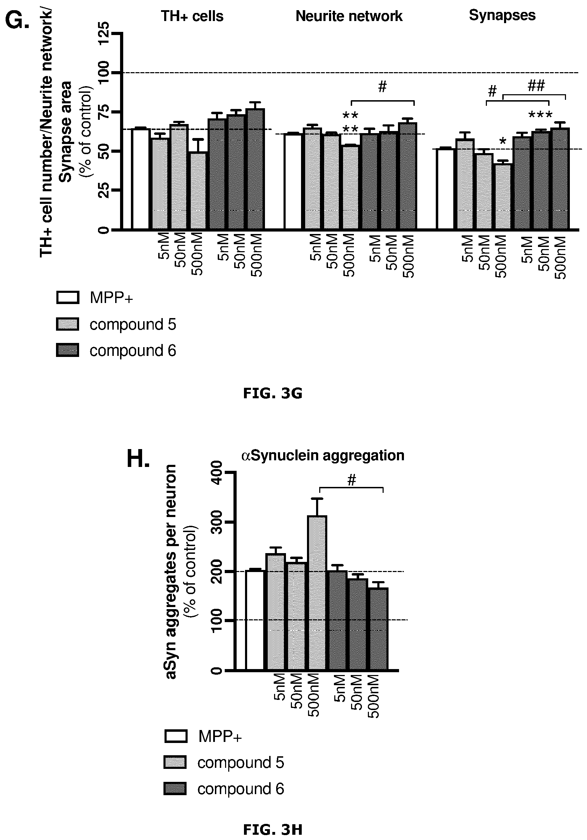

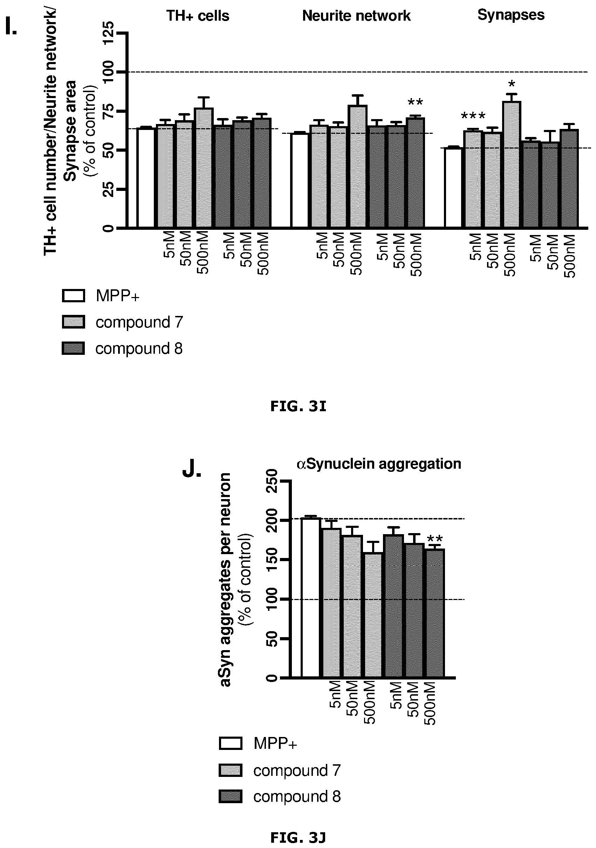

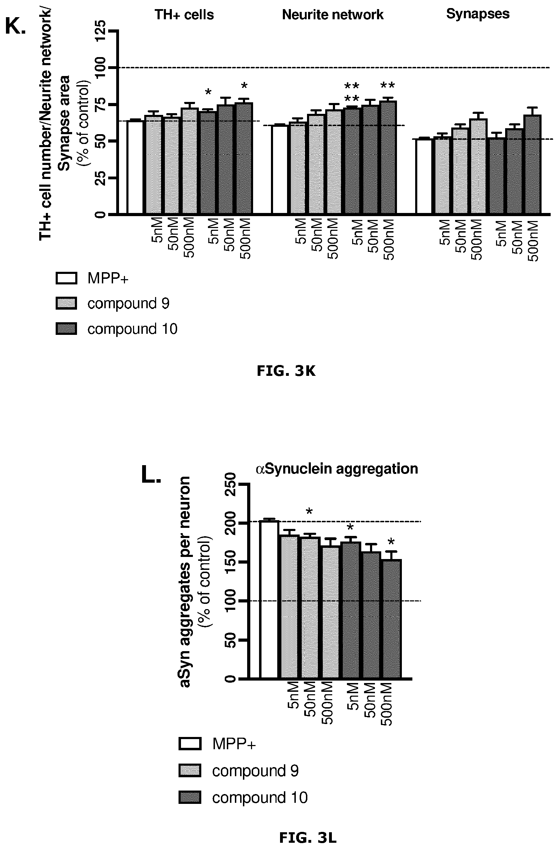

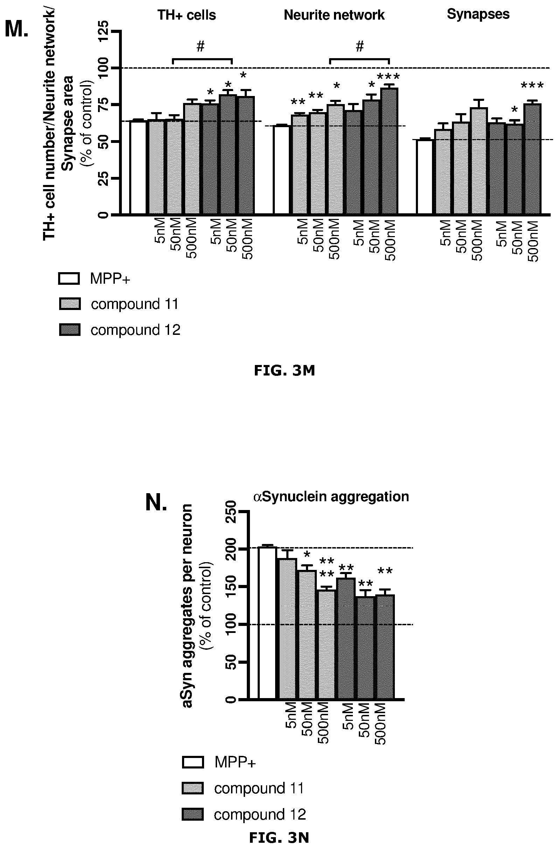

The macrocyclic peptide of the present disclosure or a pharmaceutical composition comprising said peptide can be administered continuously by infusion or by bolus injection. Generally, where the disorder permits, one should formulate and dose the fragment for site-specific delivery. Administration can be continuous or periodic. Administration can be accomplished by a constant-or programmable-flow implantable pump or by periodic injections. Peripheral or systemic administration is preferred as the present disclosure shows that macrocyclic peptides are capable of effective penetration through the neuronal cell membrane and through *in vitro* blood-brain-barrier (FIGs 3, 4 and 8). Other preferred administration routes are subcutaneous, intrathecal, intracerebroventricular, intranasal, or transdermal administration.

In another embodiment, the present disclosure provides a method for promoting survival of dopaminergic neurons comprising the step of contacting dopaminergic neurons with the macrocyclic peptide of 8 - 32 amino acids comprising the sequence SEQ ID NO: 25, SEQ ID NO: 26, SEQ ID NO: 27 or SEQ ID NO: 28. Preferably, the method is performed *in vitro* as shown below in the Experimental Section. Said dopaminergic neurons are preferably cultured non-human neurons, such as mouse or rat sympathetic neurons or human neurons derived from induced pluripotent cells (iPSC).

Based on the results provided by the present disclosure, the disclosure is also directed to a macrocyclic peptide with the length of 8 - 32 amino acids comprising the sequence SEQ ID NO: 25, SEQ ID NO: 26, SEQ ID NO: 20 or SEQ ID NO: 28 peptide, for use in the treatment of a degenerative, chronic, or progressive disease or disorder, such as a CNS disease or disorder, or a monogenic hereditary disease (having ER stress as a pathogenic component).

### Methods of making the macrocyclic peptides

Methods of synthesizing the compounds of the present disclosure are known in the art. The following exemplary method may be used. It will be appreciated that the various steps may be performed in an alternate sequence or order to give the desired compounds. Synthetic chemistry transformations and protecting group methodologies (protection and deprotection) useful in synthesizing the compounds described herein are known in the art and include, e.g., those such as described in R. Larock, Comprehensive Organic Transformations, VCH Publishers (1989); T. W. Greene and P. G. M. Wuts, Protective Groups in Organic Synthesis, 3d. Ed., John Wiley and Sons (1999); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995), and subsequent editions thereof.

The peptides of the present disclosure can be made by chemical synthesis methods, which are well known to the ordinarily skilled artisan. See, e.g., Fields et al., Chapter 3 in Synthetic Peptides: A User's Guide, ed. Grant, W. H. Freeman & Co., New York, N.Y., 1992, p. 77. One manner of making of the peptides described herein is using solid phase peptide synthesis (SPPS). The C-terminal amino acid is attached to a cross-linked polystyrene resin via an acid labile bond with a linker molecule. This resin is insoluble in the solvents used for synthesis, making it relatively simple and fast to wash away excess reagents and by-products. The N-terminus is protected with the Fmoc group, which is stable in acid, but removable by base. Any side chain functional groups are protected with base stable, acid labile groups.

The publications and other materials used herein to illuminate the background of the invention, and in particular, to provide additional details with respect to its practice, are incorporated herein by reference.

It is apparent to a person skilled in the art that as technology advanced, the basic idea of the disclosure can be implemented in various ways. The disclosure and its embodiments are therefore not restricted to the below examples, but they may vary within the scope of the claims.

### EXAMPLES

### Example 1

### Neuroprotective effect of linear and macrocyclic compounds on dopaminergic TH-positive neurons injured with MPP+

Neuroprotective effects of compounds were tested in an *in vitro* model in which primary cultures of rat embryonic mesencephalic neurons were stressed with MPP+, the active metabolite of neurotoxin 1-methyl-4-phenyl-1,2,3,6-tetrahydropyridine (MPTP). MPP+ kills dopaminergic (TH-positive) neurons via a variety of toxic mechanisms, including mitochondrial dysfunction, generation of peroxynitrite, oxidative stress, ER stress and induction of apoptosis. Since peptides derived from the C-terminal domain of CDNF and MANF were modified into pseudopeptides, their neuroprotective activity was tested in a model where full-length CDNF protein has shown to be neuroprotective (Fig. 3A&B).

### Materials and methods of synthesis and characterization of head-to-tail cyclic derivatives of CDNF and MANF, and the linear peptides

### General protocol - resin loading

The resin with auxiliary moiety (crypto-thioester Gly-Cys(Hnb)-NH) had been prepared for all syntheses. For this Fmoc-Gly-OH and Fmoc-Cys(StBu)-OH had been coupled to TentaGel R RAM resin (0.2 mmol/g) using 4 equivalents of the corresponding amino acid and equimolar amount of DIC (0.5 M in DMF) and OxymaPure (0.5 M in DMF) as coupling reagents. After removal of the Fmoc-group by treatment with 20% piperidine in DMF reductive amination was performed using 2-hydroxy-5-nitrobenzaldehyde according to the original procedure Terrier et al, 2016). Coupling of first amino acid (Ala or Gly) was then performed by using 10 eq. of Fmoc-AA-OH, 9.5 eq. of HCTU or 10 eq PyBOP/HOBt and 20 eq. of DIPEA followed by capping by acetic anhydride/DIPEA. This resin with auxiliary was used for further solid phase peptide syntheses.

### General protocol - SPPS

Solid phase peptide synthesis was carried out at Biotage Initiato+ Alstra r peptide synthesizer. Standard Fmoc protected amino acids were used for peptide elongation: Ala, Arg(Pbf), Asp(tBu), Gln(Trt), Glu(OtBu), Gly, His(Trt), Ile, Lys(Boc), Leu, Met, Ser(tBu), Thr(tBu), Trp(Boc), Val and Cys(StBu). Removal of Fmoc group was performed using 20% piperidine in DMF, coupling was performed using 4 eq of corresponding amino acid, 3.9 eq of HBTU, 4 eq of HOBt, and 8 eq of DIPEA under microwave irradiation. The crude peptides were deprotected and cleaved from the resin through a treatment with TFA/H₂O/iPr₃SiH for 2h followed by precipitation in cold Et₂O.

### General protocol - Native Chemical Ligation (NCL) and S-S oxidation

The Head-to-tail ring closure was performed using NCL between N-terminal Cys and C-terminal crypto-thioester Gly-Cys(Hnb). The NCL buffer (0.2 M phosphate buffer containing 6 M Gn*HCl, 25 mM MPAA, 50 mM TCEP*HCl, pH 6.4) had been prepared according to a published protocol (Terrier et al. 2016). The linear peptides was dissolved in the buffer to 1 mM concentration and the reaction was stirred at 38°C overnight. The cyclic peptides were isolated by RP-HPLC using appropriate gradient. The purified peptides were lyophilized and then dissolved in acetic acid and treated with 20 mM iodine in MeOH until the disulfide bridge was formed. The target peptides were then purified by RP-HPLC.

The purity of the peptides was assessed by standard HPLC methods and the identity was confirmed using standard LC-MS procedures. The masses of the charged ions obtained from the peptides in the procedure can be found in Fig. 1.

### Materials and methods of synthesis of linear derivatives of CDNF and MANF.

The linear peptides were synthesized using general SPPS protocol as described before. The purity and identity were determined in the same manner. The quality of the peptides was confirmed using LC-MS procedures. The masses of the charged ions obtained in the procedure is found in Fig. 1.

### Confirmation of the cyclisation of the head-to-tail cyclic derivatives of CDNF and MANF using NMR.

All of the head-to-tail cyclic peptides were dissolved in 20 mM sodium phosphate pH 6.0, 10 vol% D₂O, 1 mM EDTA, 100 mM NaCl, 3 µM NaN₃, 0.5 mM DSS buffer at 25°C. Their ¹H/¹³C 1D and 2D NMR spectra were recorded on a 500 MHz spectrometer.

The head-to-tail cyclic peptide compounds have been primarily analysed with 1D ¹H, 2D ¹H TOCSY, 2D ¹H ROESY and 2D ¹H-¹³C HSQC NMR (Figs. 2A-2P). 1D ¹H and 2D ¹H TOCSY NMR data were recorded for compounds 2, 4, 6, 8, 10, 12, 22 and 24 in order to confirm the presence of the N-terminal amide proton (which would be absent in linear peptides). The peak contours of the N-terminal amide protons are assigned for the compounds 2, 4, 6, 8, 10, 12, 22 and 24, and indicated in the Figs. 2B, 2D, 2F, 2H, 2J, 2L, 2N and 2P, respectively. In all of the cases, the number of observed amide protons are consistent with the number of expected amide protons of the head-to-tail cyclized peptide of the particular amino acids (as opposed to a corresponding linear analogue where the N-terminal is not included). This by itself indicates that the compounds are cyclized. However, when the data is interpreted in the light of LC-MS data of the compounds (presented in Fig. 1), the compounds were doubly confirmed to be cyclized compounds.

Due to spectral overlap in both NH and Hα, some of the the i1-i23/27 ROE correlation could not be unambiguously verified (for compounds 2, 6 and 8). For the rest of the peptides (compound 4, 10, 12, 22 and 24) 2D ¹H ROESY data were also collected which was independently sufficient to verify the through-space ROE correlation between i1:HN and i(12/13/23):_Hα, i.e. the sequential HN(i)-H_α(i_-1) correlation, to support the cyclized structure (and these coupled protons being less than 5 Å apart). An expansion of the 2D ¹H ROESY amide fingerprint region of these compounds (compound 4, 10, 12, 22 and 24) is presented in Figs. 2D, 2J, 2L, 2N and 2P, respectively, with the key sequential ROE cross peak flagged at the corresponding H^{N} frequency.

**Materials and methods related to testing of neuroprotective activity of compounds** *Culture of mesencephalic neurons.* Rat dopaminergic neurons were cultured as described by Visanji et al., 2008. Briefly, the midbrains obtained from 15-day-old rat embryos (Janvier, France) were dissected, and the ventral portion of the mesencephalic flexure, a region of the developing brain rich in dopaminergic neurons, was used for the cell preparations. The midbrain cells were dissociated by trypsinization for 20 min at 37°C (solution at a final concentration of 0.05% trypsin and 0.02% EDTA). The reaction was stopped by adding Dulbecco's modified Eagle's medium (DMEM) containing DNAase I grade II (0.5 mg/mL) and 10% of fetal calf serum (FCS). Cells were then mechanically dissociated by 3 passages through a 10 ml pipette. Cells were then centrifuged at 180 x g for 10 min at +4°C on a layer of BSA (3.5%) in L15 medium. The cell pellets were re-suspended in a defined culture serum-free medium consisting of Neurobasal (Invitrogen) supplemented with B27 (2%), L-glutamine (2 mM) and 2% of PS solution and 10ng/ml of Brain-derived neurotrophic factor (BDNF) and 1 ng/mL of Glial-Derived Neurotrophic Factor (GDNF). Viable cells were counted in a Neubauer cytometer using the trypan blue exclusion test. The cells were seeded at a density of 40 000 cells/well in 96 well-plates (pre-coated with poly-L-lysine) and maintained in a humidified incubator at 37°C in 5% CO₂/95% air atmosphere. Half of the medium was changed every 2 days with fresh medium. On 96-wells plates, only 60 wells are used. To avoid any edge effect, the first and last lines and columns were not used for culture and were filled with sterile water.

### Test compounds and MPP+ exposure.

CDNF as well as linear and macrocyclic compounds 1-24 (SEQ ID NOs: 1-24) were tested. On day 6 of culture, the compounds were dissolved in culture medium and then pre-incubated with mesencephalic neurons for 4 hours before the MPP+ application. Four hours after the preincubation of the compounds, MPP+ was added to a final concentration of 4 µM, diluted in control medium still in presence of compounds for 48 h.

### Immunostaining: TH neuron survival, neurite network, and α-syn aggregation in TH neurons.

48 h after intoxication, the cells were fixed by a solution of 4% paraformaldehyde in PBS, pH 7.3 for 20 min at room temperature. The cells were washed twice in PBS, and then permeabilized and non-specific sites were blocked with a solution of PBS containing 0.1% of saponin and 1% FCS for 15 min at room temperature. Next, the cells were incubated with (a) a monoclonal antibody anti-Tyrosine Hydroxylase (TH) produced in mouse at dilution of 1:10000 and with (b) a polyclonal antibody antialpha synuclein (a-syn) antibody produced in rabbit at dilution of 1:400 in PBS containing 1% FCS, 0.1% saponin, for 2 h at room temperature. These antibodies were revealed with a secondary antibody Alexa Fluor 488-conjugated goat anti-mouse IgG at the dilution 1:800 and with an Alexa 568-conjugated goat anti-rabbit IgG at the dilution 1:400 in PBS containing 1% FCS, 0.1% saponin, for 1 h at room temperature.

### Synapse immunostaining: TH neuron and PSD-95 (overlap between TH/PSD-95 neurons).

48 h after intoxication, the cell culture supernatant was removed, and the cells fixed by a solution of 4% paraformaldehyde in PBS, pH 7.3 for 20 min at room temperature. The cells were washed twice in PBS, and then permeabilized and non-specific sites were blocked with a solution of PBS containing 0.1% of saponin and 1% FCS for 15 min at room temperature. Then, the cells were incubated with a) a monoclonal Anti-Tyrosine Hydroxylase (TH) antibody produced in mouse at dilution of 1:10000 in PBS containing 1% FCS, 0.1% saponin, for 2 hours at room temperature, and b) a polyclonal anti-postsynaptic density protein-95 (PSD-95) antibody produced in rabbit at dilution of 1:200 in PBS containing 1% FCS, 0.1% saponin, for 2 h at room temperature. This antibody stains specifically synapses. These antibodies were revealed with Alexa Fluor 488-conjugated goat anti-mouse IgG at the dilution 1:800 and with Alexa Fluor 568-conjugated goat anti-rabbit IgG at the dilution 1:400 in PBS containing 1% FCS, 0.1% saponin, for 1 h at room temperature.

For each condition, pictures representing the whole well area were automatically acquired using ImageXpress (Molecular Devices) at 10x (20 pictures, for TH and α-syn) or at 40x magnification (60 pictures, for TH and PSD-95). The following read-outs were automatically determined by using Custom Module Editor (Molecular Devices):
- Analysis of total number of TH neurons (TH-positive neurons)
- total neurite network of TH positive neurons (in µm)
- number of α-syn inclusions in TH-postive neurons (overlapping between TH and α-syn staining)
- number of synapses in TH-positive neurons (overlap of TH and PSD-95 in µm²).

Total number of TH+ neurons, total neurite network of TH+ neurons and number of synapses of TH+ neurons are presented in Figure 3A (CDNF), Figure 3C (Compound 1 and 2), Figure 3D (Compound 3 and 4), Figure 3G (Compound 5 and 6), Figure 3I (Compound 7 and 8), Figure 3K (Compound 9 and 10), Figure 3M (Compound 11 and 12), Figure 4C (Compound 21 and 22), and Figure 4G (Compound 23 and 24).
Total number of TH+ neurons is presented in Figure 4A (Compound 13, 14, 15 and 16), Figure 4E (Compound 17, 18, 19 and 20).
α -synuclein aggregation in TH-positive neurons of a primary culture of mesencephalic cells after MPP+ injury is presented in Figure 3B (CDNF), Figure 3D (Compound 1 and 2), 3F (Compound 3 and 4), 3H (Compound 5 and 6), 3J (Compound 7 and 8), Figure 3L (Compound 9 and 10), Figure 3N (Compound 11 and 12), Figure 4B (Compound 13, 14, 15 and 16), and Figure 4D (Compound 15 and 16).

The data shows that many macrocyclic peptides dose-dependently protected TH-positive neurons and their neurites and synapses from MPP+ toxicity. Moreover, these macrocyclic compounds effectively reduced the number of α-Syn inclusions, whose accumulation is strongly induced by MPP+, in TH-positive neurons. In most cases the potency of macrocyclic peptides (compounds 2, 6, 8, 10, 12, 22 and 24) was comparable to their linear counterparts (compounds 1, 5, 7, 9, 11, 21 and 23, respectively).

### Example 2

### GRP78 binding of the compounds

CDNF and MANF protect cells from ER stress-induced apoptosis by modulating cellular responses to ER stress. Yan et al (2019) showed that the C-terminus of MANF binds to the nucleotide-binding domain (NBD) of GRP78 and regulates its cellular activities. This data suggests that MANF (and CDNF) have a regulatory interaction rather substrate-like interaction with GRP78, the most abundant chaperone protein in the ER lumen. As GRP78 also serves as a critical ligand of the three receptors of the unfolded protein response (UPR) pathway, IRE1α, PERK and ATF6, it seems that the GRP78 interaction may be a central protein-protein interaction of CDNF and MANF that could mediate their cytoprotective effects. Therefore, we tested binding of CDNF and MANF-derived compounds for their GRP78 binding affinity.

### Materials and methods

### Molecular modeling.

The GRP78-NBD in complex with different compounds mentioned here (Fig 2A-2P) were modelled on the basis of the previously solved structure of the GRP78-NBD:MANF complex using the PRIME module of Schrodinger suite version 2018-4 (Schrödinger Llc, USA) via the MAESTRO interface. The model generated was manually checked using the template structure (PDB: 6HAB, Yan et al, 2019). The model was also verified looking into its Ramachandran diagram.

### Cell-free binding assay.

His tagged active GRP78 was purchased from StressMarq (#PR484426) and was labelled using Red-Tris-NTA dye (10 fold excess of protein to dye ratio; Nanotemper Technologies GmbH). Binding of different peptides (in serial dilution) to labelled GRP78 was measured in a Monolith N.T standard capillary using a Monolith N.A. device (Nanotemper Technologies GmbH) at high power in a PBS environment in the presence of 0.5% Tween-20. Fig. 5B shows tabulated data obtained from MST binding experiments.

A computational model of the NBD of human GRP78 protein in complex with Compound 12 (Fig. 5A) was prepared based on the crystal structure of GRP78-MANF (PDB: 6HAB, Yan et al, 2019) as a template and exploiting the high level of structural homology of CDNF and MANF C-terminal domains.
GRP78 binding of the peptides was tested using microscale thermophoresis (MST) in a cell-free environment. The data shows that representative macrocyclic Compounds 12 and 22 bind to GRP78 with a binding affinity typical for protein-protein interactions, and with similar affinity previously reported for C-terminal domain of human MANF protein-GRP78 interaction (K_{D} ∼ 15 µM; Yan et al, 2019).

### Example 3

### In vitro metabolic stability of linear and macrocyclic compounds in rat plasma.

In this example, the metabolic stability of macrocyclic compounds was tested. As metabolic stability is relevant for compounds planned to be parenterally administered *in vivo,* rat plasma stability study was performed with both macrocyclic and linear compounds.

### Materials and Methods

The linear and macrocyclic compounds 1-12 and 21-24 (SEQ ID NOs: 1-12 and 21-24) were incubated in concentration 1 µM with rat plasma (Sprague-Dawley, male; 400 µl) for different time points (0, 20, 40, 60 or 120 min) at 37°C. The incubation was terminated by acetonitrile. The collected samples were centrifuged for 20 min at 2272 × g and analyzed. Stock solutions were prepared using 50% DMSO, and the compounds were spiked 1/100 to incubation to have final DMSO content of 0.5%. The samples were analyzed by UHPLC/PDA with high resolution mass spectrometry (QE-Orbitrap-MS on DDI mode) to monitor disappearance of the compound. Enalapril 1 µM was used as a disappearance rate control. The analytical method was optimized by using the parent compounds for optimum chromatographic properties (peak shape and retention) and mass spectrometric ionization. The ion chromatograms were extracted from the total ion chromatograms using calculated monoisotopic accurate masses with 5 mDa window. Disappearance was based on LC/MS peak areas, marking 0 min as 100%.

The first-order rate constants k (min-1) of the metabolism were obtained from the slope of time versus logarithm (% of remaining compound) plot using Excel software. The *in vitro* half-life (t1/2) of study compound is defined as: t1/2 =ln2/k. Calculated half-life is based on compound disappearance from rat plasma. In Figure 6A, each pair of bars shows data for an unmodified peptide (linear) and the corresponding modified peptide (cyclic). *In vitro* plasma metabolic stability increased from <30 minutes to >789 minutes. Many macrocyclic compounds (compounds 2, 4, 8, 12 and 24) showed improved stability in rat plasma as compared to their linear counterparts (compounds 1, 3, 7, 11 and 23, respectively).

### Example 4

### In vitro metabolic stability of linear and macrocyclic compounds in human plasma.

Metabolic stability studied using human plasma over the time period of 120 min, with initial test concentration of 1 µM. Samples were analyzed using LC/QE-orbitrap-MS. Calculated half-life is based on compound disappearance in human plasma.

### Materials and Methods

The linear or macrocyclic compounds 1-12 and 21-24 (SEQ ID NOs: 1-12 and 21-24) were incubated in concentration 1 µM with human plasma (mixed gender, 400 µl) for different time points (0, 20, 40, 60 or 120 min) at 37°C. The incubation was terminated by acetonitrile. The collected samples were centrifuged for 20 min at 2272 × g and analyzed. The samples were analyzed by UHPLC/PDA with high resolution mass spectrometry (QE-Orbitrap-MS on DDI mode) to monitor disappearance of the compound. Propanthelin bromide 1 µM was used as a disappearance rate control. The analytical method was optimized by using the parent compounds for optimum chromatographic properties (peak shape and retention) and mass spectrometric ionization. The Ion chromatograms were extracted from the total ion chromatograms using calculated monoisotopic accurate masses with 5 mDa window. Disappearance was based on LC/MS peak areas, marking 0 min as 100%. The first-order rate constants k (min-1) of the metabolism were obtained from the slope of time versus logarithm (% of remaining compound) plot using Excel software. The *in vitro* half-life (t1/2) of study compound is defined as: t1/2 =ln2/k. In Figure 6B, each pair of bars shows data for an unmodified peptide (linear) and the corresponding modified peptide (cyclic).

Peptide stability in human plasma was better for both linear and macrocyclic compounds compared to their stability in rat plasma, as shown by maximal reported half-lives in human plasma (a test-specific maximum for half-life was 795 min). Two macrocyclic compounds (compounds 2 and 24) showed improved stability in human plasma as compared to their linear counterparts (compounds 1 and 23, respectively).

### Example 5

### In vitro metabolic stability of linear and macrocyclic compounds in rat hepatocytes

Since hepatic metabolism plays a central role in clearance of compounds, metabolic stability of macrocyclic and linear compounds was tested in cultured rat hepatocytes.

### Materials and Methods

The linear or macrocyclic compounds 1-12 and 21-24 (SEQ ID NOs: 1-12 and 21-24) were incubated in concentration 1 µM with pooled cryopreserved rat hepatocytes (Spraque-Dawley, male; 400 µl, 1.0 million viable cells /ml) for different time points (0, 10, 20, 40 or 60 min) at 37°C. The cell density and viability were determined by trypan blue exclusion method. The incubation was terminated by acetonitrile. The collected samples were centrifuged for 20 min at 2272 × g and analyzed. The samples were analyzed by UHPLC/PDA with high resolution mass spectrometry (QE-Orbitrap-MS on DDI mode) to monitor disappearance of the compound. Verapramil 1µM was used as a disappearance rate control. The analytical method was optimized by using the parent compounds for optimum chromatographic properties (peak shape and retention) and mass spectrometric ionization. The Ion chromatograms were extracted from the total ion chromatograms using calculated monoisotopic accurate masses with 5 mDa window. Disappearance was based on LC/MS peak areas, marking 0 min as 100%. The first-order rate constants k (min-1) of the metabolism were obtained from the slope of time versus logarithm (% of remaining compound) plot using Excel software. The *in vitro* half-life (t1/2) of study compound is defined as: t1/2 =ln2/k. Calculated half-life is based on compound disappearance in rat liver hepatocytes.

In Figure 7A, each pair of bars shows data for an unmodified peptide (linear) and the corresponding modified peptide (cyclic). *In vitro* hepatocyte metabolic stability increased from >10 minutes to >395 minutes.

All macrocyclic compounds (compounds 2, 4, 6, 8, 10, 12, 22 and 24) showed significantly improved stability in rat hepatocytes as compared to their linear counterparts (compounds 1, 3, 5, 7, 9, 11, 21 and 23, respectively).

### Example 6

### In vitro metabolic stability of linear and macrocyclic compounds in human hepatocytes

### Materials and Methods

The linear or macrocyclic compounds 1-12 and 21-24 (SEQ ID NOs: 1-12 and 21-24) were incubated in concentration 1 µM with pooled cryopreserved human hepatocytes (mixed gender; 400 µl, 1.0 million viable cells /ml) for different time points (0, 10, 20, 40 or 60 min) at 37°C. The cell density and viability were determined by trypan blue exclusion method. The incubation was terminated by acetonitrile. The collected samples were centrifuged for 20 min at 2272 × g and analyzed. The samples were analyzed by UPLC/PDA with high resolution mass spectrometry (QE-Orbitrap-MS on DDI mode) to monitor disappearance of the compound. Verapramil 1 µM was used as a disappearance rate control. The analytical method was optimized by using the parent compounds for optimum chromatographic properties (peak shape and retention) and mass spectrometric ionization. The Ion chromatograms were extracted from the total ion chromatograms using calculated monoisotopic accurate masses with 5 mDa window. Disappearance was based on LC/MS peak areas, marking 0 min as 100%. The first-order rate constants k (min-1) of the metabolism were obtained from the slope of time versus logarithm (% of remaining compound) plot using Excel software. The *in vitro* half-life (t1/2) of study compound is defined as: t1/2 =ln2/k. Calculated half-life is based on compound disappearance in human liver hepatocytes.
In Figure 7B, each pair of bars shows data for an unmodified peptide (linear) and the corresponding modified peptide (cyclic).

All macrocyclic compounds (compounds 2, 4, 6, 8, 10, 12, 22 and 24) showed significantly improved stability in human hepatocytes as compared to their linear counterparts (compounds 1, 3, 5, 7, 9, 11, 21 and 23, respectively).

### Example 7

### Penetration of linear and macrocyclic compounds through a 3D in vitro model of blood-brain barrier

Due to the interest of developing CDNF- and MANF-derived peptides for treatment of CNS diseases with peripheral route of administration, and the fact that macrocyclization can improve cell-penetration and distribution properties of peptides, the ability of the compounds to pass the blood-brain barrier was tested in an established in vitro model of the blood-brain barrier. For this purpose, macrocyclic and linear compounds were incubated at 500 nM for 2 h in a two-compartment in vitro blood-brain barrier model (n=4) followed by sample collection and LC-MS/MS analysis.

### Materials and Methods

*Primary culture of astrocytes.* Rat astrocytes were prepared from a E15 embryos. Briefly, pregnant female rats (Wistar, Janvier Labs) of 15 days of gestation were deeply anesthetized in a (CO₂ chamber) and then killed by cervical dislocation. Fetuses were collected and immediately placed in ice-cold L15 Leibovitz medium with a 2% penicillin (10,000 U/mL) and streptomycin (10 mg/mL) solution (PS) and 1% bovine serum albumin (BSA). Full brains were treated for 20 min at 37°C with a trypsin- EDTA solution at a final concentration of 0.05 % trypsin and 0.02 % EDTA. Dissociated cells were cultured in DMEM 10% fetal calf serum. Purified astrocytes were used at passage 4 (P4).

*Culture of human endothelial cells.* A vial of HBMEC (Primary Human Brain Microvascular Endothelial Cells, ACBRI 376) was used at a specific passage 8 (P8).

*Primary culture of cortical neurons.* Rat cortical neurons were cultured as described by Callizot et al., 2013 with modification. Briefly, pregnant female rats (Wistar, Janvier Labs) of 15 days of gestation were deeply anesthetized in a CO₂ chamber and then killed by cervical dislocation. Fetuses were collected and immediately placed in ice-cold L15 Leibovitz medium with a 2% penicillin (10,000 U/mL) and streptomycin (10 mg/mL) solution (PS) and 1% bovine serum albumin (BSA). Cortices were treated for 20 min at 37°C with a trypsin-EDTA solution at a final concentration of 0.05% trypsin and 0.02% EDTA. The dissociation was stopped by addition of Dulbecco's modified Eagle's medium (DMEM) with 4.5 g/L of glucose, containing DNAse I grade II (final concentration 0.5 mg/mL) and 10% fetal calf serum (FCS). Cells were mechanically dissociated by three forced passages through the tip of a 10-ml pipette. Cells were then centrifuged at 515 x g for 10 min at 4°C. The pellet resuspended in a defined culture medium consisting of Neurobasal medium with a 2% solution of B27 supplement, 2 mmol/L of L-glutamine, 2% of PS solution, 10 ng/mL of brain-derived neurotrophic factor (BDNF). Viable cells were counted in a Neubauer cytometer, using the trypan blue exclusion test. The cortical neurons were seeded in the bottom of well pre-coated with poly-L-lysine at a density of 255,000 per well in 24-well plate with insert and cultured at 37°C in an air (95%)-CO₂ (5%) incubator. The culture medium was changed every other day.

*Co-Culture of endothelial cells, astrocytes and primary cortical neurons.* The procedure was performed as previously published (Xue et al., 2013 with modifications, Callizot et al., 2017). Briefly, on day 0, purified astrocytes (P4) were rapidly thawed in a water bath at 37°C. The cells were immediately put in DMEM containing 10% of FCS. Cell suspension was centrifuged at 515 x g for 5 min at 4°C and the pellets were suspended in DMEM F12 containing 10 % of FCS. Cells were seeded in the outer side of the insert membrane (PET, 1 µm) at the density of 45,000 cells per insert and cultured at 37°C in an air (95%)-CO₂ (5%) incubator. Thirty-six (36) hours after the astrocytes seeding, HBMEC (P8) were rapidly thawed in a water bath at 37°C and immediately put in DMEM containing 10% of FCS. Cell suspension was centrifuged at 515 x g for 5 min at 4°C and the pellets were suspended in EGM-2 bullet kit containing 5% of FCS, 1% of PS solution, 1.4 µM of Hydrocortisone, 5 µg/mL of acid ascorbic, 1% of lipid mixture, 10 mM of HEPES, 1ng/mL of bFGF. Cells were seeded in the inner side of the insert membrane (PET, 1µm) at the density of 50,000 cells per insert and were cultured at 37°C in an air (95%)-CO₂ (5%) incubator. Thirty-six (36) hours after the HBMEC seeding (72 hours after the seeding of astrocytes), the cortical neurons were seeded in the poly-L-lysine pre-coated well-bottom at a density of 170,000 per well and were cultured at 37°C in an air (95%)-CO₂ (5%) incubator.

*Application of linear or macrocyclic compounds.* Five (5) days after HBMEC seeding, following the first testing of the integrity of endothelial cell layer, the test compounds (Compounds 1-12 and 21-22, SEQ ID NO: 1-12 and 21-22) were added to the luminal compartment and incubated for 2 hours in concentration 500 nM.

*Quantification of test compounds.* The detection and quantification of each compound in abluminal supernatants were further performed by mass-spectrometry (MS) analysis. After sample thawing, a 100 µL aliquot of each cell culture sample was analyzed by quantifying the peptides by mass spectroscopy. The percentage of passage calculated represents the percentage of compound applied in the abluminal compartment that was measured in the abluminal compartment at the end of the application.

Fig. 8 presents the results; the amount of compounds which crossed the artificial blood-brain barrier is expressed in percentage of compound original applied concentration. Better BBB passage was observed for macrocyclic compounds compared with the linear compounds. BBB penetration increased from <3% (unmodified >30 aa peptides) to >15% (modified).

All macrocyclic compounds (compounds 2, 4, 6, 8, 10, 12 and 22) showed improved ability to pass through the in vitro blood-brain barrier as compared to their linear counterparts (compounds 1, 3, 5, 7, 9, 11 and 21, respectively).

### Example 8

### Rat pharmacokinetic study

The clearance and elimination of peptides can be mediated by multiple processes in vivo, including metabolism and renal elimination. Since in vitro studies suggested metabolic stability for macrocyclic peptides, the preliminary pharmacokinetic properties were tested in vivo by administrating compounds at a single dose level peripherally (subcutaneously and intravenously) and then the presence of compounds in plasma was determined 0.5 after peripheral dosing.

### Materials and Methods

Test compounds were subcutaneously (s.c.) or intravenously (i.v.) administered to male Spraque-Dawley rats (about six weeks old, n=3 per compound) at 0.5 mg/kg. Blood samples were taken via venepuncture (150 µl blood for 50 µl plasma) to EDTA tubes at 0.5 h after compound administration. Immediately following the sampling, the blood samples were centrifuged for plasma separation (within 30 min: 2700 G, 10 min). The samples were prepared by mixing 50 µL of plasma sample with 50 µL of internal standard solution (100 ng/ml of Enalapril for s.c. or Phenacetine and Leu-Encephalin for i.v. in acetonitrile with 1% of formic acid) and mixed. The samples were mixed for 3 minutes (1600 rpm), centrifuged for 20 minutes at 4000 rpm, supernatant, 50 µl, was diluted with 100 µl of 0.5% formic acid and submitted to LC-MS analysis. The standard samples were prepared into rat plasma by spiking the matrix into concentrations 0.1 -10 000 ng/ml of the analyte, respectively, and otherwise treated as the samples. The analytical method was optimized for reaction monitoring chromatographic (peak shape & retention) shifts and mass spectrometric properties (ionization efficiency, MS/MS detection) without comprehensive pre-validation, but with tests on the detection limit, selectivity and matrix effects. The method performance (intra-assay accuracy & precision and range) was evaluated during the sample analysis using the back-calculated data from the standard samples (>6 concentrations).

Table 1 discloses *in vivo* pharmacokinetic properties of linear and cyclic compounds after peripheral administration to rats. Compound concentration in plasma (Cp; ng/ml) at 0.5 h after intravenous (i.v.) and subcutaneous (s.c.) administration. Peripherally administrated linear peptidic compounds were not detected in plasma at the 0.5 h timepoint. Data are presented as mean +/- SEM (n=3).

Natural linear peptides with low molecular weight typically have short lifespans in the circulation as they are quickly eliminated from blood plasma within minutes due to metabolic degradation and clearance mechanism (Li et al, 2015; Lin et al, 2009). Plasma concentration of selected linear and macrocyclic compounds was measured at 0.5 h after peripheral (subcutaneous or intravenous) administration to rats. None of the tested linear compounds 1, 5 and 9 (SEQ ID NOs: 1, 5, 9) was detected in plasma at 0.5 h after peripheral administration indicating rapid elimination. Macrocyclic compounds 10 and 12 (SEQ ID NOs: 10, 12) demonstrated improved blood retention behavior after systemic peripheral administration. The linear peptides are very rapidly cleared from circulation (s.c. data) but the modified cyclic peptides have longer plasma half-lives and are found at detectable levels in rat plasma 30 min after administration.

**Table 1. In vivo pharmacokinetic properties of linear and macrocyclic compounds after peripheral administration to rats.**

| **Compound ID** | **Administration route** | **Modification** | **Cp at 0.5 h** (ng/ml) | |
|---|---|---|---|---|
| | | | **Mean** | **SEM** |
| compound 10 | s.c. | cyclic | 27.66 | 2.79 |
| compound 10 | i.v | cyclic | 5.74 | 1.00 |
| compound 12 | i.v | cyclic | 44.83 | 12.53 |
| compound 1 | s.c. | linear | not detected | |
| compound 5 | s.c. | linear | not detected | |
| compound 9 | s.c. | linear | not detected | |

### CITATION LIST

### Patent literature

EP 58,481;
US 3,773,919;
WO 2007068803;
WO 2009133247;
WO 2013/3034805;
WO 2018/202957

### Non-patent literature

Airavaara M, Shen H, Kuo CC, Peranen J, Saarma M, Hoffer B, and Wang Y. 2009. Mesencephalic astrocyte-derived neurotrophic factor reduces ischemic brain injury and promotes behavioral recovery in rats. J. Comp. Neurol. 515(1):116-24.
Airavaara M, Harvey BK, Voutilainen MH, Shen H, Chou J, Lindholm P, Lindahl M, Tuominen RK, Saarma M, Hoffer B, Wang Y. 2012. CDNF protects the nigrostriatal dopamine system and promotes recovery after MPTP treatment in mice. Cell Transplant. 21(6):1213-23.
Bertolotti A, Zhang Y, Hendershot LM, Harding HP, Ron D. 2000 Dynamic interaction of BiP and ER stress transducers in the unfolded-protein response. Nat Cell Biol. 2(6):326-32.
Callizot N, Combes M, Steinschneider R, Poindron P. 2013. Operational dissection of beta-amyloid cytophathic effects on cultured neurons. J Neurosci Res. 91(5):706-16
Di L. 2014. Strategic approaches to optimizing peptide ADME properties. AAPS J. 17(1):134-43.
Dornburg R. 1995. Reticuloendotheliosis viruses and derived vectors. Gene Therap. 2: 301-310.
Fletcher JM and Hughes RA. 2006. Novel monocyclic and bicyclic loop mimetics of brain-derived neurotrophic factor. J. Pept. Sci. 12:515-524.
Glembotski CC. 2011. Functions for the cardiomyokine, MANF, in cardioprotection, hypertrophy and heart failure. J. Mol. Cell. Cardiol. 51:512-517.
Hefti F. Neurotrophic factor therapy for nervous system degenerative diseases. 1994. J Neurobiol., 25:1418-1435.
Hellman M, Arumae U, Yu LY, Lindholm P, Peranen J, Saarma M, and Permi P. 2011. Mesencephalic astrocyte-derived neurotrophic factor (MANF) has a unique mechanism to rescue apoptotic neurons. J.Biol.Chem. 286:2675-2680.
Huttunen, HJ and Saarma M. 2019. CDNF protein therapy in Parkinson's disease. Cell Transplantation 1-18.
Langer R, Brem H, Tapper D. 1981. Biocompatibility of polymeric delivery systems for macromolecules. J. Biomed. Mater. Res. 15: 167-277
Langer R. 1982. Controlled release of macromolecules. Chem. Tech., 12:98-105
Lindholm P., Voutilainen M.H., Lauren J., Peranen J., Leppanen V.M., Andressoo J.O., Lindahl M., Janhunen S., Kalkkinen N., Timmusk T., Tuominen R.K., and Saarma M. 2007. Novel neurotrophic factor CDNF protects and rescues midbrain dopamine neurons in vivo. Nature. 448:73-77.
Lindholm P, and Saarma M. 2010. Novel CDNF/MANF family of neurotrophic factors. Dev.Neurobiol. 70:360-371.
Lin JH. 2009. Pharmacokinetics of biotech drugs: peptides, proteins and monoclonal antibodies. Curr Drug Metab. 10(7):661-91.
Li Y, Wang Y, Wei Q, Zheng X, Tang L, Kong D, Gong M. 2015. Variant fatty acid-like molecules Conjugation, novel approaches for extending the stability of therapeutic peptides. Sci Rep. 5:18039.
Nadella R, Voutilainen MH, Saarma M, Gonzalez-Barrios JA, Leon-Chavez BA, Jiménez JM, Jiménez SH, Escobedo L, Martinez-Fong DJ. 2014. Transient transfection of human CDNF gene reduces the 6-hydroxydopamine-induced neuroinflammation in the rat substantia nigra. Neuroinflammation. 16;11:209.
Remington's Pharmaceutical Sciences, 22nd edition, Allen, Loyd V., Jr, Ed., (2012)
Sousa-Victor P, Jasper H, and Neves J. 2018. Trophic Factors in Inflammation and Regeneration: The Role of MANF and CDNF. Front. Physiol. 9:1629.
Terrier et al, Chem.Sci., 2016, 7, 339-345
Tuschl T. 2002. Expanding small RNA interference. Nat. Biotechnol, 20: 446-448.
Visanji NP, Orsi A, Johnston TH, Howson PA, Dixon K, Callizot N, Brotchie JM and Rees DD. 2008. PYM50028, a novel, orally active, nonpeptide neurotrophic factor inducer, prevents and reverses neuronal damage induced by MPP+ in mesencephalic neurons and by MPTP in a mouse model of Parkinson's disease. FASEB J., 22(7):2488-97.
Voutilainen MH, Back S, Peranen J, Lindholm P, Raasmaja A, Mannisto PT, Saarma M, and Tuominen RK. 2011. Chronic infusion of CDNF prevents 6-OHDA-induced deficits in a rat model of Parkinson's disease. Exp. Neurol. 228:99-108.
Voutilainen MH, Back S, Porsti E, Toppinen L, Lindgren L, Lindholm P, Peranen J, Saarma M, and Tuominen RK. 2009. Mesencephalic astrocyte-derived neurotrophic factor is neurorestorative in rat model of Parkinson's disease. J.Neurosci. 29:9651-9659.
Wang M, Kaufman RJ. 2016. Protein misfolding in the endoplasmic reticulum as a conduit to human disease. Nature 529(7586):326-35.
Xue Q, Liu Y, Qi H, Ma Q, Xu L, Chen W, Chen G, Xu X. 2013. A novel brain neurovascular unit model with neurons, astrocytes and microvascular endothelial cells of rat. Int J Biol Sci. 9(2):174-89.
Yan Y, Rato C, Rohland L, Preissler S, Ron D. 2019. MANF antagonizes nucleotide exchange by the endoplasmic reticulum chaperone BiP. Nat Commun. 10(1):541.
Zhao H, Liu Y, Cheng L, Liu B, Zhang W, Guo YJ, Nie L. 2013. Mesencephalic astrocyte-derived neurotrophic factor inhibits oxygen-glucose deprivation-induced cell damage and inflammation by suppressing endoplasmic reticulum stress in rat primary astrocytes. J Mol Neurosci. 51(3):671-8

## Claims

1. A macrocyclic peptide having a length of 8 - 32 amino acids or a pharmaceutically acceptable salt thereof comprising an amino acid sequence of C-X₁-X₂-X₃-C (SEQ ID NO: 25),
wherein
X₁ is selected from the group consisting of R, K, I, G, A and S;
X₂ is absent or selected from the group consisting of G, A, R, K, I and S; and
X₃ is selected from the group consisting of A, G and S.

2. The macrocyclic peptide according to claim 1, comprising an amino acid sequence of E-X₄-C-X₁-X₂-X₃-C-A-E (SEQ ID NO: 26),
wherein
X₁ is selected from the group consisting of R, K, I, G, A and S;
X₂ is absent or selected from the group consisting of G, A, R, K, I, and S;
X₃ is selected from the group consisting of A, G and S; and
X₄ is selected from the group consisting of E, T, V, D, M and G.

3. The macrocyclic peptide according to claim 1 or 2, comprising an amino sequence of X₅-X₆-X₇-X₈-E-X₄-C-X₁-X₂-X₃-C-A-E-X₉-X₁₀-X₁₁ (SEQ ID NO: 27),
wherein
X₁ is selected from the group consisting of R, K, I, G, A, and S;
X₂ is absent or selected from the group consisting of G, A, R, K, I and S;
X₃ is selected from the group consisting of A, G and S;
X₄ is selected from the group consisting of E, T, V, D, M and G;
X₅ is absent or selected from the group consisting of H, D, Q, R, Y, N and S;
X₆ is absent or selected from the group consisting of S, D, G, N and R;
X₇ is absent or W;
X₈ is absent or G;
X₉ is absent or K;
X₁₀ is absent or selected from the group consisting of T, S, A, I and N; and
X₁₁ is absent or selected from D and E.

4. The macrocyclic peptide according to any one of claims 1 - 3, comprising an amino acid sequence, which is within an amino acid sequence of X₁₂-X₁₃-X₁₄-X₁₅-X₁₆-X₁₇-X₁₈-X₁₉-X₂₀-X₂₁-X₂₂-X₂₃-V-X₂₄-E-L-K-X₂₅-X₂₆-L-X₅ X₆-X₇-X₈-E-X₄-C-X₁-X₂-X₃-C-A-E-X₉-X₁₀-X₁₁ (SEQ ID NO: 28),
wherein
X₁ is selected from the group consisting of R, K, I, G, A and S;
X₂ is absent or selected from the group consisting of G, A, R, K, I and S;
X₃ is selected from the group consisting of A, G and S;
X₄ is selected from the group consisting of E, T, V, D, M and G;
X₅ is absent or selected from the group consisting of H, D, Q, R, Y, N and S;
X₆ is absent or selected from the group consisting of S, D, G, N and R;
X₇ is absent or W;
X₈ is absent or G;
X₉ is absent or K;
X₁₀ is absent or selected from the group consisting of T, S, A, I and N;
X₁₁ is absent or selected from D and E;
X₁₂ is absent or selected from the group consisting of L, I and V;
X₁₃ is absent or D;
X₁₄ is absent or selected from L and W;
X₁₅ is absent or selected from the group consisting of A, S, T, E and N;
X₁₆ is absent or selected from S and T;
X₁₇ is absent or selected from V and D;
X₁₈ is absent or selected from D and A;
X₁₉ is absent or L;
X₂₀ is absent or selected from the group consisting of R, K, S and W;
X₂₁ is absent or K;
X₂₂ is absent or selected from the group consisting of M, L, I and V;
X₂₃ is absent or R;
X₂₄ is selected from the group consisting of A, K, T, L and V;
X₂₅ is selected from the group consisting of Q, K and R; and
X₂₆ is selected from I and V.

5. The macrocyclic peptide according to any one of claims 1 - 4, consisting of a sequence selected from the group consisting of:
VDLRKMRVAELKQILHSWGEECRACAE (SEQ ID NO: 2),
VDLKKLRVKELKKILDDWGETCKGCAE (SEQ ID NO: 4),
MRVAELKQILHSWGEECRACAEK (SEQ ID NO: 6),
LRVKELKKILDDWGETCKGCAEK (SEQ ID NO: 8),
WGEECRACAEKT (SEQ ID NO: 10),
WGETCKGCAEKS (SEQ ID NO: 12),
WGEECRGACAEKT (SEQ ID NO: 22), and
WGETCKGGCAEKS (SEQ ID NO: 24).

6. The macrocyclic peptide according to any one of claims 1 - 5, wherein the macrocyclic peptide binds to GRP78.

7. The macrocyclic peptide according to any one of claims 1 - 6, wherein cysteine is in a reduced form or in disulphide bridged form.

8. The macrocyclic peptide according to any one of claims 1 - 7, wherein the macrocyclic peptide is a pseudopeptide.

9. The macrocyclic peptide according to any one of claims 1 - 8 conjugated to a detectable chemical moiety, a biochemical moiety, or polyethylene glycol (PEG).

10. The macrocyclic peptide according to any one of claims 1 - 9, wherein the peptide has at least one of the following properties:
(i) can dose-dependently protect TH-positive neurons from MPP+ toxicity;
(ii) reduces the number of a-synuclein inclusions in TH-positive neurons;
(iii) has improved stability in plasma compared to its linear counterpart;
(iv) has improved stability in hepatocytes compared to its linear counterpart; or
(v) has improved ability to pass through the blood brain barrier compared to its linear counterpart.

11. The macrocyclic peptide according to any one of claims 1 - 10 for use as a medicament.

12. The macrocyclic peptide according to any one of claims 1 - 10 for use in the treatment of a degenerative disease or disorder, a chronic disease or disorder, or a progressive disease or disorder, such as a neurodegenerative disease or disorder.

13. The macrocyclic peptide for use according to claim 12, wherein said neurodegenerative disease or disorder is a central nervous system disease selected from the group consisting of Parkinson's disease, Alzheimer's disease, multiple system atrophy, amyotrophic lateral sclerosis, frontotemporal lobar degeneration, dementia with Lewy bodies, mild cognitive impairment, Huntington's disease, traumatic brain injury, traumatic spinal cord injury, progressive supranuclear palsy, Pick's disease, pure autonomic failure, corticobasal degeneration, chronic traumatic encephalopathy, spinocerebellar ataxia, and peripheral neuropathy.

14. The macrocyclic peptide according to any one of claims 1 - 10 for use in the treatment of a monogenic hereditary disease having ER stress as a pathogenic component selected from the group consisting of Wolcott-Rallison syndrome, Wolfram syndrome, Marinesco-Sjögren syndrome, Machado-Joseph disease, and degenerative retinal diseases such as retinitis pigmentosa, and inherited nephrotic syndromes such as primary nephrotic syndrome and autosomal dominant polycystic kidney disease.

15. The macrocyclic peptide for use according to any one of claims 11 - 14, wherein said peptide is administered by peripheral administration such as intravenous, intraarterial, subcutaneous, intranasal, intraocular, intratympanic, or topical administration, enteral, parenteral or topical routes including oral, rectal, sublingual or buccal administration, intraperitoneal, intramuscular, intraarticular, transdermal, intracochlear, topic ocular, or inhalational administration, or intracranial, intrathecal, epidural or intralesional administration.

16. A pharmaceutical composition comprising the macrocyclic peptide according to any one of claims 1 - 10 and at least one of the following: a pharmaceutically acceptable carrier, a pharmaceutically acceptable excipient, a preservative, a stabilizer and/or a diluent.

17. The pharmaceutical composition according to claim 16 for use as a medicament.

18. A pharmaceutical composition according to claim 16, for use in the treatment of a degenerative disease or disorder, a chronic disease or disorder, or a progressive disease or disorder, such as a neurodegenerative disease or disorder.

19. The pharmaceutical composition for use according to claim 18, wherein said neurodegenerative disease or disorder is a central nervous system disease selected from the group consisting of Parkinson's disease, Alzheimer's disease, multiple system atrophy, amyotrophic lateral sclerosis, frontotemporal lobar degeneration, dementia with Lewy bodies, mild cognitive impairment, Huntington's disease, traumatic brain injury, traumatic spinal cord injury, progressive supranuclear palsy, Pick's disease, pure autonomic failure, corticobasal degeneration, chronic traumatic encephalopathy, spinocerebellar ataxia, and peripheral neuropathy.

20. The pharmaceutical composition according to claim 16 for use in the treatment of a monogenic hereditary disease having ER stress as a pathogenic component selected from the group consisting of Wolcott-Rallison syndrome, Wolfram syndrome, Marinesco-Sjögren syndrome, Machado-Joseph disease, and degenerative retinal diseases such as retinitis pigmentosa, and inherited nephrotic syndromes such as primary nephrotic syndrome and autosomal dominant polycystic kidney disease.

21. The pharmaceutical composition for use according to any one of claims 16 - 20, wherein said composition is administered by peripheral administration such as intravenous, intraarterial, subcutaneous, intranasal, intraocular, intratympanic, or topical administration, enteral, parenteral or topical routes including oral, rectal, sublingual or buccal administration, intraperitoneal, intramuscular, intraarticular, transdermal, intracochlear, topic ocular, or inhalational administration, or intracranial, intrathecal, epidural or intralesional administration.
